# EUROPEAN PATENT APPLICATION

(11) **EP 3 229 022 A1**
(43) Date of publication of application: **11.10.2017**
(21) Application number: 16382156.4
(22) Date of filing: 07.04.2016
(51) Int. Cl.: G01N 33/50, A61K 48/00

(54) **IDENTIFICATION OF CANDIDATE ANTICANCER DRUGS BASED ON RETINOBLASTOMA PHOSPHORYLATION AT SER249/THR252**

(71) Applicant: Universitat Pompeu-Fabra, 08005 Barcelona (ES)
(72) Inventor: POSAS GARRIGA, Francesc, 08003 Barcelona (ES); DE NADAL CLANCHET, Eulàlia, 08003 Barcelona (ES); GUBERN BURSET, Albert, 08003 Barcelona (ES); JOAQUÍN CAUDET, Manuel, 08003 Barcelona (ES)
(74) Representative: Elzaburu S.L.P.

(57) **Abstract**

Control of cancer progression by retinoblastoma phosphorylation. The invention provides a method based on the finding that phosphorylation of RB protein in the N-terminal domain, namely at Serine 249 and Threonine 252 of human RB, leads to a conformational change that makes RB insensitive to CDK inactivation, promoting a cell cycle delay that reduces cell proliferation in cancer cells. The method identifies as candidates to anticancer drugs those compounds that give rise to modifications analogous to those provoked by RB phosphorylation in the N-terminal domain, mainly increased affinity of RB for E2F, decreased transcription of E2F and/or decreased expression of E2F-dependent genes, increased RB binding to E2F-dependent promoters or decreased cell proliferation. The method can be also carried out measuring the expression of an indicator gene operatively linked to an E2F-dependent promoter. Expression vectors of mutated retinoblastoma proteins mimicking phosphorylation at the N-terminal domain are also provided for therapeutic use.

SEQUENCE LISTING

<110>  Universitat Pompeu Fabra

<120>  CONTROL OF CANCER PROGRESSION BY RETINOBLASTOMA PHOSPHORYLATION

<130>  EP-1460

<160>  59

<170>  PatentIn version 3.5

<210>  1
<211>  36
<212>  DNA
<213>  Artificial sequence

<220>
<223>  Primer for PCR amplification of GST-RB

<400>  1
catcatcatg gatccatgcc gcccaaaacc ccccga                                36


<210>  2
<211>  33
<212>  DNA
<213>  Artificial sequence

<220>
<223>  Primer for PCR amplification of GST-RB

<400>  2
catcatcatg gatcctcatt gttggatagt ggt                                   33


<210>  3
<211>  27
<212>  DNA
<213>  Artificial sequence

<220>
<223>  Primer for PCR amplification of full-length RB from pGEX2TK-RB

<400>  3
catggatcca tgccgcccaa aaccccc                                          27


<210>  4
<211>  27
<212>  DNA
<213>  Artificial sequence

<220>
<223>  Primer for PCR amplification of full-length RB from pGEX2TK-RB

<400>  4
catctcgagt catttctctt ccttgtt                                          27


<210>  5
<211>  30
<212>  DNA

<213>  Artificial sequence

<220>
<223>  RB mutagenesis primer of pair T5A

<400>  5
atgccgccca aagccccccg aaaaacgtac                                        30


<210>  6
<211>  24
<212>  DNA
<213>  Artificial sequence

<220>
<223>  RB mutagenesis primer of pair T5A

<400>  6
ggcgggtttc gggggctttt ttgc                                              24


<210>  7
<211>  27
<212>  DNA
<213>  Artificial sequence

<220>
<223>  RB mutagenesis primer of pair S230A

<400>  7
tttattaaac tcgcacctcc catgttg                                           27


<210>  8
<211>  27
<212>  DNA
<213>  Artificial sequence

<220>
<223>  RB mutagenesis primer of pair S230A

<400>  8
aaataatttg agcgtggagg gtacaac                                           27


<210>  9
<211>  27
<212>  DNA
<213>  Artificial sequence

<220>
<223>  RB mutagenesis primer of pair S249A

<400>  9
cccattaatg gtgcacctcg aacaccc                                           27


<210>  10
<211>  27
<212>  DNA
<213>  Artificial sequence

<220>

<223>  RB mutagenesis primer of pair S249A

<400>  10
gggtaattac cacgtggagc ttgtggg                                          27


<210>  11
<211>  27
<212>  DNA
<213>  Artificial sequence

<220>
<223>  RB mutagenesis primer of pair T252A

<400>  11
ggttcacctc gagcacccag gcgaggt                                          27


<210>  12
<211>  27
<212>  DNA
<213>  Artificial sequence

<220>
<223>  RB mutagenesis primer of pair T252A

<400>  12
ccaagtggag ctcgtgggtc cgctcca                                          27


<210>  13
<211>  48
<212>  DNA
<213>  Artificial sequence

<220>
<223>  RB mutagenesis primer of pair S249AT252A

<400>  13
gttataccca ttaatggtgc acctcgagca cccaggcgag gtcagaac                   48


<210>  14
<211>  48
<212>  DNA
<213>  Artificial sequence

<220>
<223>  RB mutagenesis primer of pair S249AT252A

<400>  14
caatatgggt aattaccacg tggagctcgt gggtccgctc cagtcttg                   48


<210>  15
<211>  27
<212>  DNA
<213>  Artificial sequence

<220>
<223>  RB mutagenesis primer of pair 249A252E

<400>  15

ggtgcacctc gagaacccag gcgaggt                                          27

<210>  16
<211>  27
<212>  DNA
<213>  Artificial sequence

<220>
<223>  RB mutagenesis primer of pair 249A252E

<400>  16
ccacgtggag ctcttgggtc cgctcca                                          27

<210>  17
<211>  27
<212>  DNA
<213>  Artificial sequence

<220>
<223>  RB mutagenesis primer of pair 249E252E

<400>  17
cccattaatg gtgaacctcg agaaccc                                          27

<210>  18
<211>  27
<212>  DNA
<213>  Artificial sequence

<220>
<223>  RB mutagenesis primer of pair 249E252E

<400>  18
gggtaattac cacttggagc tcttggg                                          27

<210>  19
<211>  30
<212>  DNA
<213>  Artificial sequence

<220>
<223>  primer for PCR amplification of p38alpha from pMT3-HA-p38

<400>  19
gctagtggat tcatgtctca ggagaggccc                                       30

<210>  20
<211>  30
<212>  DNA
<213>  Artificial sequence

<220>
<223>  primer for PCR amplification of p38alpha from pMT3-HA-p38

<400>  20
gtcgatctcg agtcaggact ccatctcttc                                       30

<210>  21
<211>  15
<212>  PRT
<213>  Artificial sequence

<220>
<223>  Fragment of human RB not phosphorylated at S249/T252


<220>
<221>  source
<222>  (1)..(15)
<223>  Homo sapiens, RB protein

<220>
<221>  note
<222>  (3)..(3)
<223>  Phosphorylatable Serine

<220>
<221>  note
<222>  (6)..(6)
<223>  Phosphorylatable Threonine

<400>  21

Asn Gly Ser Pro Arg Thr Pro Arg Arg Gly Gln Asn Arg Ser Cys
1               5                   10                  15


<210>  22
<211>  15
<212>  PRT
<213>  Artificial sequence

<220>
<223>  peptide used for generating antibodies against human RB
       phosphorylated at Ser249/Thr252


<220>
<221>  source
<222>  (1)..(15)
<223>  Homo sapiens, RB protein

<220>
<221>  note
<222>  (3)..(3)
<223>  phosphorylated serine residue

<220>
<221>  note
<222>  (6)..(6)
<223>  phosphorylated threonine residue

<400>  22

Asn Gly Ser Pro Arg Thr Pro Arg Arg Gly Gln Asn Arg Ser Cys
1               5                   10                  15


<210>  23
<211>  18

<212>  DNA
<213>  Artificial sequence

<220>
<223>  Real-time PCR primer for mad2

<400>  23
aggatgaaat tcgctcag                                                    18


<210>  24
<211>  18
<212>  DNA
<213>  Artificial sequence

<220>
<223>  Real-time PCR primer for mad2

<400>  24
cattgacagg ggttttgt                                                    18


<210>  25
<211>  18
<212>  DNA
<213>  Artificial sequence

<220>
<223>  Real-time PCR primer for E2F1

<400>  25
ggatctggag actgacca                                                    18


<210>  26
<211>  18
<212>  DNA
<213>  Artificial sequence

<220>
<223>  Real-time PCR primer for E2F1

<400>  26
ctccaggaca ttggtgat                                                    18


<210>  27
<211>  18
<212>  DNA
<213>  Artificial sequence

<220>
<223>  Real-time PCR primer for BRCA1

<400>  27
actgaaaggc atccagaa                                                    18


<210>  28
<211>  18
<212>  DNA
<213>  Artificial sequence

<220>
<223>  Real-time PCR primer for BRCA1

<400>  28
gaacctgcct gtcgttac                                                    18


<210>  29
<211>  18
<212>  DNA
<213>  Artificial sequence

<220>
<223>  Real-time PCR primer for CycA2

<400>  29
gtccttcatg gaaagcag                                                    18


<210>  30
<211>  18
<212>  DNA
<213>  Artificial sequence

<220>
<223>  Real-time PCR primer for CycA2

<400>  30
acgttcactg gcttgtct                                                    18


<210>  31
<211>  20
<212>  DNA
<213>  Artificial sequence

<220>
<223>  Real-time PCR primer for CycE1

<400>  31
cctccaaagt tgcaccagtt                                                  20


<210>  32
<211>  20
<212>  DNA
<213>  Artificial sequence

<220>
<223>  Real-time PCR primer for CycE1

<400>  32
ggacgcacag gtctagaagc                                                  20


<210>  33
<211>  21
<212>  DNA
<213>  Artificial sequence

<220>
<223>  Real-time PCR primer for GAPDH

<400>  33
aattcaacgg cacagtcaag g                                                21


<210>  34
<211>  21
<212>  DNA
<213>  Artificial sequence

<220>
<223>  Real-time PCR primer for GAPDH

<400>  34
ggatgcaggg atgatgttct g                                                21


<210>  35
<211>  18
<212>  DNA
<213>  Artificial sequence

<220>
<223>  PCR primer for human CycA2

<400>  35
ccccagccag tttgtttc                                                    18


<210>  36
<211>  12
<212>  DNA
<213>  Artificial sequence

<220>
<223>  PCR primer for human CycA2

<400>  36
tcaagttcat ag                                                          12


<210>  37
<211>  18
<212>  DNA
<213>  Artificial sequence

<220>
<223>  PCR primer for human E2F1

<400>  37
gcgttaaagc caatagga                                                    18


<210>  38
<211>  17
<212>  DNA
<213>  Artificial sequence

<220>
<223>  PCR primer for human E2F1

<400>  38
cttttacgcg ccaaatc                                                     17

<210>  39
<211>  19
<212>  DNA
<213>  Artificial sequence

<220>
<223>  PCR primer for mouse CycA2

<400>  39
ctcccgccct gtaagattc                                                    19


<210>  40
<211>  20
<212>  DNA
<213>  Artificial sequence

<220>
<223>  PCR primer for mouse CycA2

<400>  40
agttcaagta tcccgcgact                                                   20


<210>  41
<211>  18
<212>  DNA
<213>  Artificial sequence

<220>
<223>  PCR primer for mouse CycE1

<400>  41
cgcaggccct gacatcta                                                     18


<210>  42
<211>  17
<212>  DNA
<213>  Artificial sequence

<220>
<223>  PCR primer for mouse CycE1

<400>  42
accagcgagg agctctg                                                      17


<210>  43
<211>  10
<212>  DNA
<213>  Artificial sequence

<220>
<223>  E2F binding sequence, including two additional nucleotides that
       can follow it


<220>
<221>  note
<222>  (4)..(5)
<223>  S can be G or C

<220>
<221>  note
<222>  (9)..(9)
<223>  N can be A, T, G or C (added for incluiding it in the sequence
       listing)

<220>
<221>  misc_feature
<222>  (9)..(10)
<223>  n is a, c, g, or t

<220>
<221>  note
<222>  (10)..(10)
<223>  N can be A, T, G or C (added for including the motif in the
       sequence listing)

<400>  43
tttsscgcnn                                                              10


<210>  44
<211>  51
<212>  DNA
<213>  Artificial sequence

<220>
<223>  TATA oligonucleotide used for the construction of an
       E2F-dependent promoter

<400>  44
gatctacttg ggcataaaag gcagagcagg ggcagctgct gcttacactt a               51


<210>  45
<211>  64
<212>  DNA
<213>  Artificial sequence

<220>
<223>  3xE2F oligonucleotide used for the construction of an
       E2F-dependent promoter

<400>  45
ctgcaatttc gcgccaaact tgtgcaattt cgcgccaaac ttgtgcaatt tcgcgccaac      60

ttgc                                                                    64


<210>  46
<211>  1314
<212>  DNA
<213>  Homo sapiens


<220>
<221>  CDS
<222>  (1)..(1314)
<223>  Coding sequence of E2F1 human protein

<400>  46
atg gcc ttg gcc ggg gcc cct gcg ggc ggc cca tgc gcg ccg gcg ctg        48

Met Ala Leu Ala Gly Ala Pro Ala Gly Gly Pro Cys Ala Pro Ala Leu
1               5                   10                  15

gag gcc ctg ctc ggg gcc ggc gcg ctg cgg ctg ctc gac tcc tcg cag      96
Glu Ala Leu Leu Gly Ala Gly Ala Leu Arg Leu Leu Asp Ser Ser Gln
            20                  25                  30

atc gtc atc atc tcc gcc gcg cag gac gcc agc gcc ccg ccg gct ccc      144
Ile Val Ile Ile Ser Ala Ala Gln Asp Ala Ser Ala Pro Pro Ala Pro
        35                  40                  45

acc ggc ccc gcg gcg ccc gcc gcc ggc ccc tgc gac cct gac ctg ctg      192
Thr Gly Pro Ala Ala Pro Ala Ala Gly Pro Cys Asp Pro Asp Leu Leu
    50                  55                  60

ctc ttc gcc aca ccg cag gcg ccc cgg ccc aca ccc agt gcg ccg cgg      240
Leu Phe Ala Thr Pro Gln Ala Pro Arg Pro Thr Pro Ser Ala Pro Arg
65                  70                  75                  80

ccc gcg ctc ggc cgc ccg ccg gtg aag cgg agg ctg gac ctg gaa act      288
Pro Ala Leu Gly Arg Pro Pro Val Lys Arg Arg Leu Asp Leu Glu Thr
                85                  90                  95

gac cat cag tac ctg gcc gag agc agt ggg cca gct cgg ggc aga ggc      336
Asp His Gln Tyr Leu Ala Glu Ser Ser Gly Pro Ala Arg Gly Arg Gly
            100                 105                 110

cgc cat cca gga aaa ggt gtg aaa tcc ccg ggg gag aag tca cgc tat      384
Arg His Pro Gly Lys Gly Val Lys Ser Pro Gly Glu Lys Ser Arg Tyr
        115                 120                 125

gag acc tca ctg aat ctg acc acc aag cgc ttc ctg gag ctg ctg agc      432
Glu Thr Ser Leu Asn Leu Thr Thr Lys Arg Phe Leu Glu Leu Leu Ser
    130                 135                 140

cac tcg gct gac ggt gtc gtc gac ctg aac tgg gct gcc gag gtg ctg      480
His Ser Ala Asp Gly Val Val Asp Leu Asn Trp Ala Ala Glu Val Leu
145                 150                 155                 160

aag gtg cag aag cgg cgc atc tat gac atc acc aac gtc ctt gag ggc      528
Lys Val Gln Lys Arg Arg Ile Tyr Asp Ile Thr Asn Val Leu Glu Gly
                165                 170                 175

atc cag ctc att gcc aag aag tcc aag aac cac atc cag tgg ctg ggc      576
Ile Gln Leu Ile Ala Lys Lys Ser Lys Asn His Ile Gln Trp Leu Gly
            180                 185                 190

agc cac acc aca gtg ggc gtc ggc gga cgg ctt gag ggg ttg acc cag      624
Ser His Thr Thr Val Gly Val Gly Gly Arg Leu Glu Gly Leu Thr Gln
        195                 200                 205

gac ctc cga cag ctg cag gag agc gag cag cag ctg gac cac ctg atg      672
Asp Leu Arg Gln Leu Gln Glu Ser Glu Gln Gln Leu Asp His Leu Met
    210                 215                 220

aat atc tgt act acg cag ctg cgc ctg ctc tcc gag gac act gac agc      720
Asn Ile Cys Thr Thr Gln Leu Arg Leu Leu Ser Glu Asp Thr Asp Ser
225                 230                 235                 240

cag cgc ctg gcc tac gtg acg tgt cag gac ctt cgt agc att gca gac      768
Gln Arg Leu Ala Tyr Val Thr Cys Gln Asp Leu Arg Ser Ile Ala Asp
                245                 250                 255

cct gca gag cag atg gtt atg gtg atc aaa gcc cct cct gag acc cag 816
Pro Ala Glu Gln Met Val Met Val Ile Lys Ala Pro Pro Glu Thr Gln
            260                 265                 270

ctc caa gcc gtg gac tct tcg gag aac ttt cag atc tcc ctt aag agc 864
Leu Gln Ala Val Asp Ser Ser Glu Asn Phe Gln Ile Ser Leu Lys Ser
        275                 280                 285

aaa caa ggc ccg atc gat gtt ttc ctg tgc cct gag gag acc gta ggt 912
Lys Gln Gly Pro Ile Asp Val Phe Leu Cys Pro Glu Glu Thr Val Gly
    290                 295                 300

ggg atc agc cct ggg aag acc cca tcc cag gag gtc act tct gag gag 960
Gly Ile Ser Pro Gly Lys Thr Pro Ser Gln Glu Val Thr Ser Glu Glu
305                 310                 315                 320

gag aac agg gcc act gac tct gcc acc ata gtg tca cca cca cca tca 1008
Glu Asn Arg Ala Thr Asp Ser Ala Thr Ile Val Ser Pro Pro Pro Ser
                325                 330                 335

tct ccc ccc tca tcc ctc acc aca gat ccc agc cag tct cta ctc agc 1056
Ser Pro Pro Ser Ser Leu Thr Thr Asp Pro Ser Gln Ser Leu Leu Ser
            340                 345                 350

ctg gag caa gaa ccg ctg ttg tcc cgg atg ggc agc ctg cgg gct ccc 1104
Leu Glu Gln Glu Pro Leu Leu Ser Arg Met Gly Ser Leu Arg Ala Pro
        355                 360                 365

gtg gac gag gac cgc ctg tcc ccg ctg gtg gcg gcc gac tcg ctc ctg 1152
Val Asp Glu Asp Arg Leu Ser Pro Leu Val Ala Ala Asp Ser Leu Leu
    370                 375                 380

gag cat gtg cgg gag gac ttc tcc ggc ctc ctc cct gag gag ttc atc 1200
Glu His Val Arg Glu Asp Phe Ser Gly Leu Leu Pro Glu Glu Phe Ile
385                 390                 395                 400

agc ctt tcc cca ccc cac gag gcc ctc gac tac cac ttc ggc ctc gag 1248
Ser Leu Ser Pro Pro His Glu Ala Leu Asp Tyr His Phe Gly Leu Glu
                405                 410                 415

gag ggc gag ggc atc aga gac ctc ttc gac tgt gac ttt ggg gac ctc 1296
Glu Gly Glu Gly Ile Arg Asp Leu Phe Asp Cys Asp Phe Gly Asp Leu
            420                 425                 430

acc ccc ctg gat ttc tga 1314
Thr Pro Leu Asp Phe
        435

<210> 47
<211> 437
<212> PRT
<213> Homo sapiens

<400> 47

Met Ala Leu Ala Gly Ala Pro Ala Gly Gly Pro Cys Ala Pro Ala Leu
1               5                   10                  15

Glu Ala Leu Leu Gly Ala Gly Ala Leu Arg Leu Leu Asp Ser Ser Gln
            20                  25                  30

Ile Val Ile Ile Ser Ala Ala Gln Asp Ala Ser Ala Pro Pro Ala Pro
        35                  40                  45

Thr Gly Pro Ala Ala Pro Ala Ala Gly Pro Cys Asp Pro Asp Leu Leu
    50                  55                  60

Leu Phe Ala Thr Pro Gln Ala Pro Arg Pro Thr Pro Ser Ala Pro Arg
65                  70                  75                  80

Pro Ala Leu Gly Arg Pro Pro Val Lys Arg Arg Leu Asp Leu Glu Thr
                85                  90                  95

Asp His Gln Tyr Leu Ala Glu Ser Ser Gly Pro Ala Arg Gly Arg Gly
            100                 105                 110

Arg His Pro Gly Lys Gly Val Lys Ser Pro Gly Glu Lys Ser Arg Tyr
        115                 120                 125

Glu Thr Ser Leu Asn Leu Thr Thr Lys Arg Phe Leu Glu Leu Leu Ser
    130                 135                 140

His Ser Ala Asp Gly Val Val Asp Leu Asn Trp Ala Ala Glu Val Leu
145                 150                 155                 160

Lys Val Gln Lys Arg Arg Ile Tyr Asp Ile Thr Asn Val Leu Glu Gly
                165                 170                 175

Ile Gln Leu Ile Ala Lys Lys Ser Lys Asn His Ile Gln Trp Leu Gly
            180                 185                 190

Ser His Thr Thr Val Gly Val Gly Gly Arg Leu Glu Gly Leu Thr Gln
        195                 200                 205

Asp Leu Arg Gln Leu Gln Glu Ser Glu Gln Gln Leu Asp His Leu Met
    210                 215                 220

Asn Ile Cys Thr Thr Gln Leu Arg Leu Leu Ser Glu Asp Thr Asp Ser
225                 230                 235                 240

Gln Arg Leu Ala Tyr Val Thr Cys Gln Asp Leu Arg Ser Ile Ala Asp
                245                 250                 255

Pro Ala Glu Gln Met Val Met Val Ile Lys Ala Pro Pro Glu Thr Gln
            260                 265                 270

Leu Gln Ala Val Asp Ser Ser Glu Asn Phe Gln Ile Ser Leu Lys Ser
        275                 280                 285

Lys Gln Gly Pro Ile Asp Val Phe Leu Cys Pro Glu Glu Thr Val Gly
    290                 295                 300

Gly Ile Ser Pro Gly Lys Thr Pro Ser Gln Glu Val Thr Ser Glu Glu
305                 310                 315                 320

Glu Asn Arg Ala Thr Asp Ser Ala Thr Ile Val Ser Pro Pro Pro Ser
                325                 330                 335

Ser Pro Pro Ser Ser Leu Thr Thr Asp Pro Ser Gln Ser Leu Leu Ser
            340                 345                 350

Leu Glu Gln Glu Pro Leu Leu Ser Arg Met Gly Ser Leu Arg Ala Pro
        355                 360                 365

Val Asp Glu Asp Arg Leu Ser Pro Leu Val Ala Ala Asp Ser Leu Leu
    370                 375                 380

Glu His Val Arg Glu Asp Phe Ser Gly Leu Leu Pro Glu Glu Phe Ile
385                 390                 395                 400

Ser Leu Ser Pro Pro His Glu Ala Leu Asp Tyr His Phe Gly Leu Glu
                405                 410                 415

Glu Gly Glu Gly Ile Arg Asp Leu Phe Asp Cys Asp Phe Gly Asp Leu
            420                 425                 430

Thr Pro Leu Asp Phe
        435


<210>  48
<211>  9
<212>  PRT
<213>  Artificial sequence

<220>
<223>  HA-tag, from human influenza hemagglutinin

<400>  48

Tyr Pro Tyr Asp Val Pro Asp Tyr Ala
1               5


<210>  49
<211>  10
<212>  PRT
<213>  Artificial sequence

<220>
<223>  Myc-tag

<400>  49

Glu Gln Lys Leu Ile Ser Glu Glu Asp Leu
1               5                   10

<210>  50
<211>  723
<212>  DNA
<213>  Artificial sequence

<220>
<223>  Coding sequence of GST tag

<220>
<221>  CDS
<222>  (1)..(723)
<223>  glutathione S-transferase

<400>  50
atg tcc cct ata cta ggt tat tgg aaa att aag ggc ctt gtg caa ccc        48
Met Ser Pro Ile Leu Gly Tyr Trp Lys Ile Lys Gly Leu Val Gln Pro
1               5                   10                  15

act cga ctt ctt ttg gaa tat ctt gaa gaa aaa tat gaa gag cat ttg        96
Thr Arg Leu Leu Leu Glu Tyr Leu Glu Glu Lys Tyr Glu Glu His Leu
            20                  25                  30

tat gag cgc gat gaa ggt gat aaa tgg cga aac aaa aag ttt gaa ttg       144
Tyr Glu Arg Asp Glu Gly Asp Lys Trp Arg Asn Lys Lys Phe Glu Leu
        35                  40                  45

ggt ttg gag ttt ccc aat ctt cct tat tat att gat ggt gat gtt aaa       192
Gly Leu Glu Phe Pro Asn Leu Pro Tyr Tyr Ile Asp Gly Asp Val Lys
    50                  55                  60

tta aca cag tct atg gcc atc ata cgt tat ata gct gac aag cac aac       240
Leu Thr Gln Ser Met Ala Ile Ile Arg Tyr Ile Ala Asp Lys His Asn
65                  70                  75                  80

atg ttg ggt ggt tgt cca aaa gag cgt gca gag att tca atg ctt gaa       288
Met Leu Gly Gly Cys Pro Lys Glu Arg Ala Glu Ile Ser Met Leu Glu
                85                  90                  95

gga gcg gtt ttg gat att aga tac ggt gtt tcg aga att gca tat agt       336
Gly Ala Val Leu Asp Ile Arg Tyr Gly Val Ser Arg Ile Ala Tyr Ser
            100                 105                 110

aaa gac ttt gaa act ctc aaa gtt gat ttt ctt agc aag cta cct gaa       384
Lys Asp Phe Glu Thr Leu Lys Val Asp Phe Leu Ser Lys Leu Pro Glu
        115                 120                 125

atg ctg aaa atg ttc gaa gat cgt tta tgt cat aaa aca tat tta aat       432
Met Leu Lys Met Phe Glu Asp Arg Leu Cys His Lys Thr Tyr Leu Asn
    130                 135                 140

ggt gat cat gta acc cat cct gac ttc atg ttg tat gac gct ctt gat       480
Gly Asp His Val Thr His Pro Asp Phe Met Leu Tyr Asp Ala Leu Asp
145                 150                 155                 160

gtt gtt tta tac atg gac cca atg tgc ctg gat gcg ttc cca aaa tta       528

Val Val Leu Tyr Met Asp Pro Met Cys Leu Asp Ala Phe Pro Lys Leu
                165                 170                 175

gtt tgt ttt aaa aaa cgt att gaa gct atc cca caa att gat aag tac       576
Val Cys Phe Lys Lys Arg Ile Glu Ala Ile Pro Gln Ile Asp Lys Tyr
            180                 185                 190

ttg aaa tcc agc aag tat ata gca tgg cct ttg cag ggc tgg caa gcc       624
Leu Lys Ser Ser Lys Tyr Ile Ala Trp Pro Leu Gln Gly Trp Gln Ala
        195                 200                 205

acg ttt ggt ggt ggc gac cat cct cca aaa tcg gat ctg gtt ccg cgt       672
Thr Phe Gly Gly Gly Asp His Pro Pro Lys Ser Asp Leu Val Pro Arg
    210                 215                 220

gga tct cgt cgt gca tct gtt gga tcc ccg gga att cat cgt gac tga       720
Gly Ser Arg Arg Ala Ser Val Gly Ser Pro Gly Ile His Arg Asp
225                 230                 235

ctg                                                                   723
Leu
240


<210>  51
<211>  239
<212>  PRT
<213>  Artificial sequence

<220>
<223>  Synthetic Construct

<400>  51

Met Ser Pro Ile Leu Gly Tyr Trp Lys Ile Lys Gly Leu Val Gln Pro
1               5                   10                  15


Thr Arg Leu Leu Leu Glu Tyr Leu Glu Glu Lys Tyr Glu Glu His Leu
            20                  25                  30


Tyr Glu Arg Asp Glu Gly Asp Lys Trp Arg Asn Lys Lys Phe Glu Leu
        35                  40                  45


Gly Leu Glu Phe Pro Asn Leu Pro Tyr Tyr Ile Asp Gly Asp Val Lys
    50                  55                  60


Leu Thr Gln Ser Met Ala Ile Ile Arg Tyr Ile Ala Asp Lys His Asn
65                  70                  75                  80


Met Leu Gly Gly Cys Pro Lys Glu Arg Ala Glu Ile Ser Met Leu Glu
                85                  90                  95


Gly Ala Val Leu Asp Ile Arg Tyr Gly Val Ser Arg Ile Ala Tyr Ser
            100                 105                 110


Lys Asp Phe Glu Thr Leu Lys Val Asp Phe Leu Ser Lys Leu Pro Glu

115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr
            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Val Pro Arg
    210                 215                 220

Gly Ser Arg Arg Ala Ser Val Gly Ser Pro Gly Ile His Arg Asp
225                 230                 235

<210>  52
<211>  30
<212>  DNA
<213>  Artificial sequence

<220>
<223>  Oligonucleotide for amplifying MKK6DD

<400>  52
gctagtgaat tcatgtctca gtcgaaaggc                                        30

<210>  53
<211>  30
<212>  DNA
<213>  Artificial sequence

<220>
<223>  Oligonucleotide for amplifying MKK6DD

<400>  53
gtcgatgtcg acttagtctc caagaatcag                                        30

<210>  54
<211>  48
<212>  DNA
<213>  Artificial sequence

<220>
<223>  Oligonucleotide encoding HA epitope

<400>  54
agcttaccat ggcctacccc tacgacgtgc ccgactacgc ctccctcg                    48


<210>  55
<211>  48
<212>  DNA
<213>  Artificial sequence

<220>
<223>  Oligonucleotide encoding HA epitope

<400>  55
gatccgaggg aggcgtagtc gggcacgtcg taggggtagg ccatggta                    48


<210>  56
<211>  2787
<212>  DNA
<213>  Homo sapiens


<220>
<221>  CDS
<222>  (1)..(2787)
<223>  RB

<400>  56
atg ccg ccc aaa acc ccc cga aaa acg gcc gcc acc gcc gcc gct gcc         48
gcc gcg gaa ccc ccg gca ccg ccg ccg ccg ccc cct cct gag gag gac         96
cca gag cag gac agc ggc ccg gag gac ctg cct ctc gtc agg ctt gag        144
ttt gaa gaa aca gaa gaa cct gat ttt act gca tta tgt cag aaa tta        192
aag ata cca gat cat gtc aga gag aga gct tgg tta act tgg gag aaa        240
gtt tca tct gtg gat gga gta ttg gga ggt tat att caa aag aaa aag        288
gaa ctg tgg gga atc tgt atc ttt att gca gca gtt gac cta gat gag        336
atg tcg ttc act ttt act gag cta cag aaa aac ata gaa atc agt gtc        384
cat aaa ttc ttt aac tta cta aaa gaa att gat acc agt acc aaa gtt        432
gat aat gct atg tca aga ctg ttg aag aag tat gat gta ttg ttt gca 480
ctc ttc agc aaa ttg gaa agg aca tgt gaa ctt ata tat ttg aca caa 528
ccc agc agt tcg ata tct act gaa ata aat tct gca ttg gtg cta aaa 576
gtt tct tgg atc aca ttt tta tta gct aaa ggg gaa gta tta caa atg 624
gaa gat gat ctg gtg att tca ttt cag tta atg cta tgt gtc ctt gac 672
tat ttt att aaa ctc tca cct ccc atg ttg ctc aaa gaa cca tat aaa 720
aca gct gtt ata ccc att aat ggt tca cct cga aca ccc agg cga ggt 768
cag aac agg agt gca cgg ata gca aaa caa cta gaa aat gat aca aga 816
att att gaa gtt ctc tgt aaa gaa cat gaa tgt aat ata gat gag gtg 864
aaa aat gtt tat ttc aaa aat ttt ata cct ttt atg aat tct ctt gga 912
ctt gta aca tct aat gga ctt cca gag gtt gaa aat ctt tct aaa cga 960
tac gaa gaa att tat ctt aaa aat aaa gat cta gat gca aga tta ttt 1008
ttg gat cat gat aaa act ctt cag act gat tct ata gac agt ttt gaa 1056
aca cag aga aca cca cga aaa agt aac ctt gat gaa gag gtg aat gta 1104
att cct cca cac act cca gtt agg act gtt atg aac act atc caa caa 1152
tta atg atg att tta aat tca gca agt gat caa cct tca gaa aat ctg 1200
att tcc tat ttt aac aac tgc aca gtg aat cca aaa gaa agt ata ctg        1248
aaa aga gtg aag gat ata gga tac atc ttt aaa gag aaa ttt gct aaa        1296
gct gtg gga cag ggt tgt gtc gaa att gga tca cag cga tac aaa ctt        1344
gga gtt cgc ttg tat tac cga gta atg gaa tcc atg ctt aaa tca gaa        1392
gaa gaa cga tta tcc att caa aat ttt agc aaa ctt ctg aat gac aac        1440
att ttt cat atg tct tta ttg gcg tgc gct ctt gag gtt gta atg gcc        1488
aca tat agc aga agt aca tct cag aat ctt gat tct gga aca gat ttg        1536
tct ttc cca tgg att ctg aat gtg ctt aat tta aaa gcc ttt gat ttt        1584
tac aaa gtg atc gaa agt ttt atc aaa gca gaa ggc aac ttg aca aga        1632
gaa atg ata aaa cat tta gaa cga tgt gaa cat cga atc atg gaa tcc        1680
ctt gca tgg ctc tca gat tca cct tta ttt gat ctt att aaa caa tca        1728
aag gac cga gaa gga cca act gat cac ctt gaa tct gct tgt cct ctt        1776
aat ctt cct ctc cag aat aat cac act gca gca gat atg tat ctt tct        1824
cct gta aga tct cca aag aaa aaa ggt tca act acg cgt gta aat tct        1872
act gca aat gca gag aca caa gca acc tca gcc ttc cag acc cag aag        1920
cca ttg aaa tct acc tct ctt tca ctg ttt tat aaa aaa gtg tat cgg        1968
cta gcc tat ctc cgg cta aat aca ctt tgt gaa cgc ctt ctg tct gag 2016
cac cca gaa tta gaa cat atc atc tgg acc ctt ttc cag cac acc ctg 2064
cag aat gag tat gaa ctc atg aga gac agg cat ttg gac caa att atg 2112
atg tgt tcc atg tat ggc ata tgc aaa gtg aag aat ata gac ctt aaa 2160
ttc aaa atc att gta aca gca tac aag gat ctt cct cat gct gtt cag 2208
gag aca ttc aaa cgt gtt ttg atc aaa gaa gag gag tat gat tct att 2256
ata gta ttc tat aac tcg gtc ttc atg cag aga ctg aaa aca aat att 2304
ttg cag tat gct tcc acc agg ccc cct acc ttg tca cca ata cct cac 2352
att cct cga agc cct tac aag ttt cct agt tca ccc tta cgg att cct 2400
gga ggg aac atc tat att tca ccc ctg aag agt cca tat aaa att tca 2448
gaa ggt ctg cca aca cca aca aaa atg act cca aga tca aga atc tta 2496
gta tca att ggt gaa tca ttc ggg act tct gag aag ttc cag aaa ata 2544
aat cag atg gta tgt aac agc gac cgt gtg ctc aaa aga agt gct gaa 2592
gga agc aac cct cct aaa cca ctg aaa aaa cta cgc ttt gat att gaa 2640
gga tca gat gaa gca gat gga agt aaa cat ctc cca gga gag tcc aaa 2688
ttt cag cag aaa ctg gca gaa atg act tct act cga aca cga atg caa          2736
aag cag aaa atg aat gat agc atg gat acc tca aac aag gaa gag aaa          2784
tga                                                                      2787

<210>  57
<211>  928
<212>  PRT
<213>  Homo sapiens

<400>  57

<210>  58
<211>  921

<212>  PRT
<213>  Mus musculus

<220>
<221>  PEPTIDE
<222>  (1)..(921)
<223>  RB_MOUSE Retinoblastoma-associated protein OS=Mus musculus GN=Rb1
       PE=1 SV=2 (P13405)

<400>  58

Met Pro Pro Lys Ala Pro Arg Arg Ala Ala Ala Ala Glu Pro Pro Pro
1               5                   10                  15

Pro Pro Pro Pro Pro Pro Arg Glu Asp Asp Pro Ala Gln Asp Ser Gly
            20                  25                  30

Pro Glu Glu Leu Pro Leu Ala Arg Leu Glu Phe Glu Glu Ile Glu Glu
        35                  40                  45

Pro Glu Phe Ile Ala Leu Cys Gln Lys Leu Lys Val Pro Asp His Val
    50                  55                  60

Arg Glu Arg Ala Trp Leu Thr Trp Glu Lys Val Ser Ser Val Asp Gly
65                  70                  75                  80

Ile Leu Glu Gly Tyr Ile Gln Lys Lys Lys Glu Leu Trp Gly Ile Cys
                85                  90                  95

Ile Phe Ile Ala Ala Val Asp Leu Asp Glu Met Pro Phe Thr Phe Thr
            100                 105                 110

Glu Leu Gln Lys Ser Ile Glu Thr Ser Val Tyr Lys Phe Phe Asp Leu
        115                 120                 125

Leu Lys Glu Ile Asp Thr Ser Thr Lys Val Asp Asn Ala Met Ser Arg
    130                 135                 140

Leu Leu Lys Lys Tyr Asn Val Leu Cys Ala Leu Tyr Ser Lys Leu Glu
145                 150                 155                 160

Arg Thr Cys Glu Leu Ile Tyr Leu Thr Gln Pro Ser Ser Ala Leu Ser
                165                 170                 175

Thr Glu Ile Asn Ser Met Leu Val Leu Lys Ile Ser Trp Ile Thr Phe
            180                 185                 190

Leu Leu Ala Lys Gly Glu Val Leu Gln Met Glu Asp Asp Leu Val Ile
        195                 200                 205

Ser Phe Gln Leu Met Leu Cys Val Val Asp Tyr Phe Ile Lys Phe Ser
    210                 215                 220

Pro Pro Ala Leu Leu Arg Glu Pro Tyr Lys Thr Ala Ala Ile Pro Ile
225                 230                 235                 240

Asn Gly Ser Pro Arg Thr Pro Arg Arg Gly Gln Asn Arg Ser Ala Arg
                245                 250                 255

Ile Ala Lys Gln Leu Glu Asn Asp Thr Arg Ile Ile Glu Val Leu Cys
            260                 265                 270

Lys Glu His Glu Cys Asn Ile Asp Glu Val Lys Asn Val Tyr Phe Lys
        275                 280                 285

Asn Phe Ile Pro Phe Ile Asn Ser Leu Gly Ile Val Ser Ser Asn Gly
    290                 295                 300

Leu Pro Glu Val Glu Ser Leu Ser Lys Arg Tyr Glu Glu Val Tyr Leu
305                 310                 315                 320

Lys Asn Lys Asp Leu Asp Ala Arg Leu Phe Leu Asp His Asp Lys Thr
                325                 330                 335

Leu Gln Thr Asp Pro Ile Asp Ser Phe Glu Thr Glu Arg Thr Pro Arg
            340                 345                 350

Lys Asn Asn Pro Asp Glu Glu Ala Asn Val Val Thr Pro His Thr Pro
        355                 360                 365

Val Arg Thr Val Met Asn Thr Ile Gln Gln Leu Met Val Ile Leu Asn
    370                 375                 380

Ser Ala Ser Asp Gln Pro Ser Glu Asn Leu Ile Ser Tyr Phe Asn Asn
385                 390                 395                 400

Cys Thr Val Asn Pro Lys Glu Asn Ile Leu Lys Arg Val Lys Asp Val
                405                 410                 415

Gly His Ile Phe Lys Glu Lys Phe Ala Asn Ala Val Gly Gln Gly Cys
            420                 425                 430

Val Asp Ile Gly Val Gln Arg Tyr Lys Leu Gly Val Arg Leu Tyr Tyr
        435                 440                 445

Arg Val Met Glu Ser Met Leu Lys Ser Glu Glu Glu Arg Leu Ser Ile
    450                 455                 460

Gln Asn Phe Ser Lys Leu Leu Asn Asp Asn Ile Phe His Met Ser Leu
465                 470                 475                 480

Leu Ala Cys Ala Leu Glu Val Val Met Ala Thr Tyr Ser Arg Ser Thr
                485                 490                 495

Leu Gln His Leu Asp Ser Gly Thr Asp Leu Ser Phe Pro Trp Ile Leu
            500                 505                 510

Asn Val Leu Asn Leu Lys Ala Phe Asp Phe Tyr Lys Val Ile Glu Ser
        515                 520                 525

Phe Ile Lys Val Glu Ala Asn Leu Thr Arg Glu Met Ile Lys His Leu
    530                 535                 540

Glu Arg Cys Glu His Arg Ile Met Glu Ser Leu Ala Trp Leu Ser Asp
545                 550                 555                 560

Ser Pro Leu Phe Asp Leu Ile Lys Gln Ser Lys Asp Gly Glu Gly Pro
                565                 570                 575

Asp Asn Leu Glu Pro Ala Cys Pro Leu Ser Leu Pro Leu Gln Gly Asn
            580                 585                 590

His Thr Ala Ala Asp Met Tyr Leu Ser Pro Leu Arg Ser Pro Lys Lys
        595                 600                 605

Arg Thr Ser Thr Thr Arg Val Asn Ser Ala Ala Asn Thr Glu Thr Gln
    610                 615                 620

Ala Ala Ser Ala Phe His Thr Gln Lys Pro Leu Lys Ser Thr Ser Leu
625                 630                 635                 640

Ala Leu Phe Tyr Lys Lys Val Tyr Arg Leu Ala Tyr Leu Arg Leu Asn
                645                 650                 655

Thr Leu Cys Ala Arg Leu Leu Ser Asp His Pro Glu Leu Glu His Ile
            660                 665                 670

Ile Trp Thr Leu Phe Gln His Thr Leu Gln Asn Glu Tyr Glu Leu Met
        675                 680                 685

Arg Asp Arg His Leu Asp Gln Ile Met Met Cys Ser Met Tyr Gly Ile
    690                 695                 700

Cys Lys Val Lys Asn Ile Asp Leu Lys Phe Lys Ile Ile Val Thr Ala

705                 710                 715                 720

Tyr Lys Asp Leu Pro His Ala Ala Gln Glu Thr Phe Lys Arg Val Leu
                725                 730                 735

Ile Arg Glu Glu Glu Phe Asp Ser Ile Ile Val Phe Tyr Asn Ser Val
            740                 745                 750

Phe Met Gln Arg Leu Lys Thr Asn Ile Leu Gln Tyr Ala Ser Thr Arg
        755                 760                 765

Pro Pro Thr Leu Ser Pro Ile Pro His Ile Pro Arg Ser Pro Tyr Lys
    770                 775                 780

Phe Ser Ser Ser Pro Leu Arg Ile Pro Gly Gly Asn Ile Tyr Ile Ser
785                 790                 795                 800

Pro Leu Lys Ser Pro Tyr Lys Ile Ser Glu Gly Leu Pro Thr Pro Thr
                805                 810                 815

Lys Met Thr Pro Arg Ser Arg Ile Leu Val Ser Ile Gly Glu Ser Phe
            820                 825                 830

Gly Thr Ser Glu Lys Phe Gln Lys Ile Asn Gln Met Val Cys Asn Ser
        835                 840                 845

Asp Arg Val Leu Lys Arg Ser Ala Glu Gly Gly Asn Pro Pro Lys Pro
    850                 855                 860

Leu Lys Lys Leu Arg Phe Asp Ile Glu Gly Ala Asp Glu Ala Asp Gly
865                 870                 875                 880

Ser Lys His Leu Pro Ala Glu Ser Lys Phe Gln Gln Lys Leu Ala Glu
                885                 890                 895

Met Thr Ser Thr Arg Thr Arg Met Gln Lys Gln Arg Met Asn Glu Ser
            900                 905                 910

Lys Asp Val Ser Asn Lys Glu Glu Lys
        915                 920

<210>  59
<211>  410
<212>  PRT
<213>  Mus musculus

<220>
<221>  peptide

<222>  (1)..(410)
<223>  TFDP1_MOUSE Transcription factor Dp-1 OS=Mus musculus GN=Tfdp1
       PE=1 SV=1 (Q08639)

<400>  59
   Met Ala Lys Asp Ala Ser Leu Ile Glu Ala Asn Gly Glu Leu Lys Val
   1               5                   10                  15
   Phe Ile Asp Gln Asn Leu Ser Pro Gly Lys Gly Val Val Ser Leu Val
               20                  25                  30
   Ala Val His Pro Ser Thr Val Asn Thr Leu Gly Lys Gln Leu Leu Pro
           35                  40                  45
   Lys Thr Phe Gly Gln Ser Asn Val Asn Ile Thr Gln Gln Val Val Ile
       50                  55                  60
   Gly Thr Pro Gln Arg Pro Ala Ala Ser Asn Thr Ile Val Val Gly Ser
   65                  70                  75                  80
   Pro His Thr Pro Asn Thr His Phe Val Ser Gln Asn Gln Thr Ser Asp
                   85                  90                  95
   Ser Ser Pro Trp Ser Ala Gly Lys Arg Asn Arg Lys Gly Glu Lys Asn
               100                 105                 110
   Gly Lys Gly Leu Arg His Phe Ser Met Lys Val Cys Glu Lys Val Gln
           115                 120                 125
   Arg Lys Gly Thr Thr Ser Tyr Asn Glu Val Ala Asp Glu Leu Val Ala
       130                 135                 140
   Glu Phe Ser Ala Ala Asp Asn His Ile Leu Pro Asn Glu Ser Ala Tyr
   145                 150                 155                 160
   Asp Gln Lys Asn Ile Arg Arg Arg Val Tyr Asp Ala Leu Asn Val Leu
                   165                 170                 175
   Met Ala Met Asn Ile Ile Ser Lys Glu Lys Lys Glu Ile Lys Trp Ile
               180                 185                 190
   Gly Leu Pro Thr Asn Ser Ala Gln Glu Cys Gln Asn Leu Glu Val Glu
           195                 200                 205
   Arg Gln Arg Arg Leu Glu Arg Ile Lys Gln Lys Gln Ser Gln Leu Gln
       210                 215                 220
   Glu Leu Ile Leu Gln Gln Ile Ala Phe Lys Asn Leu Val Gln Arg Asn
   225                 230                 235                 240
   Arg Gln Ala Glu Gln Gln Ala Arg Arg Pro Pro Pro Pro Asn Ser Val
                   245                 250                 255
   Ile His Leu Pro Phe Ile Ile Val Asn Thr Ser Arg Lys Thr Val Ile
               260                 265                 270
   Asp Cys Ser Ile Ser Asn Asp Lys Phe Glu Tyr Leu Phe Asn Phe Asp
           275                 280                 285
   Asn Thr Phe Glu Ile His Asp Asp Ile Glu Val Leu Lys Arg Met Gly
       290                 295                 300
   Met Ala Cys Gly Leu Glu Ser Gly Asn Cys Ser Ala Glu Asp Leu Lys
   305                 310                 315                 320
   Val Ala Arg Ser Leu Val Pro Lys Ala Leu Glu Pro Tyr Val Thr Glu
                   325                 330                 335
   Met Ala Gln Gly Ser Ile Gly Gly Val Phe Val Thr Thr Thr Gly Ser
               340                 345                 350
   Thr Ser Asn Gly Thr Arg Leu Ser Ala Ser Asp Leu Ser Asn Gly Ala
           355                 360                 365
   Asp Gly Met Leu Ala Thr Ser Ser Asn Gly Ser Gln Tyr Ser Gly Ser
       370                 375                 380
   Arg Val Glu Thr Pro Val Ser Tyr Val Gly Glu Asp Asp Asp Asp Asp
   385                 390                 395                 400
   Asp Asp Phe Asn Glu Asn Asp Glu Glu Asp
                   405                 410

## Description

### TECHNICAL FIELD

The invention relates to the field of searching for compounds useful for the control of cancer and their therapeutic use. More particularly, it refers to identification of compounds as drug candidates among those able to trigger phosphorylation of retinoblastoma protein in some particular positions and/or those that mimic the effect of such phosphorylation.

### BACKGROUND OF THE INVENTION

The primary regulator of the G1/S-phase transition in mammalian cells is the Retinoblastoma (RB) tumour suppressor protein and its homologues p107 and p130, known as the pocket protein family (Cobrinik, 2005; Henley and Dick, 2012).

In its active state, (which has been traditionally considered to be the hypophosphorylated state), RB prevents transcription of genes required for S-phase progression by interacting with and repressing E2F transcription factors, which are dimeric proteins containing E2F and differentiation-related polypeptide (DP) subunits (Dick and Rubin, 2013). The term E2F is used to refer to a family of transcription factors (TF) that play a major role during the G1/S transition in mammalian and plant cell cycle. Replication proteins, DNA repair proteins, checkpoint regulators, cyclins and CDKs are some of E2F transcriptional targets. Some members of the family are transcription activators (E2F1, E2F2, E2F3), which promote cell cycle, while other members (E2F4-8) are repressors of the cell cycle. The balance between repressor and activator E2F regulates cell cycle progression. All E2F family members have similar domains that allow them to bind DP1 or DP2 proteins, what provides a second DNA binding site and increases E2F binding stability. Most E2F proteins also have a pocket protein binding domain, which allows binding with pocket proteins such as RB when the latter is hypophosphorylated. Cdk4(6)/cyclin D and Cdk2/cyclin E are known to phosphorylate RB and related pocket proteins, allowing them to dissociate from E2F. In the absence of RB, activator E2F proteins, such as E2F1 (together with its binding partner DP1), can mediate the transcription of E2F1 target genes (such as those of cyclins E and A, E2F1, E2F2, E2F3, Cyclin-Dependent Kinase 1, c-Myc, DHFR, PCNA or H2A) that facilitate G1/S transition and S- phase promoting genes.

RB is a highly conserved protein among mammals that shows a high percentage of identity between the human RB protein and the RB proteins of other mammals (for instance *Homo sapiens vs Mus musculus,* identities=90%; *Homo sapiens vs Rattus norvegicus,* identities=89%; *Homo sapiens vs Canis lupus familiaris,* identities=94%; *Homo sapiens vs Bos taurus* (identities=93%), and *Homo sapiens vs Sus scrofa,* identities=95%). The domain structure of the protein is also conserved among mammals, as well as the phosphorylation sites.

RB has three structured domains: the central domain named the "pocket", the amino-terminal domain (RBN) and the carboxy-terminal domain (RBC). RB structure allocates conserved interaction surfaces and binds several regulatory proteins. RB binds the E2Fs through a highly conserved region of the pocket domain (Lee et al., 2002; Xiao et al., 2003). The RBC domain is the location of a second E2Fs interaction surface, the DP heterodimerization partner of E2F binding, and kinase and phosphatase docking sites (Rubin et al., 2005; Hirschi et al., 2010). The linker sequences of RB contain approximately 13 conserved CDK-dependent phosphorylation consensus sites that have a pivotal role for the inactivation of RB (Hassler et al., 2007; Burke et al., 2010; Burke et al., 2012).

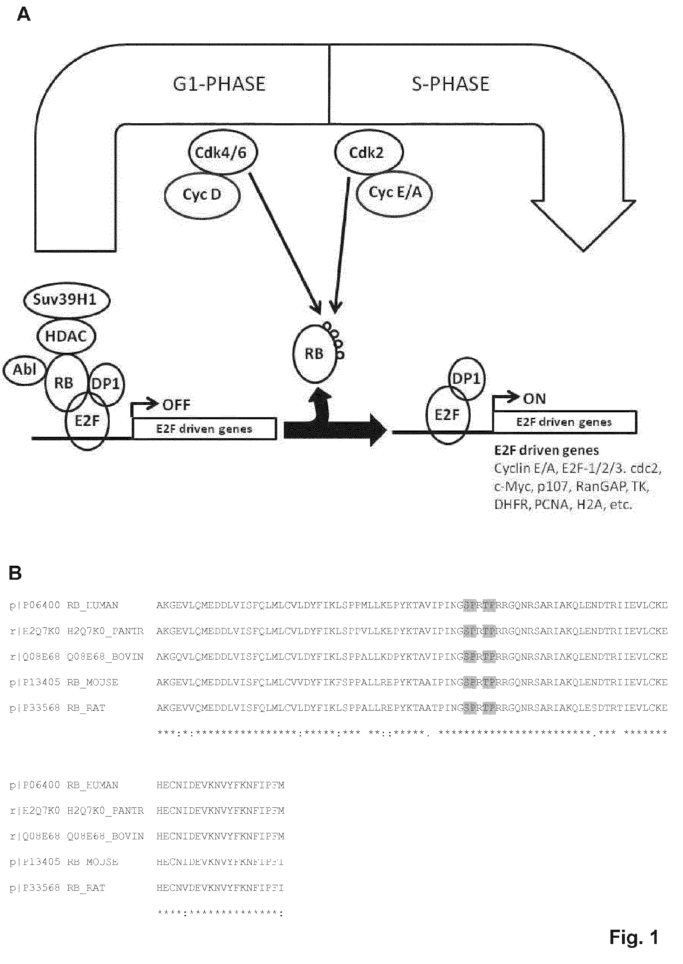

The RBN domain, in turn, contains positions that have been considered Cdk phosphorylation sites, such as Serine 249 and/or Threonine 252, abbreviated Ser249/Thr252 or simply S249/T252, in the RB of *Homo sapiens* and also *Pan troglodytes* (chimpanzee); in other mammals, as can be seen in Fig. 1B, the same phosphorylation sites can be also found in the RBN domain, in the same or in equivalent positions (for instance, as amino acids 243 (Serine) to 246 (Threonine) in *Mus musculus* RB protein), also being part of SP (Serine-Proline) or TP (Threonine-Proline) consensus phosphorylation sites that are close one to the other. Fig. 1B shows the high degree of conservation among the RB of different mammals in that part of the RBN domain. However, the structural and physiological effects of said phosphorylation are less well understood that those related to the RBC and the pocket domain. Thus, for instance, European Patent Application EP-1156332-A2 proposes the measuring of the level of phosphorylation of RB in Ser249/Thr252 as a way of measuring CDK activity in cultured cells. The application also mentions methods of identifying agents that modulate CDK activity as those able to change the level of CDK-phosphorylated RB in a sample, but no assay is described where the phosphorylation of Ser249/Thr252 is measured: only antibodies specifically recognizing RB phosphorylated at Ser807 and Ser811 (related to CDK2 activity) or phosphorylated at Ser780 (related to CDK4 activity) are used in the assay carried out with a carcinoma cell line. In said Example, RB activity itself is not measured, only phosphorylation *in vitro* assays are described; the increase in phosphorylation is associated with an increase in CDK activity, concluding that it is possible to identify as CDK inhibitors those compounds that decrease the degree of phosphorylation in Ser807, Ser811 and/or Ser780, being no specific assay or discussion about the possible effects of Ser249/Thr252 phosphorylation. No *in vivo* assay is described either.

Other authors have reported that phosphorylation of Ser249 and/or Thr252 in the RBN fragment, expressed in *in vitro* conditions, inhibits the interaction of RB with the E1A-like inhibitor of differentiation, EID1 (Hassler et al., 2007). This effect has been mentioned in reviews about RB phosphorylation (Rubin, 2013), where it was also stated that S259/T252 phosphorylation has no effect on E2F inhibition, as it had been described previously by other groups (Burke et al., 2010; Burke et al., 2012). The assays leading to such conclusion were all carried out *in vitro* and by assessing the interactions of purified proteins with an RB fragment, namely the fragment RBN, but not with the whole RB protein. Thus, no evidence pointed out whether this interactions have any physiological role *in vivo,* such as any effect on cellular proliferation.

In general, RB is considered to be inactivated by sequential phosphorylations by CDK complexes to elicit cyclin expression and promote entry into S-phase. It has been demonstrated by structural analysis that phosphorylation events induce unique conformations in RB, each capable of making different protein interactions. RB phosphorylated by both Cdk4-cyclinD and Cdk2-cyclinE causes a conformational change and the release of the bound E2Fs, allowing for an up-regulation of E2F target gene expression (Rubin, 2013; Munro et al., 2012). RB has an important role in recruiting chromatin regulators and CDK phosphorylation also disrupts RB interactions with them (Talluri and Dick, 2012). In mitosis, RB returns to a hypophosphorylated state (the state traditionally considered its active state) through the PP1 phosphatase and repress E2Fs in the ensuing G1-phase (Kolupaeva and Janssens, 2013), thus controlling G1/S transition and preventing the progression to S-phase (see Fig. 1).

The control of the G1/S transition by RB tumour suppressor is critical for cell proliferation and its inactivation is one of the most fundamental events in cancer. The majority of cancers find a way to impair RB function. More than 80% of human cancers harbour mutations in the RB/Cdk/INK pathway that controls progression of the cell cycle. Although in some cases direct mutation of the RB gene itself exists, usually there is an altered expression of RB regulators such as cyclin D, CDK4 and CDK6 activities (Burkhart and Sage, 2008; Stone et al., 2012; Manning and Dyson, 2012; Hanahan and Weinberg, 2011; Chinnam and Goodrich, 2011) or inactivation of CDK regulators such as the product of the INK4/Arf locus p16 (Gonzalez S and Serrano M, 2006). Therefore, there is a deep interest to improve therapeutic Cdk inhibitors that prevent RB inactivation. It is then crucial a better understanding of RB activity to afford fundamental insights into the biology of tumour initiation and progression. Several cellular signals, such as mitogenic signals, DNA damage or AMP-activated kinase, seem to modulate RB functions (Takahashi et al., 2012).

In response to stress, cells delay cell cycle progression to induce adaptive responses and maximize cell survival (de Nadal et al., 2011; Saito and Posas 2012; Duch et al., 2013). In mammals, Stress-Activated Protein Kinases (SAPKs) play a key role in controlling different cell cycle checkpoints (Ambrosino and Nebreda, 2001; Bulavin and Fornace, 2004). A prototypical family of SAPKs is p38 which is activated by external stimuli such as cytokines, oxidative stress, heat shock, DNA damage and osmostress. p38 activity regulates several biological processes from immune response, inflammation and development to proliferation and survival (reviewed in Cuadrado and Nebreda, 2010). p38 has been also considered to act as a tumour suppressor capable of controlling cell proliferation through the activation of different cell cycle checkpoints. It has been implicated in G2/M and G1/S phases arrest upon several stimuli (Joaquin et al., 2012a). In G1, p38 targets the p57Kip2 CDK inhibitor to inhibit Cyclin A/Cdk2 activity, which delays cell cycle and increases cell survival upon stress (Joaquin et al., 2012b). In yeast, cells control several phases of the cell cycle in response to osmostress, including G1/S. The p38-orthologous Hog1 controls the G1/S transition in response to stress by a combined action over the Sic1 CDK inhibitor and the control of cyclin expression by phosphorylating the Msa1/2 transcription factors and the Whi5 repressor (Escote et al., 2004; Adrover et al., 2011; Gonzalez-Novo et al., 2015).

Currently, there are several compounds in trial, in phase of approval or approved in countries like the US, which compounds target G1/S transition, particularly in the RB/CDK/INK pathway. Many of them are specifically inhibitors or inactivators of CDKs: Palbociclib (commercialized as Ibrance^{®} by Pfizer: http://www.ibrance.com/), which is the first drug with a mechanism confirmed to be dependent on RB, but which does not act directly on said protein, but by inactivating CDKs, as well as several inhibitors of CDK4/6 or CDKs in general; Abemaciclib (LY2835219, of Lilly: https://inv,stor.lilly.com/releasedetail.cfm?ReleaselD=935735), LEE011 of Novartis), Dinaiciclib (SCH-727965, of MSD), Cyc202 (R-roscovitine, of Cyclacel) and Cyc065 (a derivative of seliciclib, also of Cyclacel).

As all of the above mentioned drugs inhibit the CDKs, they might exhibit selectivity problems and undesired side effects on other targets (see, for instance, information about the side effects of Abemaciclib in The Asco Post of 1 May 2014: http://www.ascopost.com/issues/may-1,-2014/ly2835219-shows-strong-single-agent-activity-in-preliminary-study-in-metastatic-breast-cancer.aspx, or the information about Palbociclib side effects on the portal web of Ibrance®: http://www.ibrance.com/). Such adverse effects are not surprising, because CDKs play further roles in addition to the control of cyclin expression. Undesired unspecific off target effects versus other kinases are also expectable for CDK inhibitors, especially for small molecules. Also, the use of kinase inhibitors such as these used for ERKs results in lack in effect in many cancer types after short periods of time due to elimination of feed-back regulated loops (Solit and Rosen, 2011).

Therefore, it would be advisable to identify potential anticancer drugs that do not target CDKs directly. However, given the high percentage of different cancers where the control of the transition G1/S is altered, it should be also advisable to keep on searching for potential anticancer drugs among compounds capable of acting on one or more factors involved in the transition G1/S or its control, thus having a potential effect in a large panel of tumours.

Thus, it would be interesting to identify potential antitumor drugs having RB protein as a direct target, and not acting indirectly on RB through the inactivation of CDKs. To that aim, it would be very convenient to have available a method allowing an easy identification of compounds able to decrease or prevent the proliferation of cancer cells, by acting directly on RB and, particularly, on its conformation.

Recently, the group of Dr. Rubin (Pye et al., 2016) has proposed a fluorescence polarization assay that, in principle, has been designed for screening molecules that modulate the binding between RB and E2F. The approach is based on the fact that phosphorylation of Threonine 373 of human RB increases the affinity of the N-terminal domain of RB towards the pocket domain of RB itself, thus reducing the affinity of the pocket domain of RB towards the E2F transactivation domain, the domain that binds the pocket domain in RB. The group of Dr. Rubin set up an *in vitro* biochemical screen using a small part of the E2F, only of 20 amino acids, that binds to the RB pocket domain (aa 409-428 of E2F; E2F^{TD} peptide, with a fluorescent dye at its N terminus, E2F^{TMR}), and also using a RB protein construct that contains the RB N-terminal domain and the pocket domain but lacks internal loops in each domain (a fragment that is called RB^{NP}). Thus, those molecules that increase the fluorescence polarization ratio (the "hits"), would be causing enhanced RB binding and, therefore, inducing E2F^{TMR} aggregation. However, after a pilot screen with a 21120 small molecule library, only four triazene compounds were validated, whose activity was lost in the presence of catalase, so they are not further pursued.

Subsequently, the same researchers included in the same experimental system a peptide based on the peptides used by viruses, such as protein E7 of HPV, in order to disrupt the interaction between RB and E2F: as it is already known (Burke et al., 2012), the binding of such peptides will not allow RB to change conformation, again preventing the intramolecular interaction of two domains of RB, and then also preventing alosterical opening of the E2F^{TD} binding cleft to weaken affinity. Thus, such peptides basically serve to prevent the effect caused by T373 phosphorylation by CDKs: they prevent the intramolecular interaction of two domains within RB and, as a consequence, they also prevent the inhibition of E2F binding to RB that occurs upon T373 phosphorylation. Accordingly, the fluorescence polarization results indicated that the presence of the peptide (full length E7 or an E7 peptide that is also called LxCxE peptide) was able to alter the association of E2F^{TD} to the RB^{NP} pocket domain, but only when the RB^{NP} fragment was phosphorylated at T373, basically because it is then when such peptide can prevent the action of T373 phosphorylation. It is worth to mention that other LxCxEx peptides different from E7 protein, such as LxCxE-like peptides from Cyclin D or LIN52, do not have the same effect on E2F^{TD}/RB^{NP} binding. Moreover, it is not demonstrated that the E7 LxCxE peptide would render similar results with the full length RB. Thus, even when Pye et al. state that they have developed an assay that can detect the effect of exogenous compounds on modulating affinity of RB for the E2F transactivation domain, it is not clear that said assay can be used to screen for therapeutic compounds, because it is not carried out with full length proteins, but with RB and E2F fragments which did not recapitulate *in vivo* conditions of interactions, and because it needs RB phosphorylated at T373 in order to appreciate changes in E2F affinity for RB.

Thus, although some interesting approaches have been proposed, there is still a need of developing effective methods to easily identify potential antitumor drugs having RB protein as a direct target, which compounds act directly on RB and, particularly, on its conformation. Preferably, the method should be designed so that complete RB is used, thus recapitulating *in vivo* conditions, not being designed for the use of combinations of RB portions lacking structural parts such as the loops.

The present invention provides a solution to said problem.

### SUMMARY OF THE INVENTION

The present invention is based on the findings of the present inventors, disclosed in the present application, that show that, in mammals, stress causes down-regulation of cyclin expression that is mediated by the phosphorylation of retinoblastoma protein (RB) by p38. This phosphorylation occurs in two specific sites at the N-terminal region of RB, which are Serine 249 (Ser249) and Threonine 252 (Thr252) in human RB protein. Ser249/Thr252 phosphorylation leads to a conformational change of RB that increases the affinity of RB for E2F, triggers a stronger repressor activity and makes RB insensitive to CDK inactivation. Ser249/Thr252 phosphorylated by RB, or mutations that mimic said pair of phosphorylations, promote a cell cycle delay which reduces cell proliferation in cancer cells. Thus, and very remarkably, RB phosphorylation by p38, or mutations that mimic it, yield an RB insensitive to CDK inactivation that is able to block cell proliferation in typical cancer cells that contain high CDK activity. Therefore:
- p38 phosphorylation increases RB affinity to E2F and represses E2F-dependent gene expression.
- Phosphorylated RB delays entry into S-phase and increases cell survival upon stress.
- p38 phosphorylation results in an RB insensitive to CDK inactivation.
- Mutations mimicking RB phosphorylation prevent cell proliferation in cancer cells.

The present invention relates to a method for identifying compounds as candidates for control of cancer among those that provoke the phosphorylation of human RB protein in Ser249 and Thr252 and/or those that provoke the same effect of such phosphorylation, that is, an increase of RB affinity to E2F that represses the expression of those genes depending on E2F and/or renders RB insensitive to CDK inactivation. The present invention also relates to expression vectors of a mutant retinoblastoma protein with mutations that mimic the phosphorylation at the mentioned amino acid residues.

Therefore, in a first aspect, the present invention refers to a method for identifying a compound as a candidate to drug for the control of cancer and/or the decrease of cell growth proliferation that comprises the steps of:
a) contacting a cell culture with the compound, under suitable conditions and during enough time for having an interaction between the compound and the cells;
b) determining and quantifying a property or process that is affected by the level of phosphorylation of human RB protein in the serine amino acid residue of position 249 and the threonine amino acid residue of position 252 (Ser249/Thr252) and/or by those that provoke the same effect of such phosphorylation, which property or process is selected from the group of:
   i. the affinity of RB for E2F,
   ii. the level of transcription of E2F,
   iii. the level of expression of a gene that is under the transcriptional control or operatively linked to an E2F-dependent promoter and that depends on the binding of the E2F transcription factor for being transcribed,
   iv. the level of phosphorylation of human RB in the serine amino acid residue of position 249 and/or the threonine amino acid residue of position 252 (Ser249 and/orThr252) itself, or the level of phosphorylation of the equivalent serine and threonine residues of the N-terminal domain of the retinoblastoma protein of another mammal,
   v. the binding of RB to the E2F-dependent promoters,
   vi. the binding of RNA Pol II to the E2F-dependent promoters, and
   vii. the effect on cell growth proliferation
   or combinations thereof;
c) comparing the level of the property or process assessed in step b) with the level property determined in a control cell culture of the same cells, that have been subjected to the same conditions that the treated cells of step a) for the fact that the cells have not been contacted with the assayed compound;
d) identifying the compound as a candidate to drug for the control of cancer if the level of the assessed property or process has changed in the same way as it changes when the level of phosphorylation of Ser249/Thr252 increases, that is, if:
   i. the affinity of RB for E2F is increased in the treated cells,
   ii. the level of transcription of E2F is decreased in the treated cells,
   iii. the level of expression of the assayed gene that is under the transcriptional control or operatively linked to an E2F-dependent promoter and that depends on the binding of the E2F transcription factor, is decreased in the treated cells,
   iv. the level of phosphorylation of Ser249 and/or Thr252 of human RB protein, or the level of phosphorylation of the equivalent serine and threonine residues of the N-terminal domain of the retinoblastoma protein of another mammal, is increased in the treated cells,
   v. the binding of RB to the E2F-dependent promoters is increased,
   vi. the binding of RNA Pol II to the E2F-dependent promoters is decreased, and/or
   vii. the cell growth proliferation is decreased
or combinations thereof.

It is a preferred embodiment of the method of the present invention a method that comprises a previous step wherein the cells to be contacted with the assayed compound and the cells of the control cell culture are transfected with an expression vector wherein the coding sequence of an indicator gene is operatively linked to a promoter that requires binding of E2F transcription factor for driving the transcription of the gene and the assayed compound is identify as a candidate to drug for the control of cancer if the level of expression of the indicator gene is decreased in the cell that have been treated with the assayed compound with regard to the control cells.

In a second aspect, the invention relates to an expression vector of a mammalian retinoblastoma mutant protein wherein both the serine and the threonine residues of the consensus phosphorylation sites Serine-Proline (SP) and Threonine-Proline (TP) of the N-terminal domain which are or that can be considered equivalent to the Serine249 and the Threonine252 of the human RB protein, are replaced by a glutamic acid residue, for use in the treatment of cancer in a mammal. Preferably, the mammal is a human being, the mammalian retinoblastoma protein is the human protein and serine249 and threonine 252 are replaced each of them by a residue of glutamic acid.

### BRIEF DESCRIPTION OF THE FIGURES

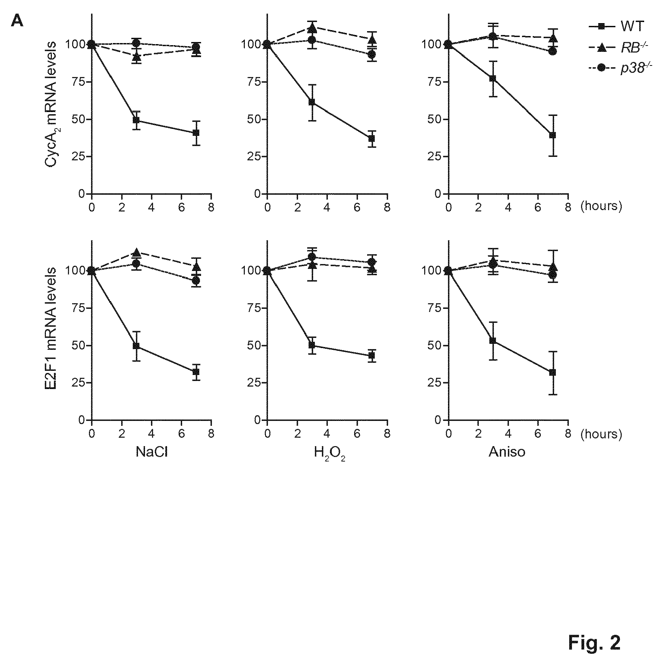

**Fig. 1****:** Retinoblastoma protein: activity and RNB domain
   - Panel A: Schematic representation of the control of G1/S transition by Retinoblasoma (RB) and its regulation by Cyclin-Dependent Kinases (CDK) activity. RB is considered to be inactivated by sequential phosphorylations by CDK complexes to elicit cyclin expression and promote entry into S-phase. The proteins involved in the repression of the transcriptional complex formed by E2F and DP1 are shown (Abl: Abelson murine leukemia viral oncogene homolog; HDAC: hystone deacetylase; Suv39H1: Histone-lysine N-methyltransferase SUV39H1). Cyclin E/A, E2F-1/2/3, cyclin-dependent kinase 1 (the product of CDK1 gene, indicated in the figure as cdc2, a common abbreviation for the corresponding protein), c-Myc, p107, Ran GTPase-activating protein (RanGAP), thymidine kinase (TK), dihydrofolate reductase (DHFR), proliferating cell nuclear antigen (PCNA) and histone H2A are indicated in the figure among the genes whose transcription is mediated by the E2F-DP1 complex. The arrows starting at the complexes CyclinD-CDK4/6 and CyclinE/A-CDK2 indicate the phosphorylating activity of the corresponding cyclin-dependent kinases (CDKs) on RB (phosphorylation represented as small circles). The position of the mentioned Cyclin-CDKs complexes next to the labels of G1-phase or S-phase, respectively, indicates the cell cycle phase when these complexes phosphorylate RB.
   - Panel B: Clustal W Multiple Sequence alignment of amino acids 201-300 of human (P0400), chimpanzee (H2Q7K0), cow (Q08E68), mouse (P13405) and rat (P33568) RB1 was performed with T-Coffee, a web server for the multiple sequence alignment of protein and RNA sequences using structural information and homology extension (Di Tommaso et al, 2011). The SP and TP sites (shaded) identified in human RB1 are conserved throughout the mammalian class. (asterisk, *) indicates positions which have a single, fully conserved residue. (colon, :) indicates conservation between groups of strongly similar properties. (period, .) indicates conservation between groups of weakly similar properties
**Fig. 2****:** Down-regulation of cyclin expression upon stress is mediated by p38 SAPK and RB.
   - Panel A: E2F-dependent target genes down-regulation upon stress is compromised in RB^{-/-} and p38^{-/-} knockout cells. Wild type (WT) (results indicated with squares), RB^{-/-} (results indicated with triangles) and p38^{-/-} (results indicated with circles) MEFs (Mouse Embryonic Fibroblasts) were stressed with 100 mM NaCl, 400 µM H₂O₂ or 25 ng/ml anisomycin (indicated as "Aniso") for the indicated length of time as indicated in the X-axis. mRNA levels of CycA₂ (cyclin A2) and E2F1 were analyzed by real time PCR. GAPDH mRNA was used as loading control. Data are represented as mean ±SEM.

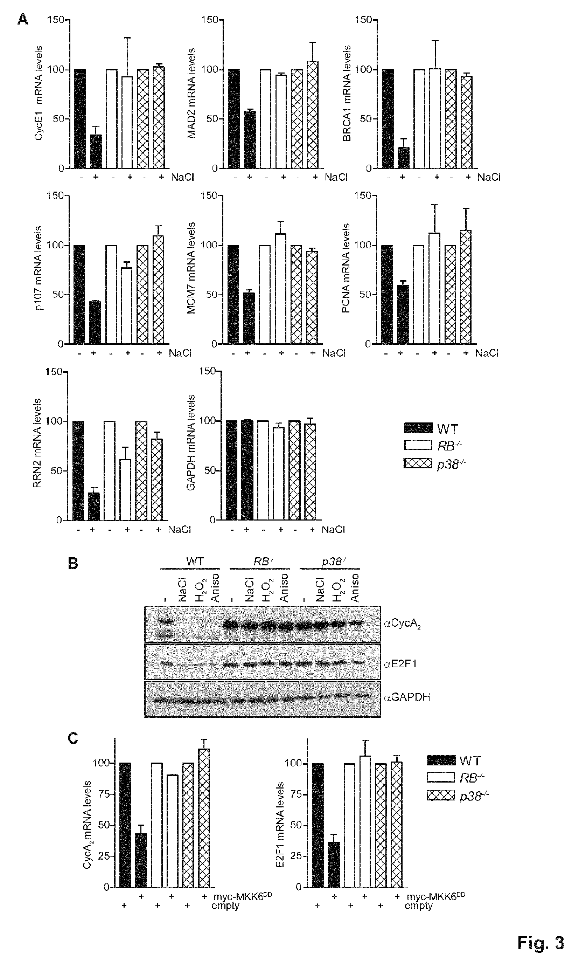

**Fig. 3****:** E2F-dependent target genes down-regulation upon osmostress or upon genetically activated p38 (MKK6^{DD}: constitutively activated MKK6 kinase) is compromised in RB^{-/-} cells
   - Panel A: Wild type (WT) (results indicated with black bars), RB^{-/-} (results indicated with white bars) and p38^{-/-} (results indicated with scratch bars) MEFs cells were stressed (bars labelled with "+" in the X-axis) or not (bars labelled with "-" in the X-axis) with 100 mM NaCl for 7 hours. The mRNA levels of CycE1 (cyclin E1), MAD2, BRCA1, p107, MCM7, PCNA and RRN2 were analyzed by real time PCR. GAPDH mRNA was used as loading control. Data are represented as mean ±SEM.
   - Panel B: Protein down-regulation of E2F-dependent target genes upon stress is compromised in RB^{-/-} and p38^{-/-} cells. Wild type (WT), RB^{-/-} and p38^{-/-} MEFs were stressed with 100 mM NaCl, 400 µM H₂O₂ or 25 ng/ml anisomycin (indicated as "Aniso") or not stressed ("-" signs over the lane) and cells lysates were analyzed by Western blot using specific CycA₂ (cyclin A2) and E2F1 antibodies. GAPDH protein levels were used as loading control.
   - Panel C: E2F-dependent target genes down-regulation upon genetic p38 activation is compromised in RB^{-/-} and p38^{-/-} cells. Wild type (WT) (results indicated with black bars), RB^{-/-} (results indicated with white bars) and p38^{-/-} (results indicated with scratch bars) MEFs cells were transfected with myc-MKK6^{DD} or a control plasmid (empty). The mRNA levels of CycA₂ (cyclin A2) and E2F1 were analyzed by real time PCR. GAPDH mRNA was used as loading control. Data are represented as mean ±SEM.

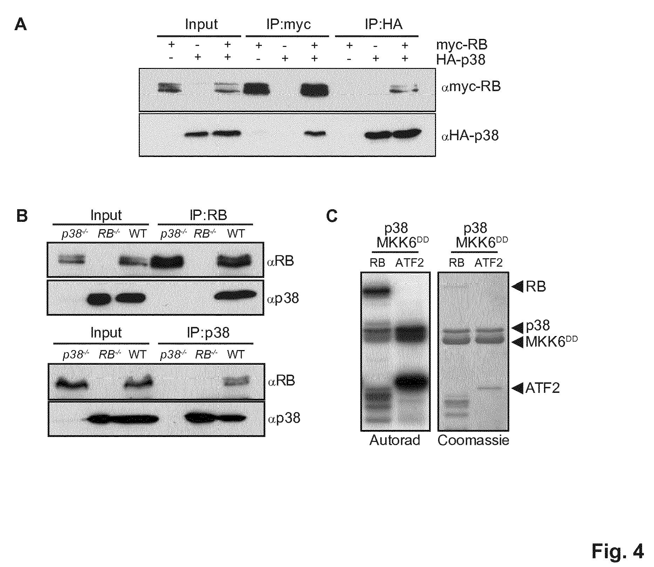

**Fig. 4****:** p38 interacts with and phosphorylates RB
   - Panel A: RB and p38 form a stable complex in cultured cells. myc-RB and HA-p38 were expressed on HEK293T cells. Cell lysates (indicated as "Input") expressing ("+" signs over the lane) or not ("-" signs over the lane) Myc-tagged RB and/or HA-tagged p38 were immunoprecipitated with either anti-myc (indicated as "IP:myc) or anti-HA (indicated as IP:HA) sepharose beads. Cell lysates and immunoprecipitated were analyzed by Western blot using antibodies anti-myc to detect RB and anti-HA to detect p38 (indicated in the right).
   - Panel B: RB and p38 form a stable complex in cultured cells. Cell lysates (indicated as "Input") from wild type (WT), RB^{-/-} and p38^{-/-} MEFs were immunoprecipitated with anti-RB (upper panels) or anti-p38 (down panels) sepharose beads. Cell lysates and immunoprecipitated were analyzed by Western blot using antibodies specific RB and p38 antibodies (indicated in the right).
   - Panel C: p38 phosphorylates RB *in vitro.* GST-RB, GST-p38, GST-MKK6^{DD} and GST-ATF2 were purified from *E. coli* and used in an *in vitro* kinase assay. Purified GST-p38 was activated with GST-MKK6^{DD} in the presence of cold ATP and used to phosphorylate GST- RB and GST-ATF2 in the presence of radiolabelled ³²P-γ-ATP. Coomassie panel shows the total amount of loaded proteins; autoradiography (indicated as "Autorad") panel shows the amount of γ-³²P incorporated on each protein. ATF2, a well-known p38 substrate, was used as a positive control. The position of the bands corresponding to RB, ATF2 and p38/MKK6^{DD} are indicated on the right.

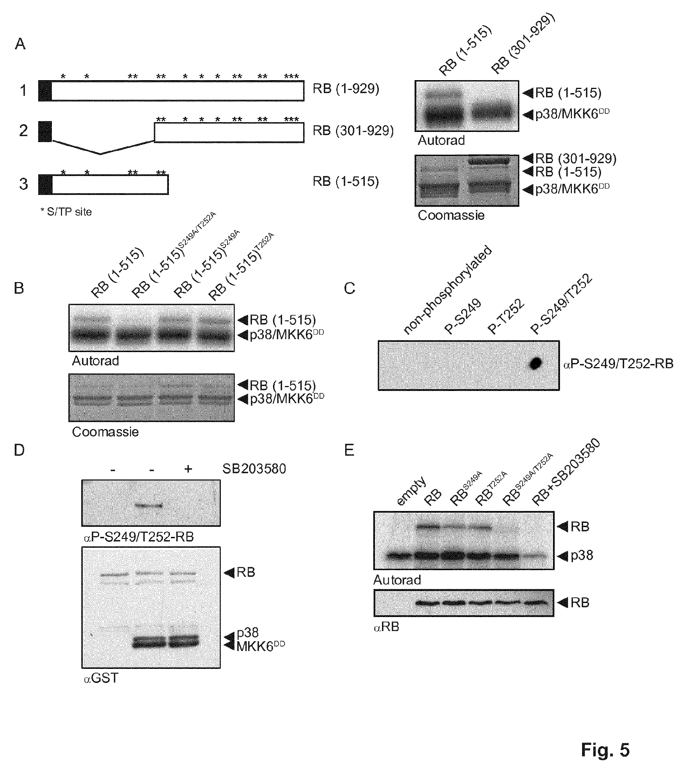

**Fig. 5****:** p38 phosphorylates RB at S249 and T252 *in vitro*
   - Panel A: p38 phosphorylates the N-terminal region of RB *in vitro.* Representation of the human RB protein, its Serine/Threonine followed by Proline (S/TP site) phosphorylation sites and their phosphorylation by p38. Left part: 1: Position of the 16 putative phosphorylation sites along the 929 amino acids of the whole RB protein following the minimum SAPK S/TP motif. 2: GST-RB (301-929) -containing 12 putative sites- mutant. 3: GST-RB (1-515) - containing 6 putative sites- mutant. Right part: Results of the *in vitro* assay of the two mentioned RB fragments in the presence of activated GST-p38 as explained in Fig. 4C, showing that the GST-RB (1-515) was phosphorylated by the p38 SAPK, whereas the GST-RB (301-929) was not.
   - Panel B: p38 phosphorylates GST-RB (1-515) at Ser249/Thr252 in vitro. Results of the *in vitro* kinase assay carried out with the GST-RB RB(1-515) fragment and the double and single mutants GST-RB(1-515)^{S249A/T252A} GST-RB(1-515)^{S249A} and GST-RB(1-515)^{T252A} of said fragment, as indicated over the lanes, as explained in Fig. 4C. It can be seen that p38 phosphorylates GST-RB (1-515) *in vitro,* while the mutant GST-RB (1-515)^{S249A/T252A} was not phosphorylated.
   - Panel C: Western blot of samples containing peptides of the RB N-terminal part: non-phosphorylated, phosphorylated only at Serine 249 (P-S249), phosphorylated only at Threonine 252 (P-T252) or phosphorylated at both amino acids (P-S249/T252), performed with a purified phospho-specific RB antibody generated against the double-phosphorylated peptide: the antibody recognized the double phosphorylated peptide P-S249/T252-RB but not the non-phosphorylated nor the single phosphorylated peptides.
   - Panel D: Western blot performed with the phospho-specific antibody of Panel C on samples of an *in vitro* cold kinase assay carried out with GST-RB purified from *E. coli.* The upper part shows the results obtained with the antibody against the peptide P-S249/T252-RB. The lower part shows the western blot using anti-GST antibody and monitoring the amount of loaded proteins (GST-RB, GST-p38 and GST-MKK6^{DD}). SB203580 p38 inhibitor was used as a control (lane with the "+" sign over it).
   - Panel E: *RB*^{*-*/*-*} MEFs were transfected with an empty plasmid, wild type myc-RB or myc-RB^{S249A/T252A}, as indicated over the lanes, in the presence (lanes 4 and 6) or absence (lanes 1, 2, 3 and 5) of MKK6^{DD} in order to activate p38. Cell lysates were analyzed by Western blot with the indicated specific antibodies for (from top to bottom) the RB double phosphorylated P-S249/T252-RB, total RB, phosphorylated p38 (P-T180/Y182-p38), p38 and MKK6^{DD}. Results show that genetically activated-p38 phosphorylates RB at Ser249/Thr252 in cultured cells.

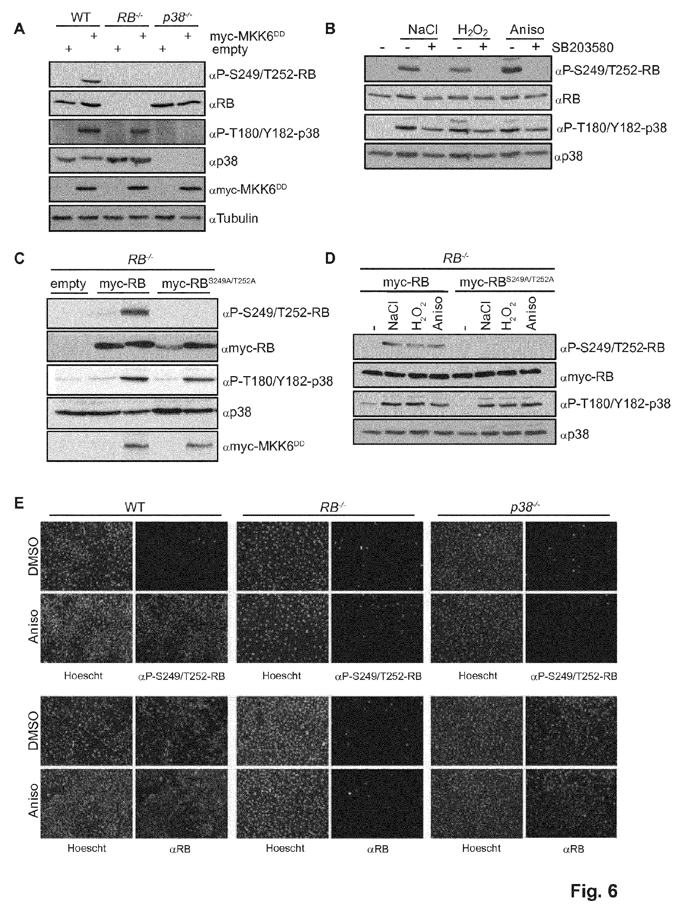

**Fig 6****:** p38 phosphorylates RB at S249 and T252 in cultured cells
   - Panel A: p38 phosphorylates full-length RB at Ser249 and Thr252 *in vitro.* HEK293T cells were transfected with myc-tagged wild type RB or with its single and double mutants RB^{S249A}, RB^{T252A} or RB^{S249A1T252A}, as indicated over the lanes. Immunoprecipitated myc-RBs were used in an *in vitro* kinase assay as explained in Fig.4C. SB20358 p38 inhibitor was used as a control (sixth lane from the left). Autoradiography (upper part; Autorad) and the Western blot performed with RB specific antibodies (lower part) are shown. The position of the bands corresponding to full-length RB and p38 proteins are indicated on the right. Wild type RB is phosphorylated by GST-p38 whereas this phosphorylation was reduced in RB^{S249A} and RB^{T252A} and abolished in RB^{S249A/T252A} mutant.
   - Panel B: Wild type (WT), RB^{-/-} and p38^{-/-} MEFs were transfected with an empty plasmid or a plasmid expressing myc-MKK6^{DD}, as indicated over the lanes, in order to activate p38. Cells lysates were analyzed by Western blot using specific antibodies, which are (from top to the bottom) the RB double phosphorylated P-S249/T252-RB, total RB, phosphorylated p38 (P-T180/Y182-p38), p38 and MKK6^{DD} and Tubulin as a loading control. Results show that genetically activated-p38 phosphorylates RB in cultured cells.
   - Panel C: Wild type MEFs cells were challenged with 100 mM NaCl, 400 µM H₂O₂ or 25 ng/ml anisomycin (indicated as "Aniso") for 2 hours in the presence (lanes labelled "+") or absence (lanes labelled "-") of SB203580. Cells lysates were analyzed by Western blot using specific antibodies, which are (from top to the bottom) the RB double phosphorylated P-S249/T252-RB, total RB, phosphorylated p38 (P-T180/Y182-p38) and p38. Results show that stress activated-p38 phosphorylates RB in cultured cells.
   - Panel D: RB^{-/-} MEFs transfected with wild type myc-RB or myc-RB^{S249A/T252A} as indicated over the lanes, and challenged with 100 mM NaCl, 400 µM H₂O₂ or 25 ng/ml anisomycin (indicated as "Aniso") for 2 hours. Cells lysates were analyzed by Western blot using specific antibodies, which are (from top to the bottom) the RB double phosphorylated P-S249/T252-RB, total RB, phosphorylated p38 (P-T180/Y182-p38) and p38. Results show that stress activated-p38 phosphorylates RB at Ser249/Thr252 in cultured cells.
   - -Panel E: Indirect immunofluorescence pictures showing the signal of RB phosphorylated at S249/T252 (antibody indicated in the figure as αP-S249/T252-RB; upper panels) and total RB protein (antibody indicated in the figure as αRB; bottom panels, obtained from wild type (WT), RB^{-/-} and p38^{-/-} MEFs (as indicated over the lanes) grown on glass covers and treated with 25 ng/ml anisomycin (indicated as "Aniso") or with DMSO for 2 hours prior to fixation. Nuclear DNA was stained with Hoeschst 33342. Pictures were taken using an inverted Olympus CKX 41 microscope and the Olympus Cell^D imaging software. Representative pictures are shown.

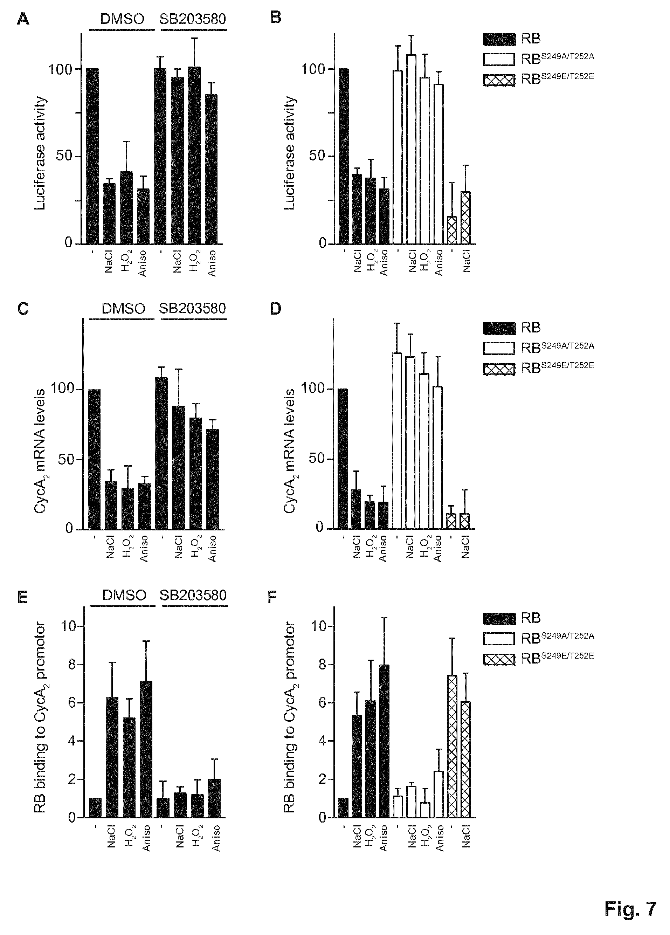

**Fig. 7****:** S249/T252 phosphorylation increases RB transcriptional repression
   - Panel A: Luciferase activity, measured in HEK293T cells transfected with HA-E2F1, HA-DP1, pE2F-Luc and wild type myc-RB and treated with 100 mM NaCl, 400 µM H₂O₂ or 25 ng/ml anisomycin (indicated as "Aniso"), as indicated in the X-axis, for 4 hours in the presence or absence (DMSO) of 1 µM SB203580. Values are expressed relative to unstressed cells. Luciferase reporter activity was measured from cell supernatants using a Luciferase Reporter Assay kit (Promega) and a Microlumat LB 960 luminometer (Berthold Technologies). The total amount of protein found in the cell extracts was measured with the Bradford reagent (Bio-Rad). Protein-corrected luciferase reporter activities are shown. Results show that E2F-dependent expression was strongly repressed upon stress and this repression was completely dependent on the activity of p38 since it was suppressed in the presence of p38 inhibitor.
   - Panel B: Luciferase activity, measured in HEK293T cells transfected with HA-E2F1, HA-DP1 and pE2F-Luc in the presence of wild type myc-RB (black bars), myc-RB^{S249A/T252A}, (white bars) or myc-RB^{S249E/T252E} (scratch bars) and treated as in (A). Values are expressed relative to unstressed cells. Results show that the RB mutant S249/T252 was unable to repress transcription of the E2F reporter upon stress when compared with RB wild type, whereas the mutant S249E/T252E inhibited expression at similar levels to those observed in wild type upon stress.
   - Panel C: mRNA levels of CycA₂ analyzed by RT-PCR in samples of wild type MEF preincubated with 1µM SB203580 30 min. or not (DMSO) before being challenged with 100 mM NaCl, 400 µM H₂O₂ or 25 ng/ml anisomycin for 7 hours. The GAPDH mRNA was used as loading control. Values are expressed relative to unstressed cells. Results show that CycA2 expression was repressed upon stress and this repression was not observed when p38 was inhibited.
   - Panel D: mRNA levels of CycA₂ analyzed by RT-PCR in *RB*^{*-*/*-*} MEFs transfected with wild type myc-RB, myc-RB^{S249A/T252A}, and myc-RB^{S249E/T252E} stressed as in (C). Results show that CycA2 expression was unable to be repressed upon stress in the presence of the unphosphorylatable RB protein, whereas gene expression is repressed in the phoshomimetic RB mutant without stress.
   - Panel E: Binding of myc-RB to the CycA₂ promoter, determined by Chromatin Immunoprecipitation (ChIP) analysis, in HEK293T cells transfected with myc-RB and treated with 100 mM NaCl, 400 µM H₂O₂ or 25 ng/ml anisomycin and treated as in (A). Results show that in the presence of stress RB association clearly increased depending on its phosphorylation by p38.
   - Panel F: Binding of myc-RB to the CycA₂ promoter, determined by CHIP, in HEK293T cells transfected with wild type myc-RB, myc-RB^{S249A/T252A}, or myc-RB^{S249E/T252E} and treated as in (A). Results show that the increase on association of RB to CycA2 promoter upon stress is not observed in the presence of the unphosphorylatable RB protein, whereas the phosphomimetic RB associated to endogenous promoters similarly to wild type RB in the presence of stress.
   In panel A to F data are represented as mean ±SEM.

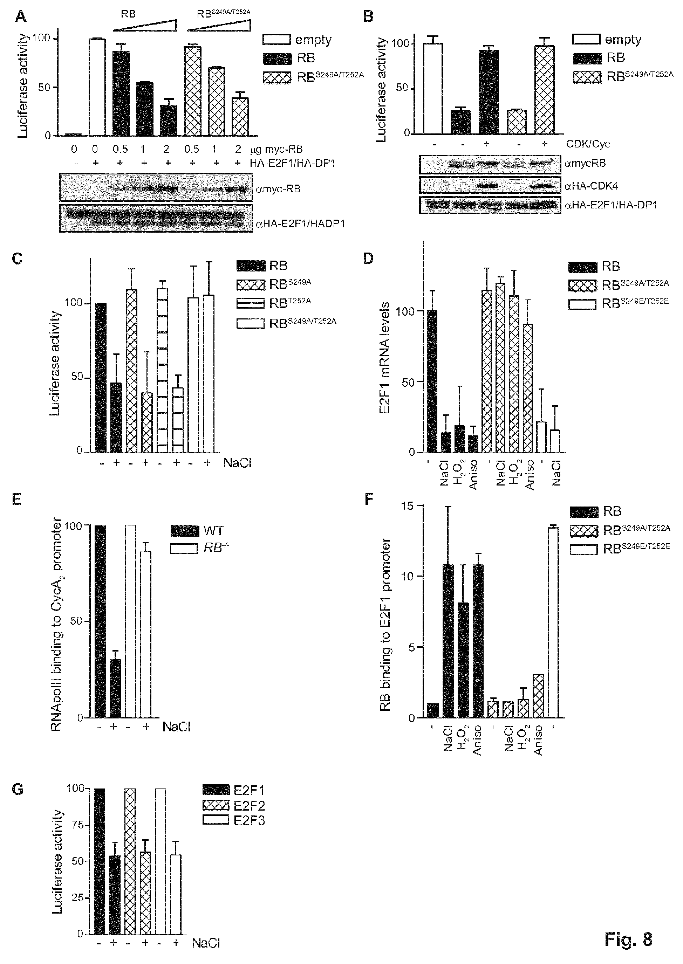

**Fig. 8****:** S249/T252 phosphorylation increases RB transcriptional repression
   - Panel A: Upper panel: Luciferase activity measured as in Fig. 7A in HEK293T cells transfected with HA-E2F1 and HA-DP1 (lanes labelled "+") or not (lanes labelled "-") together with pE2F-Luc, in the presence of growing concentration of wild type myc-RB (results indicated with black bars), or myc-RB^{S249A/T252A} (results indicated with scratch bars), as indicated over the lanes and in the in the X-axis. An empty plasmid was used as a control (results indicated with white bars). Lower panel: Cell lysates were taken and analyzed by Western blot using anti-myc and anti-HA antibodies. Results show that E2F-luc reporter monitors properly RB activity.
   - Panel B: Upper panel: Luciferase activity measured as in Fig. 7A in HEK293T cells transfected with HA-E2F1, HA-DP1 and pE2F-Luc, wild type myc-RB (results indicated with black bars), or myc-RB^{S249A/T252A}, (results indicated with scratch bars) in the presence or absence of HA-CDK4, HA-CycD, HA-CDK2 and HA-CycA₂ (lanes labelled "+") or not (lanes labelled "-") (indicated as "CDK/Cyc" in the figure. An empty plasmid was used as a control (results indicated with white bars). Lower panel: Cells lysates were taken and analyzed by Western blot specific antibodies. Results show that E2F-luc reporter monitors properly RB inactivation by CDKs.
   - Panel C: Luciferase activity measured as in Fig. 7A in HEK293T cells transfected with HA-E2F1, HA-DP1 and pE2F-Luc, in the presence of wild type myc-RB (black bars), myc-RB^{S249A} (striped bars), myc-RB^{T252A} (scratch bars) or myc-RB^{S249A/T252A}, (white bars). Cells were treated (lanes labelled "+") or not (lanes labelled "-") with 100 mM NaCl for 4 hours. Results show that mutation to alanine of both Ser249 and Thr252 is needed to turn RB insensitive to p38 modulation.
   - Panel D: mRNA levels of E2F1 analyzed by RT-PCR in samples from *RB*^{*-*/*-*} MEFs transfected with wild type RB-myc (black bars), myc- myc-RB^{S249A/T252A} (scratch bars) or myc-RB^{S249E/T252E} (white bars) stressed or not (lanes labelled "-") with 100 mM NaCl, 400 µM H₂O₂ or 25 ng/ml anisomycin (indicated as "Aniso"), as indicated in the X-axis, for 7 hours. GAPDH mRNA was used as loading control. Results show that E2F1 down-regulation upon stress is rescued in RB^{-/-} MEFs transfected with wild type myc-RB and myc-RB^{S249E/T252E} but not with myc-RB^{S249A/T252A}.
   - Panel E: Binding of RNA pol II to the CycA₂ promoter, determined by ChIP, in wild type (WT; black bars) and *RB*^{*-*/*-*} MEFs (white bars) treated (lanes labelled "+") or not (lanes labelled "-"), as indicated in the X-axis, with 100 mM NaCl for 20 minutes. Results show that stress decreases RNA Pol II recruitment at the CycA2 promoter in a RB-dependent manner.
   - Panel F: Binding of myc-RBs to the E2F1 promoter, determined by CHIP, in HEK293T cells transfected with myc-RB (black bars), myc-RB^{S249A/T252A} (scratch bars) or myc-RB^{S249E/T252E} (white bars) and treated or not (lanes labelled "-") with100 mM NaCl, 400 µM H₂O₂ or 25 ng/ml anisomycin for, as indicated in the X-axis, for 4 hours. Results show that RB binding to the E2F1 promoter upon stress is rescued in RB^{-/-} MEFs transfected with wild type myc-RB and myc-RB^{S249E/T252E} but not with myc-RBS^{249A/T252A}.
   - Panel G: Luciferase activity as in Fig. 7A in HEK293T cells transfected with HA-E2F1 (black bars), HA-E2F2 (starch bars) or HA-E2F3 (white bars) together with HA-DP1 and pE2F-Luc. Cells were treated (lanes labelled "+") or not (lanes labelled "-") with 100 mM NaCl, as indicated in the X-axis, for 4 hours.
   In panel A to G data are represented as mean ±SEM.

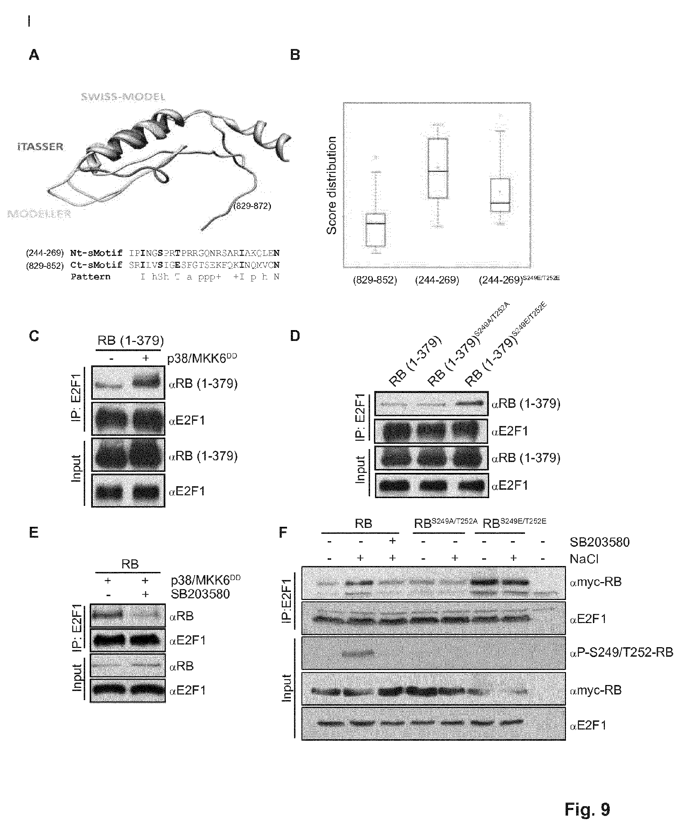

**Fig. 9****:** RB phosphorylation at S249/T252 increases the affinity of the N-terminal for E2F.
   - Panel A: Superposition of the structures of the N-terminal sMotif (244-269) region of RB modelled with Swiss-Model (light-brown in the original, medium grey in grey tones), iTASSER (red in the original, darkest lines in the drawing in grey tones) and MODELLER (yellow in the original, lightest grey in the drawing in grey tones), and the structure of the C-terminal sMotif (829-872) taken from its crystallographic complex with E2F1/DP1 (code 2AZE in PDB). In the bottom line is shown the sequence alignment of the N-terminal sMotif (244-269) and the C-terminal sMotif (829-852) of RB showing in the third line the similar hydrophobicity pattern of residues (p for polar, h for hydrophobic and + for positively charged, I for isoleucine, S for serine, T for threonine, N for asparagine). The superstition indicated that the super-secondary structure of the RB N-terminal is similar to the C-terminal region that bounds E2F1/DP1.
   - Panel B: Box-plot distribution of the ΔΔG of the interaction between the E2F1/DP1 complex and RB peptides (829-852), (244-269) and (244-269)^{S249E/252E}. Data are represented in boxes of quartiles around the average. Results show that the affinity of the N-terminal peptide clearly improved when the double mutation S249E/T252E is included.
   - Panel C: RB N-terminal fragment phosphorylated by p38 has increased affinity towards HA-E2F1 *in vitro.* GST-RB (1-379 aminoacids) purified from *E. coli* was phosphorylated (lanes labelled "+") or not (lanes labelled "-") *in vitro* with activated GST-p38 (indicated as "p38/MKK6^{DD}) and incubated with cell lysates from HEK293T cells transfected with HA-E2F1 (indicated as "Input"). Then, HA-E2F1 was immunoprecipitated using anti-HA sepharose beads (indicated as "IP:E2F1) and E2F1 interaction with RB (1-379) was analyzed by Western blot using specific antibodies, which are GST (RB (1-379)) and HA (E2F1).
   - Panel D:A phosphomimetic mutant of RB (1-379) has more affinity towards E2F1. HEK293T cells were transfected with HA-E2F1. Cell lysates (indicated as "Input") were then incubated with *E. coli* purified GST-RB (1-379), GST-RB (1-379)^{S249A/T252A} or GST-RB (1-379)^{S249E/T252E}, as shown over the lanes. E2F1 interaction with RB (1-379) was analyzed as in (C).
   - Panel E: Full-length p38-phosphorylated RB has increased affinity towards HA-E2F1 *in vitro.* GST-RB purified from *E. coli* was phosphorylated (lane labelled "+") or not (lane labelled "-") *in vitro* with activated GST-p38 (indicated as "p38/MKK6^{DD}"); SB203580 was used (lane labelled "+") or not (lane labelled "-") to preclude this phosphorylation. Cell lysates from HEK293T cells transfected with HA-E2F1 were then incubated with p38-phosphorylated GST-RB and samples were processed as in (C).
   - Panel F: p38-phosphorylated RB has increased affinity towards HA-E2F1 *in vivo.* HEK293T cells were transfected with HA-E2F1 and wild type myc-RB, myc-^{RBS249A/T252A} or myc-Rb^{S249E/T252E}, as indicated over the lanes. Cells were preincubated (lanes labelled "+") or not (lanes labelled "-") with 1 µM SB203580 30 minutes before being challenged (lanes labelled "+") or not (lanes labelled "-") with 100 mM NaCl for 2 hours. HA-E2F1 was immunoprecipitated using anti-HA sepharose beads. E2F1 interaction with RB was analyzed by Western blot using specific antibodies, which are shown in the figure.

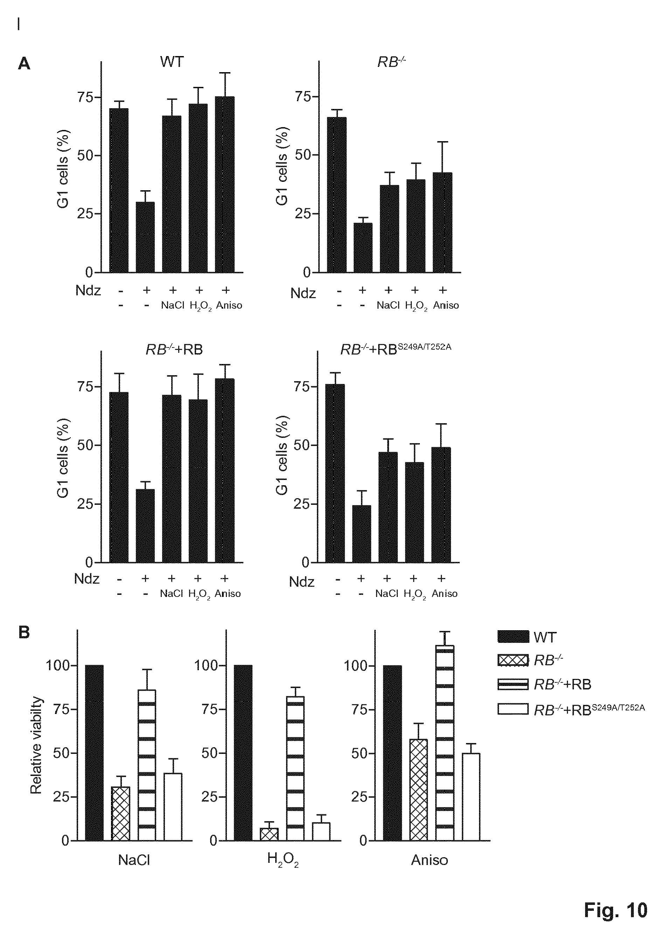

**Fig. 10****:** RB phosphorylation at S249/T252 is relevant for cell cycle progression and cell survival upon stress.
   - Panel A: Percentage of G1 cells determined by monitorization of cell cycle progression by FACS in samples of wild type (WT), RB^{-/-} MEFs or RB^{-/-} MEFs transfected with myc-RB (RB^{-/-} +RB) or myc-RB^{S249A/T252A} (RB^{-/-} +RB^{S249A/T252A}), as indicated over each bar chart. Cells had been stressed or not with 100 mM NaCl, 400 µM H₂O₂ or 25 ng/ml anisomycin (indicated as "Aniso"), as indicated below the X-axis, and, one hour later, Nocodazole had been added (lanes labelled "+") or not (lanes labelled "-") to trap the cells at the G2/M transition. Data are represented as mean ±SEM. Cell cycle progression was monitored by FACS by collecting samples after 8 hours. Percentage of G1 cells is shown.. Results show that the stress-induced G1 delay is rescued in RB-/-MEFs transfected with wild type myc-RB but not myc-RB^{S249A/T252A}.
   - Panel B: Percentage of cell viability, referred to the value obtained in wild type cells, assessed by FACS 24 hours after the addition of 200 mM NaCl, 1200 µM H₂O₂ and 50 ng/ml anisomycin (indicated as "Aniso"). Cells are the same indicated in (A). Data are represented as mean ±SEM. Results show that cell viability is compromised in *RB*^{*-*/*-*} and *RB*^{*-*/*-*} MEFs expressing myc-Rb^{S249A/T252A} but not in wild type or *RB*^{*-*/*-*} MEFS expressing myc-RB.
**Fig. 11****:** Cell viability is compromised in *RB*^{*-*/*-*} MEF under stress conditions. Cell viability, assessed by FACS, 24 hours after the addition of the amounts indicated below the X-axis of NaCl (panel A), H₂O₂ (panel B), or anisomycin (indicated as "Aniso") (panel C) to wild type (WT) or RB^{-/-} MEF cells. Data are represented as mean ±SEM.
**Fig. 12****:** RB phosphorylated at S249/T252 overcomes CDK control.
   - Panels A and B:HEK293T cells were transfected with an empty plasmid (empty) wild type myc-RB, myc-RB^{S249A/T252A}, or myc-RB^{S249E/T252E} with (lanes labelled "+") or without (lanes labelled "-") HA-CycD, HA-CDK4, HA-CycA₂ and HA-CDK2 (indicated as CDK/Cyc). CDK phosphorylation on RB, visualized in Western blot) as slow migration band with anti-myc (top, panel A) or by phospho-specific antibodies of RB taht are CDK-dependent (panel B). These results show that CDKs can target wild type myc-RB, myc-RB^{S249A1/T252A} and myc-RB^{S249E/T252E} equally.
   - Panel C: Luciferase activities measured as in Fig. 7A in HEK293T cells transfected with HA-E2F1, HA-DP1 and pE2F-Luc and an empty plasmid (white bars), wild type myc-RB (black bars), myc-RB^{S249A/T252A}, (starch bars) or myc-RB^{S249E/T252E} (striped bars), with or without HA-CycD, HA-CDK4, HA-CycA₂ and HA-CDK2 (as indicated below the X-axis with "+" or "-" signs in the line labelled CDK/Cyc) and challenged with 100 mM NaCl 4 hours when indicated (again by "+" or "-" signs in the line labelled "NaCl" below the X-axis). Data are represented as mean ±SEM. These results show that p38-phosphorylated myc-RB and myc-RB^{S249E/T252E} mutant are insensitive to CDKs.
   - Panel D: Binding of myc-RB to the CycA₂ promoter, determined by ChIP, in HEK293T cells transfected and stressed as in (C). Data are represented as mean ±SEM. Results showed that CDK activity is not able to remove p38-phosphorylated myc-RB and myc-RB^{S249E/T252E} from CycA₂ promoter.
   - Panel E: GST-RB purified from *E. coli* was phosphorylated *in vitro* with activated GST-p38 (lanes labelled "+"; indicated as "p38/MKK6^{DD}), GST-CDK2 (lane 3) or both (lane 4) and incubated with cell lysates from HEK293T cells transfected with HA-E2F1 (indicated as "Input"). Then, HA-E2F1 was immunoprecipitated using anti-HA sepharose beads (indicated as "IP:E2F1) and E2F1 interaction with RB was analyzed by Western blot using specific antibodies against RB and E2F1. These results show that RB phosphorylated by both p38 and CDK2 can still bind to E2F1.
   - Panel F: Representative pictures of HEK293T cells growing for 72 hours after having transfected the cells with an empty vector, wild type myc-RB or myc-RB^{S249E/T252E} (as indicated at the beginning of each row of pictures), in the presence or absence of HA-CycD, HA-CDK4, HA-CycA₂ and HA-CDK2 (CDK/Cyc) (as indicated with "+" for presence or "-" for absence CDK/Cyc over each column). It can be seen that CDK-phosphorylated- RB^{S249E/T252E} can repress cell growing.
   - Panel G: Binding of myc-RB to the E2F1 promoter, determined by ChIP, in HEK293T cells transfected with an empty plasmid (white bars), wild type myc-RB (black bars), myc-RB^{S249A/T252A}, (starch bars) or myc-RB^{S249E/T252E} (striped bars), with or without HA-CycD, HA-CDK4, HA-CycA₂ and HA-CDK2 (as indicated below the X-axis with "+" or "-" signs in the line labelled CDK/Cyc), and challenged or not with 100 mM NaCl for 4 hours when indicated (again by "+" or "-" signs in the line labelled "NaCl" below the X-axis). Data are represented as mean ±SEM. Results shown that CDK activity is not able to remove p38-phosphorylated-myc-RB nor myc-RB^{S249E/T252E} from E2F1 promoter.
   - Panel H: Cell count, expressed as millions of cells, of HEK293T cells transfected with an empty plasmid (white bars), wild type myc-RB (black bars) or myc-RB^{S249E/T252E} (starch bars), with or without HA-CycD, HA-CDK4, HA-CycA₂ and HA-CDK2 (as indicated below the X-axis with "+" or "-" signs in the line labelled CDK/Cyc). Cells were collected 72 hours posttransfection. Data are represented as mean ±SEM. It can be seen that CDK-phosphorylated-RB^{S249E/T252E} can repress cell growing.

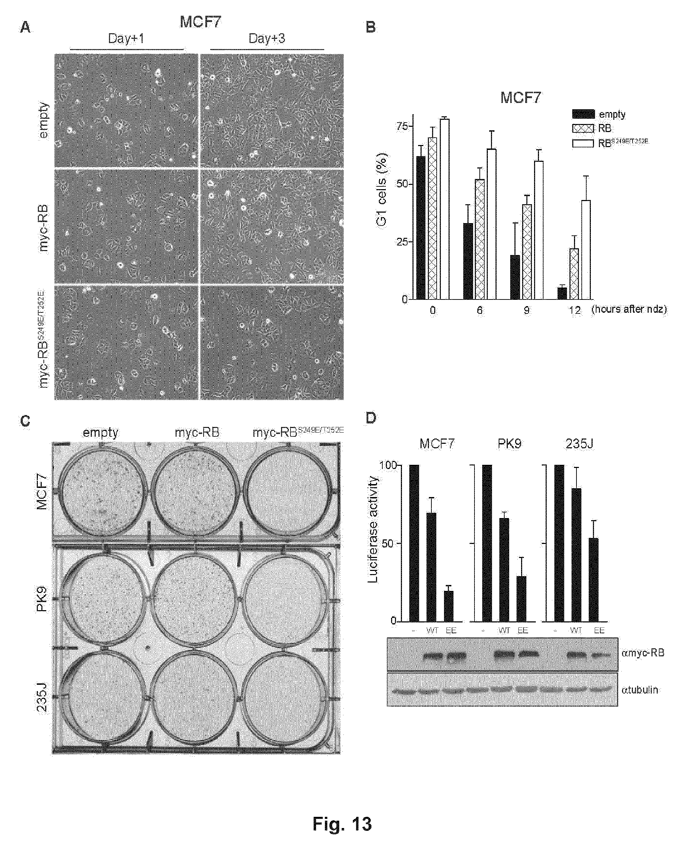

**Fig. 13****:** RB phosphorylated at S249/T252 prevents proliferation of cancer cells with high CDK activity
   - Panel A: Representative pictures of growing MCF7 breast cancer cells taken 24h (first column; Day+1) or 72h (second column; Day+3) after having been transfected with an empty vector, wild type myc-RB or myc-RB^{S249E/T252E} (as indicated at the beginning of each row of photographs). It can be seen that RB^{S249E/T252E} can repress cell growing of cancer cells with high CDK activity.
   - Panel B: Percentage of G1 cells determined by monitorization of cell cycle progression by FACS in samples of MCF7 cells transfected with an empty vector (empty: first bar of each group of three, black bars), wild type myc-RB (RB: second bar of each group of three, starch bars) or myc-RB^{S249E/T252E} (RB^{S249E/T252E}: third bar of each group of three, white bars) and treated with Nocodazole to trap the cells at the G2/M transition. Samples were collected at the times indicated below the X-axis after nocodazole (indicated as "ndz") was added. Data are represented as mean ±SEM. Results showed that RB^{S249E/T252E} delays the cell cycle.
   - Panel C: Pictures of crystal violet stained colonies of MCF7 (breast), PK9 (pancreatic) and 235J (bladder) cancer cell lines transfected with empty plasmid, wild type myc-RB or myc-RB^{S249E/T252E} It can be seen that RB^{S249E/T252E} impaired colony formation.
   - Panel D: Luciferase activity, expressed relative to that one of the cells transfected with an empty vector, measured in MCF7, PK9 or 235J cells, transfected either with an empty plasmid (indicated as "-"), wild type myc-RB or myc-RB^{S249E/T252E} and with HA-E2F1, HA-DP1 and pE2F-Luc. Data are represented as mean ±SEM. Cells lysates were taken and analyzed by Western blot, giving rise to the results shown in the bottom part of the panel. It can be seen that RB^{S249E/T252E} acts as a superrepressor.

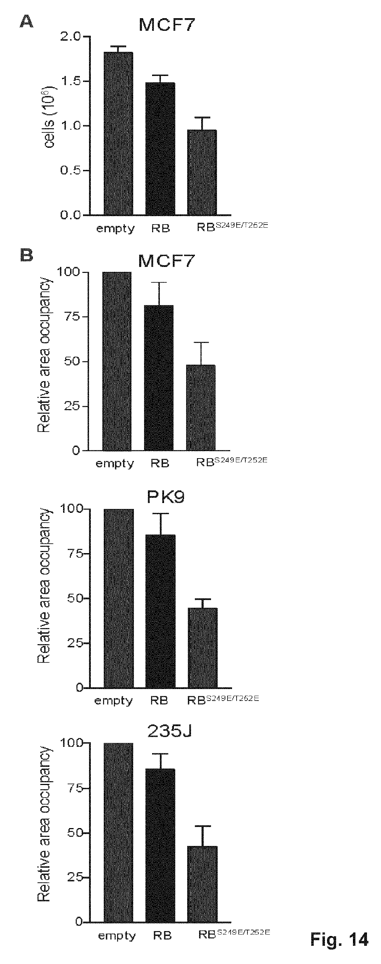

**Fig. 14****:** RB phosphorylated at S249/T252 prevents proliferation of different cancer cell lines with high CDK activity
   - Panel A: Cell count, expressed as millions of cells, of MCF7 cells transfected with and empty vector, wild type myc-RB or myc-RB^{S249E/T252E}. Cells were collected and counted 72 hours posttransfection. Data are represented as mean ±SEM.
   - Panel B: ImageJ quantification of crystal violet stained colonies shown on Fig. 13C. Data are represented as mean ±SEM.

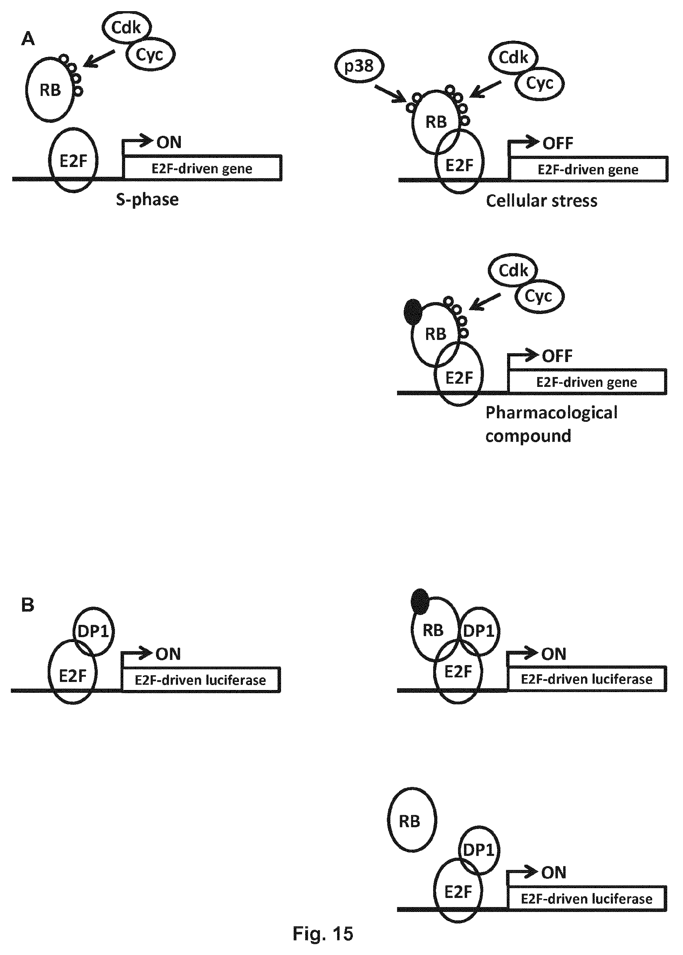

**Fig. 15****:** Representation of the approach used for detecting compounds that mimic the effect of phosphorylation of RB in S249/T252 from the transcriptional status of naturally E2F-driven genes (panel A) or an E2F-driven reporter gene such as luciferase (panel B).
   - Panel A: Representation of how phosphorylation of RB by Cdk/Cyc complexes gives rise to release of RB from E2F and transcription of E2F-driven genes (genes whose transcription is dependent on E2F transcription factor) at S-phase. Phosphorylation by p38 under conditions of cellular stress impedes release of RB, even when it has been phosphorylated by Cdk/Cyc, and prevents transcription of E2F-driven genes (OFF status). Small circles on the surface of the ellipse labelled RB represent phosphorylation sites; those on the left, connected by an arrow to p38, correspond to phosphorylation at S249/T252. A pharmacological compound (black circle on left part of the surface of RB-labelled ellipse) that mimics the effect of phosphorylation at S249/T252 should analogously prevent both release of RB from E2F and transcription of an E2F driven gene (OFF status again).
   - Panel B: Representation of the conditions for the identification of a potential anticancer pharmalogical compounds through an E2F-driven reporter (luciferase). The compound (represented by the black circle appearing on the left part of the surface of the RB-labelled ellipse) will prevent both release of RB from E2F and transcription of the luciferase E2F-driven gene (OFF status), while luciferase should be transcribed and expressed in the absence of the compound (ON status).

### DETAILED DESCRIPTION OF THE INVENTION

The control of the G1/S transition by the Retinoblastoma (RB) tumour suppressor is critical for cell proliferation and its inactivation is one of the most fundamental events in cancer. RB is inactivated by sequential phosphorylations by CDK complexes to elicit cyclin expression and promote entry into S-phase.

As it was discussed above, it was known that activation of the p38 Stress-Activated Protein Kinase (SAPK) upon stress delays cell cycle progression and control G1/S transition by targeting the p57 CDK inhibitor. The present inventors have now found that, in addition, the G1/S transition is also controlled by the down-regulation of gene expression of E2F mediated genes (such as, for instance, cyclins) in response to stress. This down-regulation of expression of E2F mediated genes, and the resulting delay of cell cycle in G1, depends on activation of the p38 SAPK and the regulation of the RB transcriptional repressor by the p38 SAPK.

Thus, a tight regulation of the G1-S transition is conserved across eukaryotes. The relevance of this regulation is illustrated by the fact that alteration of the delay in G1-S, such as the mutation in p57 CDKi or in RB, whicht prevents cyclin down-regulation, clearly decreases cell survival upon stress.

Regulation of RB by phosphorylation has been associated mainly to its inactivation by CDKs. The RB phosphorylation by CDKs occurs at the pocked domain and the C-terminal domain (RBC), albeit N-terminal domain (RBN) phosphorylations had been described and considered to be CDK phosphorylations sites too (see, for instance, the content of EP-1156332-A2, as it has been discussed above). Some researchers had even reported that phosphorylation of Ser249 and Thr252 inhibits the interaction of RB with EID1 but has no effect on E2F inhibition (Hassler et al., 2007; Rubin et al., 2013), probably because the related assays were carried out *in vitro,* not *ex vivo* cell conditions.

The present inventors have now found that the p38 SAPK phosphorylates RB at Ser249 and Thr252 in the N-terminal domain of RB. Surprisingly, and contrary to previous reports,the assays carried out by the present inventors with cell cultures (and, thus, in ex vivo conditions) show that phosphorylation of Ser249 and Thr252 at the N-terminus of human RB by p38 results in an increase on the affinity of RB towards E2F transcription factor, giving rise to a stronger association of RB at the promoters. As a consequence, Ser249/Thr252 phosphorylation (or phosphorylation in the equivalent residues of RBN of other mammals) prevents E2F-mediated gene expression, so that it prevents cyclin expression, delays cell cycle and improves cell survival upon stress.

Remarkably, phosphorylation of human RB at Ser249/Thr252 by p38 not only leads to cell cycle delay and increased cell survival upon stress, but it also results in a RB insensitive to CDK inactivation, as can be seen, for instance, in Example 6 and the following ones.

Modelling and biochemical analysis shown in the present application have served to propose a tentative model to explain the lack of RB inactivation by CDKs. The N-terminal surface of RB interacts weakly with E2F, but its affinity clearly increases upon phosphorylation at Ser249/Thr252, creating a new surface for interaction. The inactivating phosphorylations of RB by CDKs mostly lay on the pocket and C-terminal regions and thus, it is conceivable that CDK phosphorylations do not interfere with the association of E2F with the phosphorylated N-terminal domain.

The expression of a mutant that mimics RB phosphorylation (RB^{S249E/TS52E}) results in a RB that overrides CDK control and it is able to prevent cell proliferation in cancer cells. As can be seen in Example 7 of the present application, the expression of an RB^{S249E/TS52E} mutant in several cancer cell lines (such as MCF7 (breast cancer cells), PK9 (pancreatic cancer cells) and 235J (bladder cancer cells), all of them cancer cells with high CDK activity), strongly reduced colony-forming ability of all three cell lines and reduced colony formation. Assays carried out with other cell lines, such as PaTu8988S and L3.6 (pancreatic cancer) y MDA_MB_231 (breast cancer) gave rise to similar results.

It is particularly remarkable that the Ser249/Thr252 phosphorylation of human RB by p38 or, also, a mutant that mimics said phosphorylation, represses E2F-dependent transcription even in the presence of high CDK activity and even in the presence of CDK phosphorylations in RB which are known to promote E2F-RB complex dissociation, resulting in a reduction of cellular proliferation in cancer cells (see Example 6 and 7).

It is disclosed herein that the present inventors have found that the expression of a mutant of human RB that mimics the phosphorylation by p38 at Ser249/Thr252, the RB^{S249E/TS52E} blocks E2F-dependent gene expression and it is present at E2F-promoters even in the presence of high CDK activity, a typical scenario in which wild type RB is unable to associate at those promoters and to repress transcription. Correspondingly, in contrast to wild type RB, the RB^{S249E/TS52E} is able to restrict cellular growth in colony forming assays. Thus, a compound capable of inducing phosphorylation of human RB at Ser249/Thr252, or at the equivalent residues of the N-terminal domain of RB of another mammal, should be a candidate to anticancer drug, because it should give rise to the same effects observed when phosphorylation by p38 takes place, particularly the reduction of proliferation of cancer cells. Analogously, a compound capable of inducing the same conformational change in RB that results from phosphorylation at Ser249/Thr252 in human RB, leading to a stronger association with E2F, should serve to restrict cellular proliferation in tumour cells with high CDK content.

Potential anticancer drugs showing such properties and, thus, targeting RB directly, are expected to have the following advantages in the treatment of RB positive tumours:
- would serve to block any alteration in the whole CDK/CDKi/RB axis, which could have a potential effect in a larger panel of tumours;
- would prevent the inhibition of CDKs and, therefore, would not show some of the undesired side effects of some (potential or even already approved) anticancer drugs that are direct inhibitors of CDKs; such undesired effects are due to effects on pathways related to additional roles played by CDKs in addition to the control of cyclin expression;
- would avoid undesired unspecific off target effects versus other kinases, normally caused by small molecule kinase inhibitors.

Moreover, the expression of a mutant RB where the serine and threonine phosphorylatable sites of the RBN domain have been replaced by glutamic acid (such as the RB^{S249E/TS52E} mutant used in Examples of the present application as a mutant of the human RB protein) in cancer cells should also give rise to a reduction of proliferation of cancer cells, so that the present invention also provides expression vectors of such RB mutants for the treatment of cancer.

Thus, an aspect of the present invention is based on identifying as potential anticancer drugs those compounds that give rise to any (or any combination) of the changes or modifications that have been observed by the present inventors in different cell lines when cells have been subjected to factors that are known to induce p38 activation, namely high NaCl concentration (such as 100 mM) in the culture medium, presence of H₂O₂ in the culture medium, or presence of a p38 activator such as anysomycin in the culture medium. Such changes or modifications are:
- phosphorylation of human RB at Ser249 and/or Thr252, or at the equivalent phosphorylatable serine and threonine residues of the N-terminal domain of the RB of another mammal,
- a conformational change in the structure of RB protein (particularly the structure of the N-terminal fragment) which increases its affinity for E2F and makes RB override the control by CDKs,
- the increase in affinity of RB for E2F,
- the decrease of transcription of E2F1 gene,
- the decrease of transcription of other genes that are E2F-dependent, that is, genes that are under the transcriptional control of a promoter where E2F transcription factor (indeed, the complex E2F-DP1) must bound, free of RB protein, in order to have an activation of the transcription.
- the increase of RB recruitment in the E2F-dependent genes,
- the decrease of RNA Pol II recruitment in the E2F-dependent promoters, and/or
- the decrease of cell proliferation.

The determination of the existence of a change in one of, or a combination of, the above mentioned properties can be assessed following the techniques used in the assays of the present application or by any other means known for those skilled in the art.

In the assays of the present application, the existence of a change or modification in a particular property has been assessed by using cell cultures, contacting the compound under assay with the cells under suitable conditions and during enough time for having an interaction between the compound and the cells. The selected property in the sample of said cell culture after the incubation with the compound was compared with the level determined in a control cell culture of the same cells, which control cells have been subjected to the same conditions that the cells treated with the compound but have not been contacted with the assayed compound. Specific approaches and techniques have been used depending on the particular property determined.

If the selected property to be determined is the level of phosphorylation of human RB at Ser249 and/or Thr252, it can be determined, for instance, by the use of antibodies that allow the differentiation between the phosphorylated and the non-phosphorylated form of RB at said particular positions. For instance, it can be used the approach used in Example 2 of the present application, that is, using an antibody that only recognizes full-length RB protein or peptides with the double phosphorylation at Ser249/Thr252. The antibody can be polyclonal, as in the present application, or monoclonal. The antibody can be generated in a non human mammal (such as rabbit), for instance, with the peptide used for the present invention, NGpSPRpTPRRGQNRSC (SEQ ID NO:22) or any other peptide fragment of RB (including full-length RB) comprising serine 249 and threonine 252 and phosphorylated at both positions.

The high level of conservation of the RB protein among mammals makes the invention extendable to other mammals. For other mammals, the phosphorylation in the equivalent serine and threonine residues of the corresponding RB protein should be determined. As it is used in the present application, such equivalent serine and threonine residues will be the serine and threonine residues present in the RBN domain, as a part of a SP (Serine-Proline) or TP (Threonine-proline) consensus site, whise sites are located close one to the other, and in a position corresponding to those of Serine249 and Threonine252 in human RB after an alignment similar to the one shown in Fig. 1B, where amino acids 201-300 of human RB have been aligned with the corresponding region of chimpanzee, cow, mouse and rat, demonstrating the high degree of conservation, in said region, of amino acids in general and of the phosphorylation sites described in human in particular. Thus, for instance, the level of phosphorylation of Serine 243 and Threonine 246 should be determined for mouse (*Mus musculus*) retinoblastoma protein.

It is preferred that the property determined is the level of expression of a gene that is under the transcriptional control or operatively linked to an E2F-dependent promoter and that depends on the binding of the E2F1 transcription factor for being transcribed. Fig. 15A represents and summarizes the concept underlying such approach: a compound, that provokes Ser249/Thr252 phosphorylation of RB (as p38 does in stress conditions), or that mimics the effects of such phosphorylation, will increase the affinity of RB for E2F and will prevent its release from the binding to E2F, preventing the transcription of an E2F-driven gene. The gene can be one gene that is naturally an E2F-dependent gene, that is, a gene that is naturally under the transcriptional control of E2F, such as those whose transcriptional level has been determined in assays of the present application (the E2F1 gene itself, CycA₂, cdc2...). It can be also used a E2F reporter gene, defined as the coding sequence of another gene (or more of a gene, translationally linked) operatively linked to an E2F-dependent promoter and that, usually, has been introduced into the cell as an expression vector containing a cassette with the E2F promoter and the coding sequence operatively linked to said promoter.

The level of expression of naturally E2F-dependent genes can be assessed by determining the level of one of its gene products, the transcribed mRNA or the translated protein produced. Determination of mRNAs is preferred. mRNAs levels can be assessed by any one of the methods well known for those skilled in the art, such as RT-PCR performed on total RNA samples, as has been done in assays shown herein. The information shown below, at the beginning of the Examples section, provides pairs of oligonucleotides suitable to be used as primers for RT-PCR analysis of mRNAs corresponding to E2F1 itself (SEQ ID NO:25 and SEQ ID NO:26), as well as for mad2, BRCA1, CycA₂ and CycE1, as examples of human genes transcriptionally dependent on E2F; it is also provided the pair of oligonuleotides for GAPDH, as suggested reference gene for the calculation of relative levels with regard to the values obtained.

The levels of specific proteins can be also assessed for different techniques, all of them well known for those skilled in the art, with preference with those linked to immunodetection and/or isolation by means of a corresponding antibody. The assays herein disclosed shown different examples of such approach. Antibodies for different E2F-transcriptionally dependent proteins are commercially available (such as the anti-E2F1 and the anti-CycA₂ antibodies purchased from SantaCruz used in assays of the present application). Different methods for obtaining and using polyclonal or monoclonal antibodies are also well known for those skilled in the art and can be found summarized in dedicated literature such as "Antibodies: A Laboratory Manual", 2nd Edition, 2014, Cold Spring Harbor Laboratory Press.

Having an antibody directed to a certain protein (or to a phosphorylation status of a protein, as in the case of the determination of Ser249/Thr252 RB phosphorylation), the levels of the protein can be assessed, for instance, from a preparation of total protein after PAGE (PolyAcrylamide Gel Electrophoresis) linked to Western blotting techniques. The assessment of the level of reference proteins, such as α-tubulin, and the quantification with regard to the levels of said proteins in the same sample, is advisable in order to avoid differences in the results due simply to the amount of sample or similar reasons.

Other techniques linked to determination of the amount of a protein present in a sample after previous isolation of the protein from the initial preparation by incubation with the antibody and separation of the complex-protein-antibody from the initial samples by techniques such as immunoprecipitation, affinity purification, etc., are preferred. Antibodies linked to specific particles, such as beads of a specific material (for instance, Sepharose beads or magnetic beads) can be particularly useful for that purpose. For the determination of the amount of specific protein in the initial sample, the used of secondary antibodies directed to the primary antibody and linked to a molecule that gives rise to a signal easy to detect or quantify, such as fluorophore compounds or enzymes giving rise to detectable compounds after contact with certain reagents, such as the well-known horse radish peroxidase, are also suitable for developing a signal and quantifying the amount of the desired protein.

Both the increase of the binding of RB to the E2F-dependent promoters and the increase of the binding of RNA Pol II to said promoters are changes in protein-DNA interactions that can be assessed by similar techniques, such as Chromatin Immunoprecipitation assays (ChiP), as in Examples 3 and 4 of the present application, wherein the RB association to the CycA₂ and E2F1 promoter or the binding of RNA pol II to the CycA₂ promoter are assessed. In ChiP assays, protein-DNA interactions are cross-linked in cell cultures by addition of formaldehyde and purified by Dynabeads (Invitrogen) conjugated with the appropriate antibody. After reversed specific protein-DNA cross-linking, DNA is then extracted with phenol/chloroform and precipitated. Immunoprecipitated DNA fragments are analyzed by PCR or RT-PCR.

Although the expression of natural E2F-dependent genes can be determined for the purposes of the present invention, as it has been discussed above, it is particularly preferred that the gene or coding sequence that is operatively linked to the E2F-dependent promoter is either the coding sequence of a marker or reporter gene tagged or not, because it facilitates the determination of the level of the polypeptide translated from the mRNA transcribed from the E2F-dependent promoter. The relationship and similarity with the effect found in naturally E2F-driven genes is represented in Fig. 15B, where luciferase is the reporter of E2F-driven promoter. As it is used herein, a marker or reporter gene is a gene that encodes a protein which exhibit a characteristic easy to detect or quantify or a protein whose activity gives rise to a product with a characteristic easy to detect or quantify. The reporter protein is the protein encoded by the coding sequence of the reporter gene. Examples of reporter genes and proteins are well known among those skilled in the art. The term includes selection markers such as antibiotic resistance genes, whose encoded proteins, when expressed, allow cells to grow in the presence of the corresponding antibiotic, while non-expressing cells are killed by the antibiotics. For the purposes of the present invention, it is preferred that the reporter gene encodes an enzyme whose catalytic activity can be detected by a simple assay method or a protein with a property such as intrinsic fluorescence so that expression of the reporter gene can be detected in a simple and rapid assay requiring minimal sample preparation. More preferably, the reporter gene encodes an enzyme or protein, the expression of which in a cell can be detected by visual examination of the growth, colour, or morphology of the colony or cells, directly or after exposure to a reagent. Examples of such reporter genes are: the reporter gene lacZ, whose expression results in the β-galactosidase enzyme, which gives rise to the blue colour compound indoxil upon hydrolization of X-gal; proteins with intrinsic fluorescence, such as GFP (Green Fluorescent Protein), RFP (Red Fluorescent Protein) or, as in assays of the present application, firefly or Renilla luciferase (commonly abbreviated as ``Luc"). Proteins with intrinsic fluorescence are a suitable option, because the level of a fluorescent signal emitted when they are excited with light of the suitable wavelength is easy to detect and quantify..

As for other reporter proteins, there are many commercially available vectors that can be the backbone for the construction of a luciferase reporter vector, such as the pGL4 or the pGL2 plasmids that can be purchased, for instance, from Promega (see https://www.promega.es/resources/product-guides-and-selectors/luciferase-reporter-vector-selector/ for pGL4 vectors and https://www.promega.es/products/reporter-assays-and-transfection/reporter-vectors-and-cell-lines/pgl2-luciferase-reporter-vectors/ for pGL2 vectors. The pGL2 Basic Vector can be a suitable backbone option, since expression of luciferase activity in cells transfected with this plasmid depends on insertion and proper orientation of a functional promoter upstream from the coding sequence of the luciferase, *luc* (https://www.promega.com/∼/media/files/resources/protocols/technical%20man uals/0/pgl2%20luciferase%20reporter%20vectors%20protocol.pdf). The expression vector used in Examples 3, 6 and 7 of the present application, pGL2-E2F-luc (abbreviated in the Examples as pE2F-Luc), is a derivative of such vector.

Regarding the promoter, it must contain an E2F binding sequence, such as TTTSSCGC where S is G or C (SEQ ID NO:43) (Kreck et al., 1993). Such minimal promoter is also suitable for the purposes of the present invention. A possible option is the E2F-dependent promoter used in the assays of the present application, which has been constructed using two oligonucleotides, one bearing a TATA box and the other containing three E2F wild type sites, and that were cloned into the plasmid pGL2BASIC (Promega). The TATA oligonucleotide is 5'-GATCTACTTGGGCATAAAAGGCAGAGCAGGGGCAGCTGCTGCTTACACTT A-3' (SEQ ID NO:) and the 3xE2F oligonucleotide is 5'-CTGCAATTTCGCGCCAAACTTGTGCAATTTCGCGCCAAACTTGTGCAATTT CGCGCCAACTTGC-3' (SEQ ID NO:45)

In those embodiments of the method of the invention where the expression of the reporter protein is assessed, as commented above, the coding sequence of the reporter gene will usually be contained in an expression vector, where it will be operatively linked to the E2F promoter. Transient expression is usually preferred, so that the reporter expression vector can be a plasmid, as in the Examples of the present application, or a viral vector (such as pWPI, pBABE). Methods for transient transfection of mammalian cells are well known for those skilled in the art. Many kits and reagents are commercially available for that purpose, as those used in assays of the present application.

Irrespective of the selected reporter expression vector, when the level of expression of a reporter gene operatively linked to an E2F promoter is determined, the method of the present invention comprises a previous step wherein both the cells to be contacted with the assayed compound and the control cell culture are also transfected with the same expression vector wherein the coding sequence of a reporter gene is operatively linked to a promoter that requires binding of E2F transcription factor for driving the transcription of the gene. Then, once the assay has been carried out, the assayed compound is identify as a candidate to drug for the control of cancer if the level of expression of the reporter gene is decreased in the cells that have been treated with the assayed compound with regard to the control cells.

Preferably, the cells used in the assay (both the control cells and the cells to be treated with the compound under assay) are also transfected with an expression vector of mammalian E2F1 (or E2F2 or E2F3) protein and with an expression vector of mammalian RB protein. Any expression vector suitable for expression in mammalian cells could be a suitable backbone for the construction of the corresponding expression vectors adequate for the purposes of the present invention. The coding sequence of the selected E2F protein (E2F1, E2F2 or E2F3) and RB of many mammals are known and available. In particular, the coding sequence of the corresponding human proteins are well known and are accessible in data bases, such as the HGNC database of human gene names of the HUGO Gene Nomenclature Committee (HGNC: http://www.genenames.org/), the GenBank database of the National Center of Biotechnology Information (NCBI), the ensemble project of genome databases (www.ensembl.org) or the database of protein sequences of UniProt (www.uniprot.org). The accession codes and identifications in each database for the corresponding genes and proteins are: a) for retinoblastoma, HGNC ID 9884 (approved symbol: RB1), Genbank accession M15400 version M15400.1 GI:190958, NCBI Reference sequence for mRNA NM_000321.2, Ensembl ENSG00000139687 Release of December 2015, UniProtKB ID for the protein P06400; b) for E2F1, HGNC ID 3113 (approved symbol: E2F1), NCBI Reference sequence for mRNA NM_005225, Ensembl ENSG00000101412 Release of December 2015, UniProtKB ID for the protein Q01094.

The expression vector can be a plasmid, as in the present Examples, and the coding sequence can be operatively linked to an inducible promoter that can drive the transcription only in certain conditions, or to a constitutive promoter such as CMV (human cytomegalovirus immediate-early) promoter, as in the plasmids pRC-HA-E2F1 (whose backbone is a pRC vector, pRC/CMV: https://tools.thermofisher.com/content/sfs/vectors/prccmv_map.pdf) and pCDNA3-9E10-RB (whose backbone is the pCDNA3 vector: https://tools.thermofisher.com/content/sfs/manuals/pcdna3_1_man.pdf), which are used in assays of the Examples of the present application. The coding sequences of E2F1 and RB contained in the pRC-HA-E2F1 and pCDNA3-9E10RB vectors are, respectively, those of SEQ ID NO:46 (E2F1) and SEQ ID NO:56 (RB). Said sequences give rise to the corresponding human proteins. But, as has been said, the homolog proteins of other mammals can be also be used for the method of the present invention, particularly in the case of RB. Mouse RB protein (mRNA with accession number NM_009029, protein sequence shown in SEQ ID NO:58) and human RB protein show a high percentage of homology and, specially, the phosphorylation sites of interest in the RBN domain are well conserved between both proteins, so that the assays of the present application and the method of the present invention could be carried out also with the mouse RB protein. This can be an interesting embodiment of the present invention, wherein also mouse cells such as MEFs could be used.

With regard to the E2F factor itself, it is important to point out that the assays carried out having transfected vectors expressing E2F1, E2F2 or E2F3 gave rise to no significant change in the luciferase activity detected upon conditions of NaCl stress (see Fig. 8G). Therefore, the coding sequences of any of said three factors can be used in the vectors designed for carrying out the method of the present application.

These and other coding sequences used for carried out the present invention, in its different aspects, can be linked to a tag for facilitating detection, isolation and purification of the protein, such as an HA-tag (amino acids 98-106 of the human influenza hemagglutinin: YPYDVPDYA (SEQ ID NO:48), as is the case for the E2F1 protein used in the luciferase assays herein presented. They can be also linked to other common tags, such as the myc-tag (EQKLISEEDL: SEQ ID NO:49) or the GST-tag (whole GST protein of 26kDa, SEQ ID NO:51), which has been used in the assays with RB expression vectors of the present application, where it has been shown that they do not interfere with its activity, but facilitate its isolation, detection and/or quantification thanks to antibodies directed to the tag itself.

Additionally, it is also advisable to transfect the cells also with a vector for expression of DP1 protein. Again, information about the human protein can be found in different data bases: HGNC ID 11749 (approved symbol: TFDP1), Genbank accession BC011685 version BC011685.2 GI:33877644, NCBI Reference sequence for mRNA NM_007111 Ensembl ENSG00000198176 Release of December 2015, UniProtKB ID for the protein Q14186). In the assays of the present application, the murine protein has been used (SEQ ID NO:59); carrying out the method of the present application with the murine protein is a possible embodiment of said method. The alternatives about the kind of vectors, promoters and expression conditions to be used are the same as above, as well as the preference for transient transfection and constitutive promoters. Again, there are available vectors that can be used directly, such as the plasmid usedin assays of the present application, pCDNA3-HA_DP1 (initially published by Kreck et al., 1993).

Transfection and expression of all E2F1, RB and DP1 expression vectors avoids false results due to shortage of one of the proteins. Preferably, all vectors to be transfected are mixed together before transfection and transferred in the same DNA preparation.

Finally, the increase in affinity of RB for E2F is another property that can be also used in the method of the present invention for assessing the effects on RB of a certain compound. Such possible increase in affinity can be determined through the determination of the level of RB-E2F complex in the control cells and in the cells treated with the compound under assay. This can be done by the use of antibodies directed either to the E2F or the RB component of the complex, separation of the proteins bound to the antibodies from the initial sample (for instance, by immunoprecipitation) and determination of the amount of the other component (RB or E2F, respectively) present in the samples obtained after such separation. Such approach can be carried out with the natural RB/E2F proteins present in the cells, or can be facilitated by previous transfection of all the cells (both the control cells and the cells to be treated with the compound under assay) with a vector expressing a tagged E2F1 protein (HA-E2F1, for instance), as in Example 4, so that the complexes RB-E2F can be immunoseparated or immunoprecipitated by the use of an antibody directed to the tag itself. The increase of affinity of RB for E2F can be also assessed through expression and purification in vitro of tagged (for instance with GST) RB protein (full-length or N-terminal fragment) and incubation with control cells and in the cells treated with the compound expressing HA-E2F as in Example 4. The interaction between these two proteins can be also assessed by expressing in *E. coli* and purifying the N-terminal fragment of RB and incubating it with extracts expressing HA-E2F; such incubation is done with and without assayed compound, similarly to the assayed described in Example 4 whose results are shown in Fig. 9C.

As has been explained previously, and irrespective of the specific property assessed, the present invention provides a method for determining whether a compound provokes the phosphorylation of human RB in Ser249/Thr252 (or the phosphorylation of the equivalent Serine and Threonine residues of another mammalian RB), or induces otherwise the same conformational change in RB, so that a similar effect of loss of CDK control and reduction of cancer cell proliferation is expected from said compound. Said method has as an additional advantage: that it can be carried out *ex vivo,* in cell cultures, without requiring necessarily the use of animal models for performing the preliminary identification of a compound as a good candidate to anticancer drug.

The cells used for carrying out the method of the invention can be different cells of common use among those skilled in the art. Given the high similarity among mouse RB and human RB proteins, even mouse cell such as MEF cells can be used, as in certain assays shown herein. Preferably, human cell lines such as HEK293T or HeLa will be used, because they are known to be cells easy to transfect, that express the plasmid products in high amounts.. When the effects on a specific cancer are preferred to be studied, specific cancer cell lines can be also used such as, for instance breast, pancreas or bladder cancer cell lines, as in Example 7, as well as lines associated to tumours in other organs, such as lung. The use of cancer cell lines with high CDK activity, as it is the case of the breast, pancreas or bladder cell lines used in Example 7, can be also an option. Such alternatives related to cancer cells can be particularly useful to assess the decrease of cancer cell proliferation when applying the method of the present invention or, particularly, when it is desired to have additional indications showing that the assayed compounds can be considered candidates for the treatment of breast, pancreas or bladder cancer, or cancer associated to other organs such as lung.The therapeutic use in cancer of the compounds identified by the method of the present invention, that is, a compound identified by the method of the invention for use in the treatment of cancer or, even more specifically, for use in the treatment of breast pancreas or bladder cancer or cancer of another different organ, is also encompassed by the scope of the present invention.

In the other hand, as mentioned above, the assays of Examples of the present application show that RB mutants harbouring mutations that mimic human RB phosphorylated in Ser249/Thr252 also have the effect of reducing the proliferation of cancer cells. Therefore, expression vectors capable to express such mutant RB proteins in cancer cells of a mammal suffering cancer can also be a tool for controlling cancer in said particular mammal. Thus, if the mammal to be treated is a human being, a mutant human RB with glutamic acid in positions 249 and 252 can be the expressed protein.

The expression vector where the coding sequence of the RB mutant is inserted can be any of the vectors commonly used for expression in mammals. Non-integrative viral vectors are preferred, such as attenuated adenovirus vectors or adeno-associated vectors. If the control of cancer in a particular organ is sought, the vector and/or the promoter will preferably chosen so that the vector has a particular tropism for the organ and/or that the promoter drives the expression of the operatively linked coding sequence particularly in that organ.

The expression vector could be administered to the mammal by a selected route, such as the systemic route, for instance by injection. For its administration, the vector will be formulated in a form suitable for the selected administration route. When the systemic route has been used, the vector could be administered as a composition, for instance suspended in water or in an aqueous solution such as saline solution as vehicle; pharmaceutically accepted excipients could be also present

The invention will be now explained in more detail with the help of the Examples and Figures that appear below.

### EXAMPLES

The assays of the Examples below were carried out with the following products and methodologies:

### - Cells, transfection and infection

HEK293T, wild type MEFs, p38alpha^{-/-} MEFs ⁻, RB^{-/-} MEFs and p57^{-/-} MEFs, MCF7, PK9, 235J and MDA-MB-231 were all cultured in Dulbecco's modified Eagle's medium (Biological Industries) containing 10% fetal calf serum (Sigma) and supplemented with 1 mM sodium pyruvate, 2mM L-glutamine, 100 U/ml Penicillin and 100 µg/ml Streptomycin (GibCO) and cultured in a 5% CO₂ humidified incubator at 37°C.

When indicated, cells were treated with 100-250 mM NaCl, 400-1200 µM H₂O₂ (SIGMA) or 5-50 ng/ml Anisomycin (SIGMA) for the appropriate times. When indicated, cells were incubated with 1 µm SB203580 (Calbiochem) for 30 min. prior to the treatments.

Cells were transiently transfected with the indicated plasmids using the FuGENE 6 transfection reagent (Roche Applied Science), Lipofectamin LTX or Lipofectamin 3000 (Invitrogen) according to the manufacturer's directions.

### - Plasmids and constructs

The following plasmids were used:

pGEX2TK-RB was provided by Dr. Xavier Mayol (IMIM, Barcelona); it is a plasmid of the pGEX-series of GST fusion vectors derived from pGEXT2TK plasmid (commercially available from Pharmacia and GE Healthcare Life Sciences; https://www.gelifesciences.com/gehcls_images/GELS/Related%20Content/Files /1314787424814/litdoc28959589_20140411171953.pdf where the full-length coding sequence of human RB is inserted so that a GST-RB fusion protein with thrombin cleavage site can be expressed from *E. coli.*

pGEX2TK-RB (aa 301-929) was obtained digesting the full-length pGEX2T-RB by *EcoR*I and the released *Eco*Rl C-terminal fragment was subcloned into the *Eco*RI site of pGEX2TK.

pGEX2TK-RB (aa 1-515) was obtained using the full-length pGEX2T-RB digested with *Nco*I*lSma*I*.* A C-terminal fragment was released and the remaining vector was blunt-ended and religated.

pGEX4T1-RB (aa 1-379) was obtained using the full GST-RB as template, amplified by PCR (CAT CAT CAT GGA TCC ATG CCG CCC AAA ACC CCC CGA (SEQ ID NO:1) and CAT CAT CAT GGA TCC TCA TTG TTG GAT AGT GGT (SEQ ID NO:2)) and cloned into *BamH*l site of pGEX4T1.

pGEX2TK-RB (aa 379-792) and pGEX2TK-RB (aa 379-928) were obtained from Addgene (https://www.addgene.org/21748/ and https://www.addgene.org/21749/, respectively).

The pCDNA3-9E10-RB was obtained using the full-length RB from pGEX2TK-RB as a template, amplified by PCR (CAT GGA TCC ATG CCG CCC AAA ACC CCC (SEQ ID NO:3) and CAT CTC GAG TCA TTT CTC TTC CTT GTT (SEQ ID NO:4)) and cloned into the *BamH*I/*Xho*I sites of pCDNA3-(https://tools.thermofisher.com/content/sfs/manuals/pcdna3_1_man.pdf), which contains a myc N-terminal epitope between the *Hind*III/*Bam*HI sites.

The RB mutants pCDNA3-9E10-RBT5A, pCDNA3-9E10-RBS230A, pCDNA3-9E10-RBS249A, pCDNA3-9E10-RBS249A/T252A, pCDNA3-9E10-RBS249E/T252E were generated by site directed mutagenesis using the Quickchange XL kit from Stratagene following the manufacturers' instructions and pCDNA3-9E10-RB as a template with the following mutagenesis primer pairs: **T5A** (ATG CCG CCC AAA GCC CCC CGA AAA ACG TAC (SEQ ID NO:5) and GGC GGG T TT **C**GG GGG GCT TTT TGC (SEQ ID NO:6)), **S230A** (TTT A TT AAA C TC **G**CA CCT CCC ATG TTG (SEQ ID NO:7) and AAA TAA TTT GAG CGT GGA GGG TAC AAC (SEQ ID NO:8)), **S249A** (CCC ATT AAT GGT GCA CCT CGA ACA CCC (SEQ ID NO:9) and GGG TAA TTA CCA CGT GGA GCT TGT GGG (SEQ ID NO:10)), **T252A** (GGT TCA CCT CGA GCA C CC AGG CGA GGT (SEQ ID NO:11) and CCA AGT GGA GCT CGT GGG TCC GCT CCA (SEQ ID NO:12)), **S249A/T252A** (GTT ATA C CC ATT AAT GGT **G**CA CCT CGA **G**CA C CC AGG CGA GGT CAG AAC (SEQ ID NO.13) and CAA TAT GGG TAA TTA CCA CGT GGA GCT CGT GGG TCC GCT CCA GTC TTG (SEQ ID NO:14)), **249A/252E** (GGT GCA CCT CGA GAA C CC AGG CGA GGT (SEQ ID NO:15) and CCA CGT GGA GCT CTT GGG TCC GCT CCA (SEQ ID NO:16)) **249E/252E** (C CC ATT AAT GGT GAA CCT CGA GAA C CC (SEQ ID NO:17) and GGG TAA TTA CCA CTT GGA GCT CTT GGG (SEQ ID NO:18)).

pMT3-HA-p38α was obtained from Addgene (https://www.addgene.org/12658/).

pGEX5x3-p38α was obtained amplifying p38α by PCR from pMT3-HA-p38 using the following oligonucleotides, 5-GCTAGTGGATTC ATG TCT CAG GAG AGG CCC-3 (SEQ ID NO:19)' and 5'-TCGATCTCGAGTCAGGACTCCATCTCTTC-3 (SEQ ID NO:20) and subcloned in the BamHI/Xhol restriction sites of pGEX5x3 plasmid.

pEFmlink-9E10-MKK6^{DD} construction is described in Alonso et al., 2000. In brief, MKK6 was amplified by PCR using oligonucleotides that created a *Nco*I*l* site at the ATG and an *Xho*I downstream of the stop codon and was cloned into to pEFmlink (https://www.thermofisher.com/order/catalog/product/V92120). The mutants MKK6^{DD} (the two phosphorylation sites Ser 207 and Thr 211 in the activation loop of MKK6 changed to Glu) were generated by site-directed mutagenesis.

pGEX2TK-MKK6^{DD}: MKK6^{DD} was amplified by PCR from pEFmlink-MKK6^{DD} using the following oligonucleotides: 5'-GCTAGTGAATTCATGTCTCAGTCGAAAGGC-3' (SEQ ID NO:52) and 5'-GTCGATGTCGACTTAGTCTCCAAGAATCAG-3' (SEQ ID NO:53) and subcloned in the *Eco*RI/*Sal*I restriction sites of pGEX2T1 plasmid.

pGEX2TK-ATF2 is described in Liu and Green, 1995. In brief, GST-ATF2 was constructed by fusing an appropriate DNA fragment in frame to the GST-sequence of the pGEX2TK plasmid.

pRC-HA-CDK4 (http://www.addgene.org/1876/), pRC-HA-CycD (http://www.addgene.org/8958/), pRC-HA-CDK2 (http://www.addgene.org/1884/), pRC-HA-CycA2 (http://www.addgene.org/8959/) and pRC-HA-CycE (http://www.addgene.org/8963/)
were a gift from Dr. Watson (Saville and Watson, 1998).

pGEX2TK (https://www.gelifesciences.com/gehcls_images/GELS/Related%20Content/File s/1314787424814/litdoc28959589_20140411171953.pdf), pGEX5x3 (http://www.gelifesciences.com/webapp/wcs/stores/servlet/catalog/en/GELifeSci ences-es/products/AlternativeProductStructure_16996/28954555), pEFmlink (https://www.thermofisher.com/order/catalog/product/V92120), pRC vectors (https://tools.thermofisher.com/content/sfs/vectors/prccmv_map.pdf) and pCDNA3 (https://tools.thermofisher.com/content/sfs/manuals/pcdna3_1_man.pdf) are commercially available.

pRC-HA-E2F1 can be found in addgene web site (https://www.addgene.org/21667/). Its construction was initially published by Kreck et al. (1993), and contains the coding sequence of E2F1 (SEQ ID NO:46) inserted in the pRc/CMV vector (https://tools.thermofisher.com/content/sfs/vectors/prccmv_map.pdf).

pGL2-E2F-luc is described (Kreck et al., 1993) and was a gift from Dr. Tauler. It was constructed using two oligonucleotides, one bearing a TATA box and the other containing three E2F wild type sites, were cloned into pGL2BASIC (Promega). The TATA oligonucleotide is 5'-GATCTACTTGGGCATAAAAGGCAGAGCAGGGGCAGCTGCTGCTTACACTT A-3' (SEQ ID NO:44) and the 3xE2F oligonucleotide is 5'-CTGCAATTTCGCGCCAAACTTGTGCAATTTCGCGCCAAACTTGTGCAATTT CGCGCCAACTTGC-3' (SEQ ID NO:45).

pCDNA3-DP1 is described in Kreck et al., 1993 and was a gift from Dr. Tauler. pCDNA3-HA-DP1 was created by cloning to the NH₂-terminal to the cDNA an oligonucleotide encoding the HA epitope (5'-GCTTACCATGGCCTACCCCTACGACGTGCCCGACTACGCCTCCCTCG-3': SEQ ID NO:54 and 5'-GATCCGAGGGAGGCGTAGTCGGGCACGTCGTAGGGGTAGGCCATGGTA-3': SEQ ID NO:55).

### - Antibodies

Anti-E2F1 (sc-251), anti-RB (sc-50), anti-CycA₂ (sc-596), anti-GAPDH (sc-32233) and anti-p38 (sc-535) were from SantaCruz. Anti-RB (9309), anti-p38alpha (9228), anti-phospho-S795-RB (9301), anti-phospho-S780-RB (9307), anti-phospho-p38 (9215), anti-phospho-S608-RB (2181) and anti-phospho-S807/811-RB (9308) were from Cell Signaling. Anti-RB (554136) was from BDPharmingen. Anti-α-tubulin (S9026) from was from Sigma. Anti-phospho-T373-RB (ab52975) and anti-RNA Pol II (8WG16) were from Abcam. Mouse monoclonal anti-HA and mouse monoclonal anti-myc were house made from the 12CA5 and 9E10 hybridomas, respectively.

Rabbit polyclonal antibodies specifically targeting RB phosphorylation at S249/T252 were generated by Genscript Corporation; antibodies were generated from the peptide NGSPRTPRRGQNRSC (human S249/T252 non-phosphorylated RB, SEQ ID NO:21), which was phosphorylated at amino acids 3 and 6 (being possible to represent it as NGpSPRpTPRRGQNRSC (SEQ ID NO:22)) when the antibody was for recognizing human S249/T252 phosphorylated RB). Horse Radish Peroxidase conjugated anti-rabbit and antimouse antibodies and the Enhanced Chemiluminiscence kit were from GE Healthcare.

### - Bacterial expression and purification of recombinant proteins

*E. coli* cells were grown at 37°C until they reached an OD₆₀₀ of 0.5 and GST-fused proteins were induced for 4 hours by adding 1 mM IPTG and switching the culture temperature to 25°C. After induction, cells were collected by centrifugation and resuspended in 1/50th volume of STET 1 x buffer (100 mM NaCl, 10 mM Tris-HCl pH 8.0, 10 mM EDTA pH 8.0 and 5% Triton X-100 supplemented with 2 mM DTT, 1 mM PMSF, 1 mM Benzamidine, 200 µg/ml Leupeptine and 200 µg/ml Pepstatine). Cells were lysed by ice-cold brief sonication and cleared by high speed centrifugation. GST-fused proteins were pulled down from supernatants with 300 µl of gluthatione-sepharose beads (GE Healthcare, 50% slurry in equilibrated with STET) by mixing 45 min. at 4°C. The gluthatione-sepharose beads were collected by brief centrifugation and washed four times in STET 1 x buffer and two times in 50 mM Tris-HCl pH 8.0 buffer supplemented with 2 mM DTT. The GST-fused proteins were then eluted in 200 µl of 50 mM Tris-HCl pH 8.0 buffer supplemented with 2 mM DTT and 10 mM reduced gluthatione (Sigma) by mixing for 45 min at 4°C and stored at -80°C.

### - In vitro p38 SAPK and Cdk2 kinase assays

GST-p38alfa SAPK was activated *in vitro* in a small volume (15 µl/assay) by mixing with GST-MKK6^{DD} in 1 x kinase assay buffer (50 mM Tris-HCl pH 7.5, 10 mM MgCl₂, 2 mM DTT) in the presence of 100 µM cold ATP plus/minus 10 µM SB203580 (Calbiochem) for 20 min at 30°C. 15 µl of the activated GST-p38 SAPK was used to phosphorylate *in vitro* either eluted GST-fused RB from *E*. *coli* or immunoprecipitated RB from mammalian cells. The reactions were carried in 1 x kinase assay buffer in the presence of 1 µCi/assay of radiolabelled ³²P-γ-ATP (3000 Ci/mmol from Perkin-Elmer) in a final volume of 40 µl/assay for 20 min. at 30°C.

Cdk2/CycA complexes from Millipore (14-448) were incubated in 1 x kinase assay buffer in the presence of 50 µM cold ATP in order to phosphorylate GST-fused RB proteins for 20 min. at 30°C. Reactions were stopped by adding SB5X (250 mM Tris-HCl pH 6.8, 0.5 M DTT, 10% SDS, 20% glycerol, 0.5% Bromophenol Blue) and boiling at 100°C for 5 min. Phosphorylated proteins were subjected to PAGE-SDS and Coomassie blue stained or transfer blotted onto a PVDF membrane and exposed to BIOMAX XAR films (KODAK).

### -Luciferase reporter assays

Cells were transfected with E2F-luc (plasmid pGL2-E2F-luc), HA-E2F1 (plasmid pRC-HA-E2F1), HA-DP1 (plasmid pCDNA3-HA-DP1) in the presence or absence of myc-RB, myc-RB^{249A/T252A}, myc-RB^{249E/252E}, HA-CDK2, HA-CDK4, HA-CycE, HA-CycA₂ (plasmids pCDNA3-9E10-RB, pCDNA3-9E10-RBS249A/T252A, pCDNA3-9E10-RBS249E/T252E, pCMV-HA-CDK2, pCMV-HA-CDK4, pCMV-HA-CycE, pCMV-HA-CycA₂, respectively) as indicated in the figures Fig. 7A-B and Fig. 8A-B. Treated cells were washed twice with cold PBS and lysed with 1 × cell culture lysis reagent (Promega). Cell lysates were cleared by microcentrifugation. Luciferase reporter activity was measured from cell supernatants using a Luciferase Reporter Assay kit (Promega) and a Microlumat LB 960 luminometer (Berthold Technologies). The total amount of protein found in the cell extracts was measured with the Bradford reagent (Bio-Rad). Protein-corrected luciferase reporter activities are shown.

### - Real Time (RT)-PCR and mRNA Analysis

Total RNA was purified from MEFs cells using the RNeasy kit (Qiagen) following the manufacturer's instructions. 100 ng of total RNA was then converted to cDNA using the reverse transcriptase Superscript kit (Invitrogen) according to the manufacturer's protocol. cDNA was analyzed by real-time PCR using a DNA Biosystems 7700 sequence detector and the SYBR Green kit (Applied Biosystems). Real-time PCRs were performed in triplicates and referenced to the GAPDH mRNA levels. The following oligonucleotides were used: mad2 (AGG ATG AAA TTC GCT CAG and CAT TGA CAG GGG TTT TGT, SEQ ID NO:23 and SEQ ID NO:24 respectively), E2F1 (GGA TCT GGA GAC TGA CCA and CTC CAG GAC ATT GGT GAT, SEQ ID NO: 25 and SEQ ID NO:26 respectively), BRCA1 (ACT GAA AGG CAT CCA GAA and GAA CCT GCC TGT CGT TAC, SEQ ID NO: 27 and SEQ ID NO:28 respectively), CycA₂ (GTC CTT CAT GGA AAG CAG and ACG TTC ACT GGC TTG TCT, SEQ ID NO: 29 and SEQ ID NO:30 respectively), CycE1 (CCT CCA AAG TTG CAC CAG TT and GGA CGC ACA GGT CTA GAA GC, SEQ ID NO:31 and SEQ ID NO:32 respectively), GAPDH (AAT TCA ACG GCA CAG TCA AGG and GGA TGC AGG GAT GAT GTT CTG, SEQ ID NO:33 and SEQ ID NO:34 respectively).

### - Chromatin Immunoprecipitation (CHIP) Assays

Protein-DNA interactions were cross-linked in cell cultures by addition of 1% (v/v) formaldehyde (Sigma) for 20 min. at room temperature. Cross-linking was stopped by the addition of 0.125 M glycine for 5 min. Cells were washed and harvested in PBS containing 4 µg/ml Complete Protease Inhibitor (Roche Applied Science) and then lysed on ice for 10 min. in 50 mM Tris-HCl, pH 8.1, 1% (w/v) SDS, 10 mM EDTA containing 4 µg/ml of Complete Protease Inhibitor Mixture. Lysates were sonicated in a Bioruptor water bath (Diagenode) set at full power (0.5 min. sonication/0.5 min. resting intervals at 4°C for 12 min.). Samples were centrifuged and the chromatin was quantified from the supernatants with a nanodrop apparatus. 10% of the volume was retained as an input, and ∼40 µg of chromatin was used for IP and diluted in ChIP buffer (6.7 mM Tris-HCl, pH 8.1, 0.01% SDS, 1.1% Triton X-100, 1.2 mM EDTA, 167 mM NaCl supplemented with 4 µg/ml Complete Protease Inhibitor Mixture). Dynabeads (Invitrogen) were conjugated overnight at 4°C with the appropriate antibody before being added to the diluted cell extracts. After a further overnight incubation at 4°C, dynabeads were serially washed with the following buffers: low ionic strength (120 mM Tris-HCl, pH 8.1, 0.1% SDS, 1% Triton X-100, 2 mM EDTA, 150 mM NaCl), high ionic strength (500 mM NaCl), LiCl buffer (10 mM Tris-HCl, pH 8.1, 250 mM LiCl, 1% Nonidet P40, 1% deoxycholate, 1 mM EDTA) and TE (10 mM Tris-HCl, pH 8.1, 1 mMEDTA). Protein-DNA complexes were eluted from the dynabeads by incubation at room temperature with elution buffer (0.1 M NaHCO₃, 1% SDS). Protein-DNA cross-linking was reversed by adding 200 mM NaCl and incubated for 4 hours at 65°C and 1 hour at 45°C in 40 mM Tris-HCl, pH 6.5, 10 mM EDTA, and 20 µg of proteinase K solution (Novagen). DNA was then extracted with phenol/chloroform and precipitated. Immunoprecipitated DNA fragments were analyzed by PCR or RT-PCR as described above. PCR primers for individual genes used are the followings: human CycA₂ (CCC CAG CCA GTT TGT TTC and TCA AGT TCA TAG, SEQ ID NO: 35 and SEQ ID NO:36 respectively), human E2F1 (GCG TTA AAG CCA ATA GGA and CTT TTA CGC GCC AAA TC, SEQ ID NO: 37 and SEQ ID NO:38 respectively), mouse CycA₂ (CTC CCG CCC TGT AAG ATTC and AGT TCA AGT ATC CCG CGA CT, SEQ ID NO: 39 and SEQ ID NO:40 respectively) and mouse CycE1 (CGC AGG CCC TGA CAT CTA and ACC AGC GAG GAG CTC TG, SEQ ID NO:41 and SEQ ID NO:42 respectively). RT-PCRs were performed in triplicates, referenced to the inputs, and represented as fold induction over the mock transfected cells. Negative controls were also made in the absence of DNA to rule out nonspecific DNA amplifications. In ChIPs on tagged proteins, the present inventors carried out a mock ChiP where the corresponding empty vector plasmid had been transfected; any amplification coming from the mock IP is regarded as background. In ChiPs on endogenous proteins, the present inventors carried out either a no-antibody control or an anti-α-tubulin antibody.

### - Western blot and immunoprecipitation assays

Transfected cells were washed with ice-cold PBS and scraped into 500 µl of IP/lysis buffer (10 mM Tris-HCl pH 7.5, 1% NP-40, 2 mM EDTA, 50 mM NaF, 50 mM β-glycerolphosphate, 1 mM Sodium Vanadate, 1 mM PMSF, 1 mM Benzamidine, 200 µg/ml Leupeptine and 200 µg/ml Pepstatine). The lysates were cleared by microcentrifugation and 10% retained as input. The remainders were subjected to immunoprecipitation with either 5 µl of agarose-conjugated anti-HA, anti-myc or 50 µl sepharose-protein A beads (GE Healthcare, 50% slurry equilibrated in IP buffer) coupled to specific antibodies by mixing overnight at 4°C. Immune complexes were collected by brief centrifugation and washed rapidly three times with IP buffer. Immunoprecipitates and the input samples were subjected to PAGE-SDS and Western blotting.

### - Immunocytochemistry

Cells were grown in chamber slides and fixed in 95% ethanol, 5% acetic acid for 10 min. and permeabilized with 1% formaldehyde, 0.25% Triton X-100 in TBS for 5 min. Fixed cells were blocked with 3% BSA/TBS for 30 min. The primary antibodies were incubated at 1/50 dilution in blocking buffer overnight. The secondary anti-rabbit IgG-FITC antibody (Sigma, F0382) was used at 1/100 dilution in blocking buffer for 1 hour.

### - Cell cycle, cell viability and colony formation assays

Exponentially growing wild type, RB^{-/-} MEFs and MCF7 were stressed with NaCl, H₂O₂ or anisomycin as indicated on the figures. One hour later, nocodozale (Sigma) was added to a final concentration of 100 ng/ml to trap the cells at the G2/M phase. DNA was labelled *in vivo* by incubating cells with 8 µM of Hoechst 33342 (Sigma) for 1 hour before being trypzinized and collected for cell-cycle FACS analysis. Cell viability upon stress was assessed by labelling living cells with 1 µg/ml propidium iodide (PI) (Sigma) for 10 min followed by FACS analysis. The stained cells were acquired on an LSR flow cytometer (Becton Dickinson) using the CellQuest software. Cell cycle profiles and viability were then analyzed with the WinMDI software.

For colony formation assays, cells were transfected with the indicated constructs. Cells were tryspinized, counted and then seeded out in appropriate dilutions to form colonies in 1-2 weeks. Colonies were fixed with paraformaldehyde (3.7%) for 10 min. and then stained with crystal violet (0.05%) for 10 min., washed twice with water and left inverted until dried. Colony area occupancy was analyzed using ImageJ software.

### - In silico modelling

In the assays set forth in the present application, it has been attempted to model the structure of the N-terminal domain region 244-269 of human RB with three different approaches due to the lack of templates with similarity and percentage of identity sufficient to be used as templates. First, a model from Swiss-Model server (Biasini et al., 2014) for the whole sequence of RB and selected the N-terminal region (aa 244-269) was assessed; second, the threading approach of iTASSER (Yang and Zhang, 2015) to model this region was used; and third, it was hypothesized a potential alignment of the N-terminal loop with the C-terminal region of RB (aa 829-852) which structure is known. The structure of the RB C-terminal domain bound to E2F1/DP1, obtained by X-ray crystallography at 2.55 A resolution is encoded in chain C of 2AZE of PDB (Protein Data Bank) (Rubin et al., 2005; Rose et al., 2011). Following the third approach and considering that the RB N-terminal binds E2F1/DP1, the RB N-terminal loop was built by comparative modelling with MODELLER (Eswar et al., 2006), using the structure of the complex of RB with E2F/DP1 (code 2AZE in PDB) as template, and modelled to analyze the potential interaction with E2F1/DP1. The electrostatic potential of the surfaces of the RB C-terminal and N-terminal sMotif were compared facing the surface of E2F1/DP1 complex by solving the Poisson-Boltzmann equation of the potential with a finite elements approach. The loop between the β-strand and the helix of the N-terminal region was refined with loopmodel (Fiser et al., 2000) with the only restraints at the positions of the flanking secondary structures, thus allowing for the increase of flexibility upon binding with E2F1/DP1.

In addition to the native sequence of the N-terminal side sMotif, the potential mutations of the N-terminal region that could affect the interaction were also modelled. The RB N-terminal phosphorylable residues (Ser249 and Thr252) were mutated *in silico* to glutamate in order to mimic the effect of a negative charge caused by the phosphorylation. Also S249A/T252A was considered for the sake of comparison. In total, we tested the interaction of the N-terminal and C-terminal sMotifs of RB with E2F1/DP1 for two native and mutant forms, as summarized below in Table 1.

**Table 1**

| **Code** | **Complex Form** |
|---|---|
| WT-Ct | Wild Type C-terminal sMotif of RB in complex with E2F1/DP1 |
| WT-Nt | Wild type N-terminal sMotif of RB in complex with E2F1/DP1 |
| S249E/T252E | N-terminal sMotif of mutant form S249E and T252E of RB in complex with E2F1/DP1 |
| S249A/T252A | N-terminal sMotif of mutant form S249A and T252A of RB in complex with E2F1/DP1 |

A total of 2500 models with unrestricted flexible refinement of the loop region of the sMotif were built using MODELLER (Eswar et al., 2006) and loopmodel approach for each form in the table. Also for the sake of comparison, we included 50 models for each form applying the native restrictions on the loop conformation. All modelled interactions were assessed with DOPE-scores (Marti-Renom et al., 2002) and the structures in the top 1% ranking with minimum DOPE energy were selected, while the rest of models were discarded. The interactions of RB N-terminal and C-terminal sMotifs with E2F/DP1 were scored with ΔΔG of Rosetta (InterfaceAnalyzer (Stranges and Kuhlman, 2013)). Among the top 1% best conformers according to DOPE, the best pose interaction between the sMotif and the complex E2F1/DP1 of each sequence under study were selected. Distribution of scores was plotted with matplotlib (Hunter, 2007) and distributions were compared using the Mann-Whitney-Wilcoxon test using SciPy library for python (Oliphant, 2007). USCF Chimera package (Yang et al., 2012) was used for visual inspection of the models and for the graphical representations. Electrostatic potentials over the accessible surface of E2F/DP1 were calculated with Adaptative Poisson-Boltzmann Solver (APBS) software (Baker et al., 2001) using PDB2PQR to assign the topology (Dolinsky et al., 2007).

### - Statistical Analysis

Data analysis was carried out with Prism software (GraphPad). Responses among different treatments were analyzed with one-way analysis of variance followed by Bonferroni's multiple comparison test. All experiments were carried out in triplicate. The results are presented as means ± S.D.

### - Example 1: Down-regulation of cyclin expression upon stress is mediated by p38 SAPK and RB transcriptional repressor

To assess the effect of p38 and stress in cyclin expression, the present inventors followed expression of cyclins and E2F-dependent genes in wild type, p38α^{-/-} and RB^{-/-} KO MEFs (Mouse Embryonic Fibroblasts). Wild type, p38^{-/-} and RB^{-/-} MEFs were stressed with 100 mM NaCl, 400 µM H₂O₂ or 25 ng/ml anisomycin, which are different types of stresses that are known to induce p38 activation. Upon exposure, the present inventors monitored the expression of several genes under the control of E2F in the G1/S transition (e.g. CycA₂ and E2F1 genes) at different times (0, 2, 4, 6, 8 hours). Upon stress, there is a clear down-regulation of the expression of E2F-dependent target genes that is not observed in p38^{-/-} KO cells (Fig. 2A and 3A). Correspondingly, CycA₂ and E2F1 protein levels also decreased depending on p38 upon stress (Fig. 3B).

To confirm the involvement of p38 in cyclin down-regulation, the present inventors genetically activated p38 by expressing the constitutively active form of MKK6 (MAPKK; MKK6^{DD}) (Joaquin et al., 2012), a protein kinase that activates p38 by phosphorylation (EC 2.7.12.2). Induction of p38 activation by MKK6^{DD} resulted in cyclin A and E2F1 down-regulation that was completely dependent on the presence of p38 (Fig. 3C). Thus, p38 SAPK mediates down-regulation of E2F-dependent genes upon stress.

RB is a key repressor of E2F gene expression (Dick and Rubin, 2013) and, as commented above, previous analysis suggested a connection between p38 and RB. Thus, the present inventors assessed whether down-regulation of cyclin and E2F-dependent genes expression upon stress or genetically activated p38 was mediated by RB . As for p38^{-/-} cells, down-regulation of cyclin expression was not observed in RB^{-/-} KO MEFs in such conditions (Fig. 2A, 3A, 3B and 3C). Thus, the present inventors hypothesized that p38 might be mediating cyclin down-regulation through RB regulation.

### - Example 2: The p38 SAPK interacts with and phosphorylates Ser249 and Thr252 at the N-terminal region of RB

Most SAPKs interact with their corresponding substrates in cells. Thus, the present inventors tested whether p38 was able to interact with RB by performing immunoprecipitation experiments in extracts from HEK293T cells expressing Myc-tagged RB and HA-tagged p38. Cell lysates were immunoprecipitated with either anti-myc or anti-HA sepharose beads and analyzed by Western blot.

Binding of HA-p38 was observed when Myc-RB was precipitated from cell extracts (Fig. 4A). Correspondingly, Myc-RB was also able to co-immunoprecipitate when HA-p38 was precipitated using anti-HA antibodies (Fig. 4A). Notably, endogenous RB also interacted with endogenous p38 SAPK (Fig. 4B). By using specific antibodies against endogenous RB and p38 proteins, the present inventors were able to confirm the interaction of the two proteins in MEFs, which was abolished in RB^{-/-} or p38^{-/-} deficient cells (Fig. 4B). These results show that the RB and the p38 SAPK do interact *ex vivo* and form a stable complex.

To assess whether RB was a direct target for the p38 SAPK, the present inventors subjected RB to an *in vitro* kinase assay expressing and purifying from *E. coli* lysates the following glutathione S-transferase (GST) fused proteins: GST-RB, GST-p38α, GST-MKK6^{DD} and GST-ATF2 (Fig. 4C). The proteins are expressed in a pGEX vector, with the GST moiety located at the N-terminus followed by the target protein. The fusion protein is captured by immobilized glutathione and impurities are washed away. ATF2, a well characterized p38 substrate, was used as a positive control. Purified GST-p38α was activated by mixing it with GST-MKK6^{DD} in the presence of cold ATP. Once activated, GST-p38α was used to phosphorylate *in vitro* eluted GST-fused RB in the presence of radiolabelled ³²P-γ-ATP. Coomassie panel shows the total amount of loaded proteins where autoradiography (Autorad in the figure) panel shows the amount of γ-³²P incorporated on each protein indicating that both RB and ATF2 were phosphorylated *in vitro*. These results show that *in vitro* activated p38 SAPK was able to phosphorylate RB fused to GST and expressed in bacteria as efficiently as known targets of p38 such as ATF2.

As human RB has 929 amino acids and contains 16 putative S/TP MAPK consensus sites, the present inventors generated two RB truncated variants; the N-terminal containing six SAPK consensus serine/threonie proline (SP/TP) sites (aa 1-515) and the C-terminal containing twelve S/TP sites (aa 301-929) (see Fig. 5A) *In vitro* kinase assays showed that the N-terminal RB fragment was *in vitro* phosphorylated by p38 to the same extent as the full-length protein whereas the C-terminal fragment was not phosphorylated (Fig. 5A, right part).

Systematic *in vitro* analysis of single and double mutants (S249A/T252A, S249A and T252A mutants) of the S/TP sites found at the RB N-terminal fragment (GST-RB (1-515) fragment, used as control) in the presence of activated GST-p38α, yielded that the combined mutation of Ser249 and Thr252 to Ala abolished phosphorylation by p38 (see Fig. 5B): p38 phosphorylated GST-RB (1-515) at S249/T252 *in vitro,* while the GST-RB (1-515)^{S249A/T252A} mutant was not phosphorylated.

The present inventors then created a Myc-tagged full-length RB that contained mutations on Ser249 and Thr252 or both to Ala and transfected and expressed them in HEK293 cells. Immunoprecipitated RB was then incubated with active purified p38 from *E. coli,* in order to carry out an *in vitro* kinase assays. SB20358 p38 inhibitor was used as a control. As can be seen in Fig. 6A, wild type RB is phosphorylated by GST-p38α, whereas this phosphorylation was reduced in RB^{S249A} and RB^{T252A} and the combined mutations at Ser249 and Thr252 completely abolished phosphorylation of RB by p38.

The group of the inventors then created rabbit polyclonal antibodies against the double phosphorylated Ser249 and Thr252 sites. The antibodies only recognized peptides with the double phosphorylation (Fig. 5C) and were validated by assessing phosphorylation of full-length RB *in vitro* by p38 (Fig. 5D).

The present inventors then assessed phosphorylation of RB *ex vivo* using those antibodies in wild type, RB^{-/-} and p38^{-/-} MEF cells. Induction of p38 activation by MKK6^{DD} expression or by stress (100 mM NaCl, 400 µM H₂O₂ or 25 ng/ml anisomycin for 2 hours, in the presence or absence of 10 µM SB203580) led to RB phosphorylation at Ser249 and Thr252 sites, except in the presence of SB203580 (Fig. 6B and 6C, respectively).

To assess antibody specificity *ex vivo,* the present inventors expressed Myc-tagged wild type and a double RB mutant (RB^{S249A/T252A}) in RB^{-/-} cells and subjected them to stress or expressed MKK6^{DD} as before. Only wild type RB was phosphorylated upon p38 activation (Fig. 6D and 5E). Phosphorylation of RB by p38 upon stress (Anisomycin) was also observed using immunofluorescence in wild type in contrast to RB^{-/-} and p38^{-/-} MEF cells (Fig. 6E).

Therefore, p38 phosphorylates RB at Ser249 and Thr252 in response to stress.

### - Example 3: Phosphorylation of RB by p38 increases RB transcriptional repression of E2F-dependent promoters

To assess the relevance of RB phosphorylation by p38 the present inventors set up an *ex vivo* reporter assay for RB activity using an E2F reporter system (Kreck et al., 1993). Briefly, HEK293T cells were transfected with a luciferase E2F reporter plasmid (pE2F-Luc) together with HA-E2F1 (transcription factor), HA-DP1 (co-activator) and wild type or mutants (RB^{S249A/T252A} and RB^{S249E/T252E}) Myc-tagged RB. Cells were subjected to stress (NaCl, H₂O₂ or Anisomycin) for four hours in the presence or absence of a p38 inhibitor (SB203580).

Upon stress, E2F reporter expression was strongly repressed and this repression was completely dependent on the activity of p38 since it was suppressed in the presence of p38 inhibitor (Fig. 7A and Fig. 8A, where it is demonstrated that the E2F-luc reporter monitors RB activity). It is worth mentioning that the *ex vivo* reporter assay for RB activity using and E2F reporter system (Kreck *et al.,* 1993) can be carried out expressing E2F1, E2F2 or E2F3 obtaining similar results (Fig. 8G).

Then, the present inventors assessed whether the p38-phosphorylation sites on RB were relevant for E2F-dependent transcription repression upon stress by expressing the unphosphorylatable RB^{S249A/T252A} mutant and found that this mutant was unable to repress transcription upon stress when compared with wild type (Fig. 7B). Of note, both proteins were expressed and able to repress E2F-dependent transcription similarly in the absence of stress (Fig. 8A) as well as were inactivated by overexpression of the CDK4 kinase (Fig. 8B, where it can be seen how E2F-luc reporter monitors RB inactivation by CDKs). Single mutations of Ser249 or Thr252 to Ala did not suppress the repression of E2F-dependent transcription observed in the double mutant upon stress (Fig. 8C) confirming that mutation to alanine of both S249 and T252 is needed to turn RB insensitive to p38 modulation.

To recapitulate the results from the reporter system the present inventors assessed expression of the endogenous cyclin A (CycA₂) or E2F1 genes in RB^{- /-} cells expressing wild type or mutant RB^{S249A/T252A}. Correspondingly, CycA₂ expression was repressed upon stress and this repression was not observed when p38 was inhibited (Fig. 7C) or in the presence of the unphosphorylatable RB protein (Fig. 7D, where it can be seen that CycA₂ down-regulation upon stress is rescued in RB^{-/-} MEFs transfected with wild type myc-RB but not with myc-RB^{S249A/T252A}). Similar results were obtained when E2F1 expression was assessed (Fig. 8D). Therefore, N-terminal phosphorylation of RB by p38 represses transcription of E2F-dependent genes upon stress.

RB inhibits transcription by associating to E2F-dependent promoters, thus it was formally possible that inhibition of E2F-dependent transcription was caused by an increased association of RB to E2F promoters (Dick and Rubin, 2013). Initially, the present inventors followed association of RNA Pol II to the CycA₂ promoter in wild type and RB^{-/-} cells. As expected, RNA Pol II association decreased upon stress and this decrease was not observed in RB^{-/-} cells (Fig. 8E). Then, the present inventors followed RB association to CycA₂ promoter by ChIP analysis and found that, in the presence of stress, RB association clearly increased depending on p38 activation (Fig. 7E, where it can be seen that stress increases myc-RB recruitment at the CycA₂ promoter). The increase on association of RB to CycA₂ promoter upon stress was not observed in the unphosphorylatable RB mutant (Fig. 7F, where it can be seen that S249/T252 phosphorylation increases myc-RB at the CycA₂ promoter). Similar results were obtained when RB binding to E2F1 promoter was assessed (Fig. 8F, where it can be observed that phosphorylation of RB upon stress increases myc-RB recruitment at the E2F1 promoter). These results indicate that the decrease in gene expression observed upon RB phosphorylation is caused by an increase of the association of RB with E2F-dependent promoters.

As phosphorylation of RB by p38 mediates gene repression upon stress, then it would be formally possible that a mutant that mimics such phosphorylation could repress E2F-dependent transcription even in the absence of stress or p38 activation. The present inventors created an RB mutant that replaced Ser249 and Thr252 to Glu (RB^{S249E/T252E}) and expressed in HEK293T cells. Expression of this allele was similar to wild type or RB^{S249A/T252A} mutant. Remarkably, RB^{S249E/T252E} inhibited expression of the E2F-dependeny reporter at similar levels to those observed upon stress (Fig. 7B) and no significant differences were observed in the presence or absence of stress (Fig. 8C). Of note, CycA₂ and E2F1 expression from the endogenous genes was inhibited by the RB^{S249E/T252E} allele (Fig. 7D and 8D). Correspondingly, RB^{S249E/T252E} associated to endogenous promoters similarly to wild type RB in the presence of stress (Fig. 7F and 8F).

Thus, even in the absence of stress, the N-terminal phosphorylation of RB leads to E2F-dependent transcriptional repression.

### - Example 4: N-terminal phosphorylation of RB increases its affinity towards E2F

The association of RB with E2F is mainly mediated by the pocket domain and the C-terminal regions. Thus, the present inventors studied whether the phosphorylation of the N-terminal RB could stimulate a conformational change that lead the association of the N-terminal domain with E2F.

None of the crystal structures published of RB contains the loop with Ser249 and Thr252, possibly for being in a highly disordered region (Hassler et al., 2007; Burke et al., 2010; Burke et al., 2012). Due to the lack of templates, the structure of the RB N-terminal domain (region 245-269) was initially modelled. Both the best model of iTASSER (Yang and Zhang, 2015) and the modelling of Swiss-Model yielded a structure formed by a coiled region (with a tendency to a β-stand) and a α-helix. Remarkably, this super-secondary structure is similar to the C-terminal region that bounds E2F1/DP1 (sMotif βα, MCL.EH.4M.9 and similar topologies in ArchDB (Bonet et al., 2014)) (Fig. 9A, where it can be seen that the modelled sequence at the N-terminal sMotif is aligned with a large degree of similarity with this C-terminal sMotif).

According to this, we hypothesize that the N-terminal region is able to bind E2F1/DP1 similarly as the C-terminal region does. To analyze this potential interaction, the RB N-terminal loop was built by comparative modelling with MODELLER (Eswar et al., 2006), using the structure of the complex of RB with E2F/DP1 (code 2AZE in PDB) as template. The alignment between the RB N-terminal region and the RB C-terminal sMotif maintains the key elements of the RB C-terminal strand-loop-helix motif. RB N-terminal phosphorylable residues Ser249 and Thr252 were aligned with Ser834 and Glu837 respectively. The alignment also anticipated the amphypatic behaviour of the helix in the RB N-terminal region, preserving the hydrophobic residues required for the core and the distribution of charged residues emulating the RB C-terminal sMotif (Fig. 9A).

In addition to the native sequence of the N-terminal side sMotif, the potential mutations that could affect the interaction with E2F1/DP1 were also modelled by using MODELLER and loopmodel approach. Thus, the RB N-terminal phosphorylable residues (S249 and T252) were *in silico* mutated to glutamate in order to mimic the effect of a negative charge caused by the phosphorylation. Then, all modelled interactions of RB N-terminal (245-269^{S249E/T252E}) sMotifs with E2F/DP1 were assessed with DOPE-scores (Marti-Renom et al., 2002) and selected the top 1% best conformers to score them with ΔΔG of Rosetta (InterfaceAnalyzer (Stranges and Kuhlman, 2013)). Distribution of ΔΔG energies showed that a double mutation S249E/T252E improved the binding affinity towards E2F with respect to the native sequence. For the sake of comparison, we also modelled the mutant form S249A/T252A, which had worst binding affinity than S249E/T252E, and modelled with the same approach the C-terminal loop of RB (829-82), which showed the best affinities (Fig. 9B). Electrostatic potentials of the complexes of E2F1/DP1 with the different peptide sequences showed that the helix of the RB C-terminal sMotif is amphipathic with a positive polar face and a hydrophobic face in contact with the strand forming the sMotif core and with the pocket formed by E2F1 and DP1. Again, the alignment between the RB N-terminal region and the RB C-terminal sMotif maintains the key elements of the RB C-terminal strand-loop-helix motif with a better similitude with the RBS249E/T252E than wild type or RBS249A/T252A mutant.

Based in the predictions from the modelling, the N-terminal region of RB should be able to interact with E2F and this interaction should improve by phosphorylation or in the RB^{S249E/T252E} mutant. To test this, initially, GST-purified N-terminal RB from *E. coli* (RB (1-379)) was incubated *in vitro* with cell extracts expressing or not HA-E2F. N-terminal RB was able to interact with E2F. Of note, the interaction of the N-terminal RB with E2F increased when RB was phosphorylated *in vitro* by p38 (Fig. 9C, where it can be seen that p38 phosphorylated RB (1-379) increased affinity towards HA-E2F1, after immunoprecipitation with anti-HA sepharose beads). Of note, the interaction with E2F1 of the N-terminal RB containing S249E/TS52E mutation (RB (1-379)^{S249E/T252}E), was stronger than wild type or the non phosphorylated mutant (RB (1-379)^{S249A/T252A}) mutant (Fig. 9D), showing that the phosphomimetic mutant of RB (1-379) has more affinity towards E2F1.

Then the present inventors assessed whether the phosphorylation of full-length RB by p38 increased the affinity of RB for E2F in *ex vivo* conditions. Cells expressing HA-E2F1 were transfected with MKK6^{DD} and p38 in the presence or not of the p38 inhibitor (SB). RB precipitated more efficiently when RB was phosphorylated by p38 (Fig. 9E), showing that p38-phosphorylated RB has increased affinity towards HA-E2F1. Similar results were obtained when p38 was activated by stress in cultured cells, specifically by incubation with 100 mM NaCl (Fig. 9F). Remarkably, the interaction of full-length RB^{S249E/T252E} with E2F in non-stressed conditions was similar to phosphorylated RB upon stress and stronger than RB^{S249A/T252A} (Fig. 9F).

These results confirmed that the phosphorylation of RB by p38 or the RB phosphomimic mutant displays a stronger affinity towards E2F. Taken together, the phosphorylation of RB by p38 leads to an increase on the affinity of the N-terminal towards E2F resulting in a more efficient transcriptional repressor.

### - Example 5: RB phosphorylation leads to cell cycle delay and increased cell survival upon stress

Cells delay G1 upon stress depending on p38 (Joaquin et al., 2012a). Since phosphorylation of RB by p38 induced a reduction on E2F-dependent gene expression, the present inventors analyzed whether RB was important to mediate a cell cycle delay in G1 upon stress.

Wild type and RB^{-/-} MEFs cultures were subjected to different stresses (NaCl, H₂O₂ or Anisomycin) and cell cycle progression was followed by FACS. Nocodazole was added 1 hour after stress to trap the cells at G2/M and the percentages of cells that progressed from G1 to G2 after 8 hours were assessed. As expected, cells remained in G1 in wild type cells upon stress,, whereas there was a clear increase of cells progressing into G2 in RB^{-/-} cells, indicating a clear role for RB to block cell cycle upon stress (Fig. 10A). RB cells still displayed a certain delay compared to wild type which could be attributed to additional regulatory mechanisms such as for instance p57 regulation (Joaquin et al., 2012b). Of note, the expression of wild type RB in RB^{-/-} KO cells rescued cell cycle arrest upon stress. In clear contrast, cells expressing the unphosphorylatable RB^{S249A/T252A} allele failed to delay cell cycle and resulted in a phenotype identical to RB^{-/-} null cells (Fig. 10A). Thus, phosphorylation of RB by p38 is important to delay cell cycle upon stress.

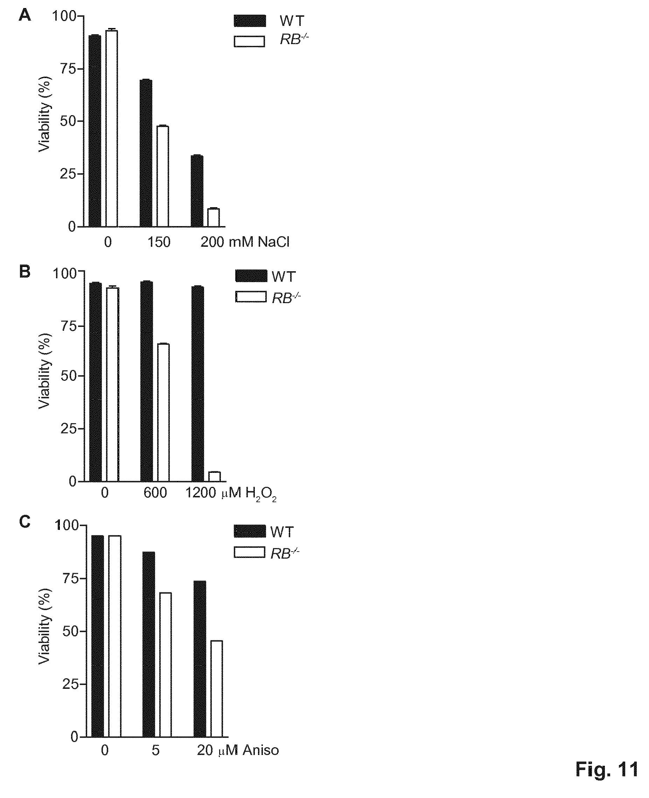

Delaying cell cycle progression in G1 has shown to be important to maximize cell survival upon stress in yeast and mammals (Escote et al., 2004; Joaquin et al., 2012b). To assess the biological relevance of the cell cycle delay mediated by RB phosphorylation, the present inventors monitored cell viability by propidium iodide (PI) staining and FACS in response to several stresses (NaCl, H₂O₂, anisomycin) in wild type and RB^{-/-} knock out cells and found that RB^{-/-} cells were more sensitive to stress than wild type cells (Fig. 10B and Fig. 11). The present inventors then assessed the relevance of RB phosphorylation by p38 to promote cell survival upon stress and found that knock out cells expressing wild type RB were able to survive similarly to wild type cells, whereas cells expressing RB^{S249A/T252A} displayed a survival rate as low as the observed in RB^{-/-} knock out cells (Fig. 10B). Therefore, cell cycle delay mediated by phosphorylation of RB by p38 is essential to promote cell survival upon stress.

### - Example 6: p38-phosphorylated RB is insensitive to CDK inactivation

A mechanism by which CDKs inactivates RB activity is mediated by several phosphorylations, most of them in the C-terminal regional and pocket domains of RB which promote the dissociation of RB from E2F (Rubin, 2013; Munro et al., 2012). The facts that p38 phosphorylation lies in a different domain to those phosphorylations by CDKs and that such phosphorylations increases the affinity of the N-terminal region towards E2F prompted the present inventors to test whether the phosphorylation of p38 was independent and dominant over the inactivation phosphorylations mediated by CDKs.

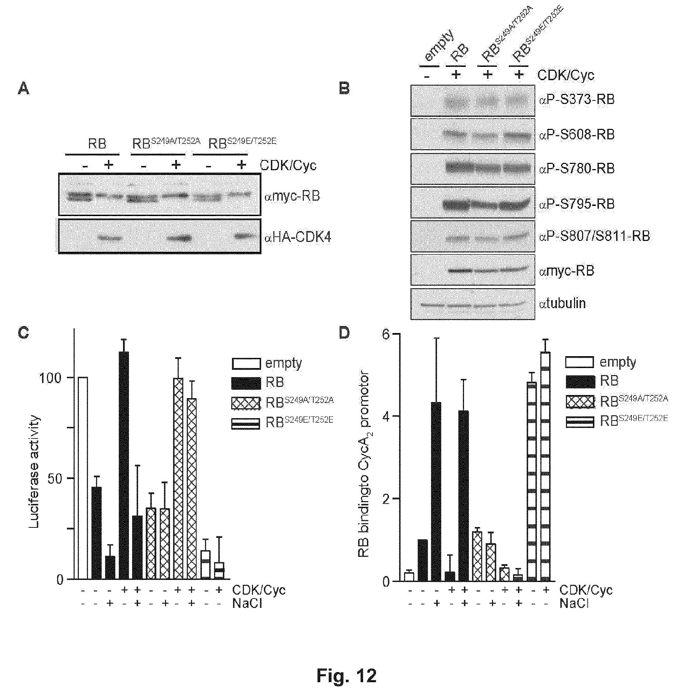

Initially, it was assessed whether the different mutants of RB (RB^{S249A/T252A} and RB^{S249E/T252E}) were phosphorylated by CDKs similarly to wild type in cells overexpressing CDKs. HEK293T cells were transfected with Myc-tagged RBs and CDK4/CycD and CDK2/CycA and RB mobility and phosphorylation was assessed by specific antibodies. It was found that the mutant forms of RB were phosphorylated by CDKs at the same extent than wild type (Fig. 12A and 12B).

Then, the present inventors assessed RB activity using the E2F-reporter assay of Example 3 and found that overexpression of CDK activity clearly increased E2F-dependent transcription in the absence of stress (as expected), whereas the presence of stress (NaCl) prevented this increase on gene expression (Fig. 12C). In clear contrast, the suppression of CDK induction by stress was not observed in the non-phosphorylatable RB^{S249A/T252A} mutant. Correspondingly, the RB^{S249E/T252E} did not display any increase on transcription upon CDK activation (Fig. 12C), indicating that RB phosphorylated by p38 and myc-RB^{S249E/T252E} mutant are insensitive to CDKs.

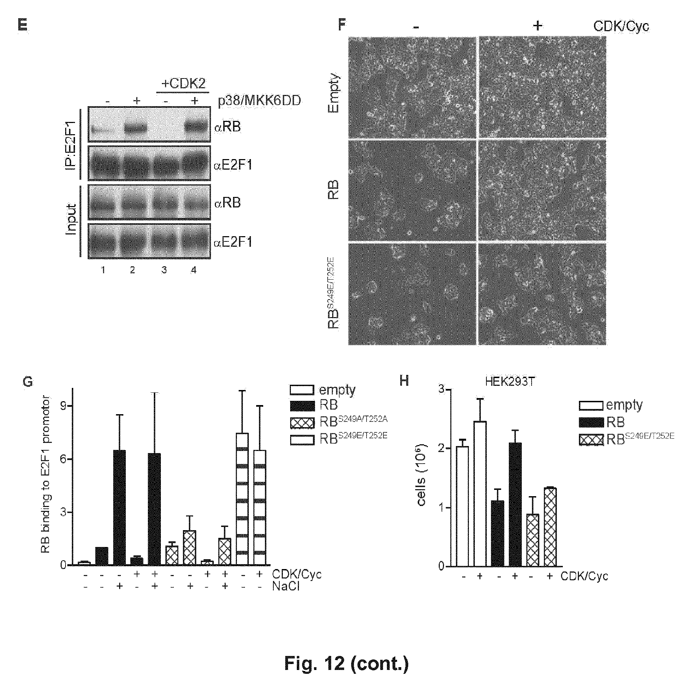

To support this observation, the present inventors followed association of RB to the endogenous CycA₂ promoter by ChIP assays. CDK activation promoted the release of RB from the promoter in the absence of stress but was unable to do so in the presence of stress (Fig. 12D). The dissociation of RB from the promoter by CDKs occurred even in the presence of stress in the RB^{S249A/T252A} mutant as expected from the transcription data. Furthermore, the p38 phosphomimic RB^{S249E/T252E} mutant strongly associated to the CycA₂ promoter in the absence of stress and even in the presence of high CDK activity (Fig. 12D). These results confirm that CDK activity is not able to remove p38-phosphorylated RB and myc-RB^{S249E/T252E} from CycA₂ promoter. Similar results were obtained when binding of RB at E2F1 promoter was analyzed (Fig. 12G). Therefore, the phosphorylation of RB by p38 prevents the inactivation of RB by CDKs.

The biochemical data set forth in the present application indicate an increase on the affinity of RB to E2F upon p38 phosphorylation. Thus, based on the above discussed gene expression assays, it should be expected that the increase of E2F-RB affinity should occur even in the presence of CDK activity. Purified GST-tagged full-length RB protein was phosphorylated *in vitro* by p38, CDK2 or both kinases and then incubated with cell extracts expressing HA-E2F. As expected, association of RB with E2F decreased by the phosphorylation with CDK2 (Fig. 12E; lanes 1 and 3). Remarkably, association of RB with E2F was more efficient upon p38 phosphorylation and this increase on the association was not affected by phosphorylation with CDK2 (Fig. 12E lanes 2 and 4).

Expression of CDK promotes cell proliferation due to inactivation of RB activity (Hassler et al., 2007; Burke et al., 2010; Burke et al., 2012). Thus, the present inventors hypothesized that, since p38 phosphorylation results in an RB insensitive to CDK inactivation, then it should prevent cell proliferation in the presence of high CDK activity. HEK293T cells were transfected with a control vector, wild type RB or the phosphomimetic RB^{S249E/T252E} mutant with or without transfection of CDK2 and CDK4 and cell proliferation was assessed after 72 hours. Both wild type and RB^{S249E/T252E} mutant reduced cell proliferation similarly when compared to control cells in the absence of high CDK, but only the RB^{S249E/T252E} mutant was able to decrease proliferation in the presence of high CDK (Fig. 12F and quantified in Fig.12H). Therefore, the data confirmed that CDK-phosphorylated-RB^{S249E/T252E} can repress cell growth.

Taken together the mentioned data suggests that the RB phosphorylation by p38 or mutations in RB that mimic this phosphorylation leads to a CDK insensitive RB that represses E2F-dependent transcription and prevents cell proliferation even in the presence of high CDK activity.

### - Example 7: A mutant that mimics RB phosphorylation (RB^{EE}) prevents cell proliferation in cancer cells

As it was discussed above, many cancer cells display unregulated cell proliferation due to mutations that lead to an increase on CDK activity. Thus, expression of the mutant that mimics RB phosphorylation should lead to inhibition of cell proliferation of those cells. To test this possibility, the present inventors initially transfected MCF7 (breast cancer cells) with wild type and the RB^{S249E/T252E} mutant. Cell growth was assessed after three days. Cells expressing the phosphomimetic mutant displayed a clear reduction on cellular growth when compared to cells expressing wild type RB (Fig. 13A and quantification in Fig. 14A).

Next, cell cycle progression was assessed in MCF7 cells by FACS. Nocodazole was added to trap the cells at G2/M and the % of cells that progressed from G1 to G2 was measured at the indicated times after nocodazole was added. Cells containing the RB^{S249E/T252E} mutant remained in G1 longer than cells transfected with an empty plasmid or cells carrying wild type RB (Fig. 13B), albeit expressing similar levels of RB protein and consistent with their ability to repress E2F-dependent transcription (Fig. 13D).

The present inventors then followed in a Colony formation assay the long term effect of RB on proliferation of MCF7 cells. It was found the colony-forming ability of MCF7 cell line was strongly reduced by the expression of the RB^{S249E/T252E} mutant (Fig. 13C and quantification in 14B). Those analysis were then extended to additional cancer cell lines such as PK9, PaTu8988S and L3.6 (pancreatic cancer), 235J (bladder cancer) and MDA_MB_231 (breast cancer). Expression of the RB^{S249E/T252E} mutant reduced expression of E2F-reporter expression on those cell lines and reduced colony formation as in MCF7 cells (Fig. 13C, 13D and quantification in 14B), showing that RB^{S249E/T252E} acts as a superrepressor. Therefore, expression of a mutant that mimics the N-terminal phosphorylation of RB is able to reduce cellular proliferation in cancer cells.

### References

Adrover,M.A., Zi,Z., Duch,A., Schaber,J., Gonzalez-Novo,A., Jimenez,J., Nadal-Ribelles,M., Clotet,J., Klipp,E., and Posas,F. (2011). Time-dependent quantitative multicomponent control of the G-S network by the stress-activated protein kinase Hog1 upon osmostress. Sci. Signal. 4, ra63.
Alonso,G., Ambrosino, C, Jones,M., Nebreda, A.R. (2000). ""Differential activation of p38 mitogen-activated protein kinase isoforms depending on signal strength". J Biol Chem 275: 40641-40648
Ambrosino,C. and Nebreda,A.R. (2001). Cell cycle regulation by p38 MAP kinases. Biol. Cell 93, 47-51.
"Antibodies: A Laboratory Manual", 2nd Edition, 2014, Cold Spring Harbor Laboratory Press
Baker,N.A., Sept,D., Joseph,S., Holst,M.J., and McCammon,J.A. (2001). Electrostatics of nanosystems: application to microtubules and the ribosome. Proc. Natl. Acad. Sci. U. S. A 98, 10037-10041.
Biasini,M., Bienert,S., Waterhouse,A., Arnold,K., Studer,G., Schmidt,T., Kiefer,F., Cassarino,T.G., Bertoni,M., Bordoli,L., and Schwede,T. (2014). SWISS-MODEL: modelling protein tertiary and quaternary structure using evolutionary information. Nucleic Acids Res. 42, W252-W258.
Bonet,J., Planas-Iglesias,J., Garcia-Garcia,J., Marin-Lopez,M.A., Fernandez-Fuentes,N., and Oliva,B. (2014). ArchDB 2014: structural classification of loops in proteins. Nucleic Acids Res. 42, D315-D319.
Bulavin,D.V. and Fornace,A.J. (2004). p38 MAP kinase's emerging role as a tumor suppressor. Adv Cancer Res 92, 95-118.
Burke,J.R., Deshong,A.J., Pelton,J.G., and Rubin,S.M. (2010). Phosphorylation-induced conformational changes in the retinoblastoma protein inhibit E2F transactivation domain binding. J. Biol. Chem. 285, 16286-16293.
Burke,J.R., Hura,G.L., and Rubin,S.M. (2012). Structures of inactive retinoblastoma protein reveal multiple mechanisms for cell cycle control. Genes Dev. 26, 1156-1166.
Burkhart,D.L. and Sage,J. (2008). Cellular mechanisms of tumour suppression by the retinoblastoma gene. Nat. Rev. Cancer 8, 671-682.
Chinnam,M. and Goodrich,D.W. (2011). RB1, development, and cancer. Curr. Top. Dev. Biol. 94, 129-169.
Cobrinik,D. (2005). Pocket proteins and cell cycle control. Oncogene 24, 2796-2809.
Cuadrado,A. and Nebreda,A.R. (2010). Mechanisms and functions of p38 MAPK signalling. Biochem. J. 429, 403-417.
Cuadrado,M., Gutierrez-Martinez,P., Swat,A., Nebreda,A.R., and Fernandez-Capetillo,O. (2009). p27Kip1 stabilization is essential for the maintenance of cell cycle arrest in response to DNA damage. Cancer Res 69, 8726-8732.
Delston,R.B., Matatall,K.A., Sun,Y., Onken,M.D., and Harbour,J.W. (2011). p38 phosphorylates Rb on Ser567 by a novel, cell cycle-independent mechanism that triggers Rb-Hdm2 interaction and apoptosis. Oncogene 30, 588-599.
Dick,F.A. and Rubin,S.M. (2013). Molecular mechanisms underlying RB protein function. Nat. Rev. Mol. Cell Biol. 14, 297-306.
Dolinsky,T.J., Czodrowski,P., Li,H., Nielsen,J.E., Jensen,J.H., Klebe,G., and Baker,N.A. (2007). PDB2PQR: expanding and upgrading automated preparation of biomolecular structures for molecular simulations. Nucleic Acids Res. 35, W522-W525.
Escote,X., Zapater,M., Clotet,J., and Posas,F. (2004). Hog1 mediates cell-cycle arrest in G1 phase by the dual targeting of Sic1. Nat. Cell Biol. 6, 997-1002.
Eswar,N., Webb,B., Marti-Renom,M.A., Madhusudhan,M.S., Eramian,D., Shen,M.Y., Pieper,U., and Sali,A. (2006). Comparative protein structure modeling using Modeller. Curr. Protoc. Bioinformatics. Chapter 5, Unit.
Fiser,A., Do,R.K., and Sali,A. (2000). Modeling of loops in protein structures. Protein Sci. 9, 1753-1773.
Gonzalez-Novo,A., Jimenez,J., Clotet,J., Nadal-Ribelles,M., Cavero,S., de,N.E., and Posas,F. (2015). Hog1 targets Whi5 and Msa1 transcription factors to down-regulate cyclin expression upon stress. Mol. Cell Biol. 35, 1606-1618.
Gonzalez,S., Serrano,M. (2006). A new mechanism of inactivation of the INK4/ARF locus. Cell Cycle 5: 1382-1384.
Grana,X., Garriga,J., and Mayol,X. (1998). Role of the retinoblastoma protein family, pRB, p107 and p130 in the negative control of cell growth. Oncogene 17, 3365-3383.
Hanahan,D. and Weinberg,R.A. (2011). Hallmarks of cancer: the next generation. Cell 144, 646-674.
Hassler,M., Singh,S., Yue,W.W., Luczynski,M., Lakbir,R., Sanchez-Sanchez,F., Bader,T., Pearl,L.H., and Mittnacht,S. (2007). Crystal structure of the retinoblastoma protein N domain provides insight into tumor suppression, ligand interaction, and holoprotein architecture. Mol. Cell 28, 371-385.
Henley,S.A. and Dick,F.A. (2012). The retinoblastoma family of proteins and their regulatory functions in the mammalian cell division cycle. Cell Div. 7, 10.
Hirschi,A., Cecchini,M., Steinhardt,R.C., Schamber,M.R., Dick,F.A., and Rubin,S.M. (2010). An overlapping kinase and phosphatase docking site regulates activity of the retinoblastoma protein. Nat. Struct. Mol. Biol. 17, 1051-1057.
Hunter,J.D. (2007). Matplotlib: A 2D graphics environment. Computing in science and engineering 9, 90-95.
Joaquin,M., Gubern,A., and Posas,F. (2012). A novel G1 checkpoint mediated by the p57 CDK inhibitor and p38 SAPK promotes cell survival upon stress. Cell Cycle 11, 3339-3340.
Joaquin,M., Gubern,A., and Posas,F. (2012b). A novel G1 checkpoint mediated by the p57 CDK inhibitor and p38 SAPK promotes cell survival upon stress. Cell Cycle 11, 3339-3340.
Joaquin,M., Gubern,A., Gonzalez-Nunez,D., Josue,R.E., Ferreiro,I., de Nadal E., Nebreda,A.R., and Posas,F. (2012a). The p57 CDKi integrates stress signals into cell-cycle progression to promote cell survival upon stress. EMBO J. 31, 2952-2964.
Kreck,W., Livingston,D.M.m Shirodakar,S., (1993). "Binding to DNA and the the retinoblastoma gene product promoted by complex formation of different e2f family members". Science 262, 1557-1560.
Knudsen,E.S. and Knudsen,K.E. (2008). Tailoring to RB: tumour suppressor status and therapeutic response. Nat. Rev. Cancer 8, 714-724.
Knudsen,E.S. and Wang,J.Y. (2010). Targeting the RB-pathway in cancer therapy. Clin. Cancer Res. 16, 1094-1099.
Kolupaeva,V. and Janssens,V. (2013). PP1 and PP2A phosphatases--cooperating partners in modulating retinoblastoma protein activation. FEBS J. 280, 627-643.
Lafarga,V., Cuadrado,A., Lopez,d.S., I, Bengoechea,R., Fernandez-Capetillo,O., and Nebreda,A.R. (2009). p38 Mitogen-activated protein kinase- and HuR-dependent stabilization of p21(Cip1) mRNA mediates the G(1)/S checkpoint. Mol. Cell Biol. 29, 4341-4351.
Lee,C., Chang,J.H., Lee,H.S., and Cho,Y. (2002). Structural basis for the recognition of the E2F transactivation domain by the retinoblastoma tumor suppressor. Genes Dev. 16, 3199-3212.
Manning,A.L. and Dyson,N.J. (2012). RB: mitotic implications of a tumour suppressor. Nat. Rev. Cancer 12, 220-226.
Liu,F., and Green,M.R. (1995). Promoter targeting by adenovirus E1 a through interaction with different cellular DNA-binding domains. Nature 368, 520-525.
Marti-Renom,M.A., Madhusudhan,M.S., Fiser,A., Rost,B., and Sali,A. (2002). Reliability of assessment of protein structure prediction methods. Structure. 10, 435-440.
Moberg K, Starz MA, Lees JA (1996). E2F-4 switches from p130 to p107 and pRB in response to cell cycle reentry. Mol Cell Biol. 16(4):1436-49.
Munro,S., Carr,S.M., and La Thangue,N.B. (2012). Diversity within the pRb pathway: is there a code of conduct? Oncogene 31, 4343-4352.
Oliphant,T.E. (2007). Python for scientific computing. Computing in Science & Engineering 9, 10-20.
Pye, C.R., Bray,W.M., Brown,E.R., Burke,J.R., Lokey, R.S., and Rubin,S.M. (2016) A Strategy for Direct Chemical Activation of Retinoblastoma Protein. ACS Chem. Biol., DOI:10. 1021/acschembio.6b00011
Quin,X.Q., Chittenden,T, Livingston,D.M., Kaelin,W.G. Jr. (1992) "Full-length RB cDNA was subcloned into a PGEX-2TK". Genes Dev. 6(6), 953-64..
Real,S., Meo-Evoli,N., Espada,L., and Tauler,A. (2011). E2F1 regulates cellular growth by mTORC1 signaling. PLoS One 6, e16163.
Reinhardt,H.C., Aslanian,A.S., Lees,J.A., and Yaffe,M.B. (2007). p53-deficient cells rely on ATM- and ATR-mediated checkpoint signaling through the p38MAPK/MK2 pathway for survival after DNA damage. Cancer Cell 11, 175-189.
Rose,P.W., Beran,B., Bi,C., Bluhm,W.F., Dimitropoulos,D., Goodsell,D.S., Prlic,A., Quesada,M., Quinn,G.B., Westbrook,J.D., Young,J., Yukich,B., Zardecki,C., Berman,H.M., and Bourne,P.E. (2011). The RCSB Protein Data Bank: redesigned web site and web services. Nucleic Acids Res. 39, D392-D401.
Rubin,S.M. (2013). Deciphering the retinoblastoma protein phosphorylation code. Trends Biochem. Sci. 38, 12-19.
Rubin,S.M., Gall,A.L., Zheng,N., and Pavletich,N.P. (2005). Structure of the Rb C-terminal domain bound to E2F1-DP1: a mechanism for phosphorylation-induced E2F release. Cell 123, 1093-1106.
Rushlow,D.E., Mol,B.M., Kennett,J.Y., Yee,S., Pajovic,S., Theriault,B.L., Prigoda-Lee,N.L., Spencer,C., Dimaras,H., Corson,T.W., Pang,R., Massey,C., Godbout,R., Jiang,Z., Zacksenhaus,E., Paton,K., Moll,A.C., Houdayer,C., Raizis,A., Halliday,W., Lam,W.L., Boutros,P.C., Lohmann,D., Dorsman,J.C., and Gallie,B.L. (2013). Characterisation of retinoblastomas without RB1 mutations: genomic, gene expression, and clinical studies. Lancet Oncol. 14, 327-334.
Sage,J. and Cleary,M.L. (2012). Genomics: The path to retinoblastoma. Nature 481, 269-270.
Sangwan,M., McCurdy,S.R., Livne-Bar,I., Ahmad,M., Wrana,J.L., Chen,D., and Bremner,R. (2012). Established and new mouse models reveal E2f1 and Cdk2 dependency of retinoblastoma, and expose effective strategies to block tumor initiation. Oncogene 31, 5019-5028.
Saville,M.K. and Watson,R.J. (1998). The cell-cycle regulated transcription factor B-Myb is phosphorylated by cyclin A/Cdk2 at sites that enhance its transactivation properties. Oncogene 17, 2679-2689.
Solit,D.B., and Rosen,N. (2011) Resistance to BRAF inhibition in melanomas. N Eng J Med 364(8):772-774.
Stone,A., Sutherland,R.L., and Musgrove,E.A. (2012). Inhibitors of cell cycle kinases: recent advances and future prospects as cancer therapeutics. Crit Rev. Oncog. 17, 175-198.
Stranges,P.B. and Kuhlman,B. (2013). A comparison of successful and failed protein interface designs highlights the challenges of designing buried hydrogen bonds. Protein Sci. 22, 74-82.
Takahashi,C., Sasaki,N., and Kitajima,S. (2012). Twists in views on RB functions in cellular signaling, metabolism and stem cells. Cancer Sci. 103, 1182-1188.
Talluri,S. and Dick,F.A. (2012). Regulation of transcription and chromatin structure by pRB: here, there and everywhere. Cell Cycle 11, 3189-3198.
Di Tommaso P, Moretti S, Xenarios I, Orobitg M, Montanyola A, Chang JM, Taly JF, Notredame C. Nucleic Acids Res. 2011 Jul;39(Web Server issue):W13-7. doi: 10.1093/nar/gkr245. Epub 2011 May 9
Wang,H., Bauzon,F., Ji,P., Xu,X., Sun,D., Locker,J., Sellers,R.S., Nakayama,K., Nakayama,K.I., Cobrinik,D., and Zhu,L. (2010). Skp2 is required for survival of aberrantly proliferating Rb1-deficient cells and for tumorigenesis in Rb1+/- mice. Nat. Genet. 42, 83-88.
Wang,S., Nath,N., Minden,A., and Chellappan,S. (1999). Regulation of Rb and E2F by signal transduction cascades: divergent effects of JNK1 and p38 kinases. EMBO J. 18, 1559-1570.
Witkiewicz,A.K., Ertel,A., McFalls,J., Valsecchi,M.E., Schwartz,G., and Knudsen,E.S. (2012). RB-pathway disruption is associated with improved response to neoadjuvant chemotherapy in breast cancer. Clin. Cancer Res. 18, 5110-5122.
Xiao,B., Spencer,J., Clements,A., Ali-Khan,N., Mittnacht,S., Broceno,C., Burghammer,M., Perrakis,A., Marmorstein,R., and Gamblin,S.J. (2003). Crystal structure of the retinoblastoma tumor suppressor protein bound to E2F and the molecular basis of its regulation. Proc. Natl. Acad. Sci. U. S. A 100, 2363-2368.
Yang,J. and Zhang,Y. (2015). I-TASSER server: new development for protein structure and function predictions. Nucleic Acids Res. 43, W174-W181.
Yang,Z., Lasker,K., Schneidman-Duhovny,D., Webb,B., Huang,C.C., Pettersen,E.F., Goddard,T.D., Meng,E.C., Sali,A., and Ferrin,T.E. (2012). UCSF Chimera, MODELLER, and IMP: an integrated modeling system. J. Struct. Biol. 179, 269-278.
Zhang,J., Benavente,C.A., McEvoy,J., Flores-Otero,J., Ding,L., Chen,X., Ulyanov,A., Wu,G., Wilson,M., Wang,J., Brennan,R., Rusch,M., Manning,A.L., Ma,J., Easton,J., Shurtleff,S., Mullighan,C., Pounds,S., Mukatira,S., Gupta,P., Neale,G., Zhao,D., Lu,C., Fulton,R.S., Fulton,L.L., Hong,X., Dooling,D.J., Ochoa,K., Naeve,C., Dyson,N.J., Mardis,E.R., Bahrami,A., Ellison,D., Wilson,R.K., Downing,J.R., and Dyer,M.A. (2012). A novel retinoblastoma therapy from genomic and epigenetic analyses. Nature 481, 329-334.
Zhu L, Xie E, Chang LS (1995) Differential roles of two tandem E2F sites in repression of the human p107 promoter by retinoblastoma and p107 proteins. Mol Cell Biol 15: 3552-3562.

## Claims

**1.** A method for identifying a compound as a candidate to drug for the control of cancer that comprises the steps of:
a) contacting a cell culture with the compound, under suitable conditions and during enough time for having an interaction between the compound and the cells;
b) determining and quantifying a property or process that is affected by the level of phosphorylation of human retinoblastoma protein (RB) in the serine amino acid residue of position 249 and/or the threonine amino acid residue of position 252 (Ser249/Thr252) and/or by any other condition that provokes the same effect of such phosphorylation which property or process is selected from the group of:
i. the affinity of RB for E2F,
ii. the level of transcription of E2F,
iii. the level of expression of a gene that is under the transcriptional control or operatively linked to an E2F-dependent promoter and that depends on the binding of the E2F transcription factor for being transcribed,
iv. the level of phosphorylation of human retinoblastoma protein (RB) in the serine amino acid residue of position 249 and/or the threonine amino acid residue of position 252 (Ser249 and/or Thr252) itself, or the level of phosphorylation of the equivalent serine and threonine residues of the N-terminal domain of the retinoblastoma protein of another mammal,
v. the binding of RB to the E2F-dependent promoters,
vi. the binding of RNA Pol II to the E2F-dependent promoters, and
vii. the effect on cell growth proliferation
or combinations thereof;
c) comparing the level of the property or process assessed in step b) with the level of said property determined in a control cell culture of the same cells, that have been subjected to the same conditions that the treated cells of step a) except for the fact that the cells have not been contacted with the assayed compound;
d) identifying the compound as a candidate to drug for the control of cancer if the level of the assessed property or process has changed in the following sense:
i. the affinity of RB for E2F is increased in the cells treated with the compound,
ii. the level of transcription of E2F is decreased in the cells treated with the compound,
iii. the level of expression of the assayed gene that is under the transcriptional control or operatively linked to an E2F-dependent promoter and that depends on the binding of the E2F transcription factor, is decreased in the cells treated with the compound,
iv. the level of phosphorylation of Ser249 and/or Thr252 of human retinoblastoma protein, or the level of phosphorylation of the equivalent serine and threonine residues of the N-terminal domain of the retinoblastoma protein of another mammal, is increased in the cells treated with the compound,
v. the binding of RB to the E2F-dependent promoters is increased,
vi. the binding of RNA Pol II to the E2F-dependent promoters is decreased, and/or
vii. the cell growth proliferation is decreased.

**2.** The method according to claim 1,
which:
comprises a previous step wherein the cells to be contacted with the assayed compound and the cells of the control cell culture are transfected with an expression vector wherein the coding sequence of a reporter gene is operatively linked to a promoter that requires binding of E2F transcription factor for driving the transcription of the gene,
and wherein
the assayed compound is identified as a candidate to drug for the control of cancer if the level of expression of the reporter gene is decreased in the cells that have been treated with the assayed compound with regard to the control cells.

**3.** The method according to claim 2, wherein the cells have been also transfected with an expression vector of retinoblastoma protein and an expression vector of E2F1, E2F2 or E2F3 protein.

**4.** The method according to claim 3, wherein the cells have been additionally transfected with an expression vector of DP1 protein.

**5.** The method according to any one of claims 2 to 4, wherein the expression of the reporter gene gives rise to a reporter protein which is a fluorescent protein or an enzyme whose activity results in a product that can be detected by analysis of the growth, colour or morphology of the cells, directly or after exposure to a reagent.

**6.** The method according to claim 5, wherein the reporter protein is the luciferase fluorescent protein.

**7.** The method according to claim 1, wherein the level of phosphorylation of retinoblastoma protein (RB) in the serine amino acid residue of position 249 and the threonine amino acid residue of position 252 (Ser249/Thr252) is determined in the control cells and in the cells treated with the assayed compound, and wherein the level of retinoblastoma protein phosphorylated at Ser249/Thr252 is determined by means of an antibody that recognized the retinoblastoma protein phosphorylated at Ser249 and Thr252.

**8.** The method according to any one of claims 1 to 7, wherein the cultured cells are cells of a cancer cell line.

**9.** The method according to claim 8, wherein the cell are cells of a cancer cell line with high CDK activity.

**10.** The method according to claim 8 or 9, wherein the cells are cells of a cancer cell line of breast, pancreas or bladder cancer.

**11.** The method according to any one of claims 1 to 10, wherein the compounds identified as candidates for the control of cancer are identified as candidates for the treatment of breast, pancreas or bladder cancer.

**11.** A compound identified as a candidate to drug for the control of cancer by the method of any one of claims 1 to 10, for use in the treatment of cancer.

**12.** A compound identified as a candidate to drug for the control of cancer by the method of any one of claims 1 to 10, for use in the treatment breast, pancreas or bladder cancer.

**13.** An expression vector of a retinoblastoma mutant protein wherein both the serine and the threonine residues of the consensus phosphorylation sites Serine-Proline (SP) and Threonine-Proline (TP) of the N-terminal domain which are or that can be considered equivalent to the Serine249 and the Threonine252 of the human RB protein of the N-terminal domain are replaced by a glutamic acid residue, for use in the treatment of cancer in a mammal.

**14.** An expression vector for use according to claim 13, wherein the retinoblastoma mutant protein is the human protein, the serine and threonine residues replaced by a glutamic acid residue are serine 249 and threonine 252 respectively, and the mammal is a human being.

**15.** An expression vector for use according to claim 13 or 14, wherein the cancer is breast, pancreas or bladder cancer.
